# EUROPEAN PATENT APPLICATION

(11) **EP 2 589 663 A1**
(43) Date of publication of application: **08.05.2013**
(21) Application number: 11187808.8
(22) Date of filing: 04.11.2011
(51) Int. Cl.: C12P 17/18

(54) **Process for production of clavulanic acid**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention belongs to the field of pharmaceutical industry and specifies the process for preparing clavulanic acid by using a microorganism, comprising the steps of providing a microorganism being capable of producing clavulanic acid and which produces d-(L-α-aminoadipyl)-L-cysteinyl-D-valine synthetase (ACVS), selectively reducing or abolishing ACVS activity by modifying or treating said microorganism, cultivating it and isolating clavulanic acid. Furthermore, the present invention refers to a process for preparing clavulanic acid by using said modified microorganism. Further, the present invention relates to a recombinant plasmid comprising a modified *pcbAB* gene, to a recombinant microorganism comprising said recombinant plasmid, and to the use of said recombinant plasmid for modifying a microorganism. Finally, the present invention also refers to a microorganism that is modified or treated by selectively reducing or abolishing activity of ACVS, as well as to the use of said microorganism for the production of clavulanic acid.

## Description

### Field of the invention

The present invention belongs to the field of pharmaceutical industry and relates to a process for preparing clavulanic acid by using a microorganism, comprising the step of providing a microorganism being capable of producing d-(L-α-aminoadipyl)-L-cysteinyl-D-valine (ACVS), wherein said microorganism is modified or treated in that its enzyme ACVS is selectively reduced or abolished. Furthermore, the present invention refers to a recombinant plasmid comprising a specific *pcbAB* gene that encodes partially or completely inactivated enzyme ACVS, a microorganism comprising said recombinant plasmid, as well as to the use of said recombinant plasmid for modifying a microorganism. Finally, the present invention relates to a microorganism that is modified or treated by selectively reducing or abolishing activity of ACVS, and to the use of said microorganism for the production of clavulanic acid.

### Description of the background art

Historically, β-lactam antibiotics such as penicillin and cephalosporin were among the first useful antibiotics discovered and remain at the forefront of clinical use to combat bacterial infections. β-lactam resistant bacterial pathogens, however, started to emerge after application to general medical use mostly due to the wide-spread use of these antibiotics for more than 50 years. This has, in turn, drastically reduced their efficacy in combating bacterial infections. As a consequence, strategies aimed at overcoming acquired resistance have become of increasing interest. One of the best examples in broad clinical application is the development of β-lactamase inhibitors. The discovery of clavulanic acid (CA) was reported in 1976 (Reading and Cole, 1977). CA has been shown to be a potent inhibitor of β-lactamases produced by staphylococci, plasmid-mediated β-lactamases produced by *E*. *coli,* as well as β-lactamases produced by species from Klebsiella, Proteus, and Hemophilus (Brown *et al.,* 1976). Later, CA was found to be active against broad spectrum of Gram-positive and Gram-negative bacteria, however with a low activity. Clavulanic acid, sharing a similar chemical structure with the β-lactam antibiotics, binds irreversibly with the enzyme β-lactamase to give a stable complex (Liras and Rodriguez-Garcia, 2000), thereby rendering it inactive. Co-formulation of CA with other broad-spectrum antibiotics which are themselves susceptible to β-lactamases became the preferred use of this important secondary metabolite (Brown, 1986).

Today, the use of well established commercial products such as Augmentin(R) and Timentin(R), the combination of clavulanic acid and β-lactam antibiotics (Amoxicillin and Ticarcillin, respectively) is globally spread and often prescribed as a broad spectrum antibiotic of choice. In fact Augmentin(R) (GlaxoSmithKline) and its generic equivalents are the most widely prescribed anti-infective agents. The global market in antibiotics combined with clavulanic acid has attained sales close to 2 billion dollars yearly (Saudagar *et al.,* 2008).

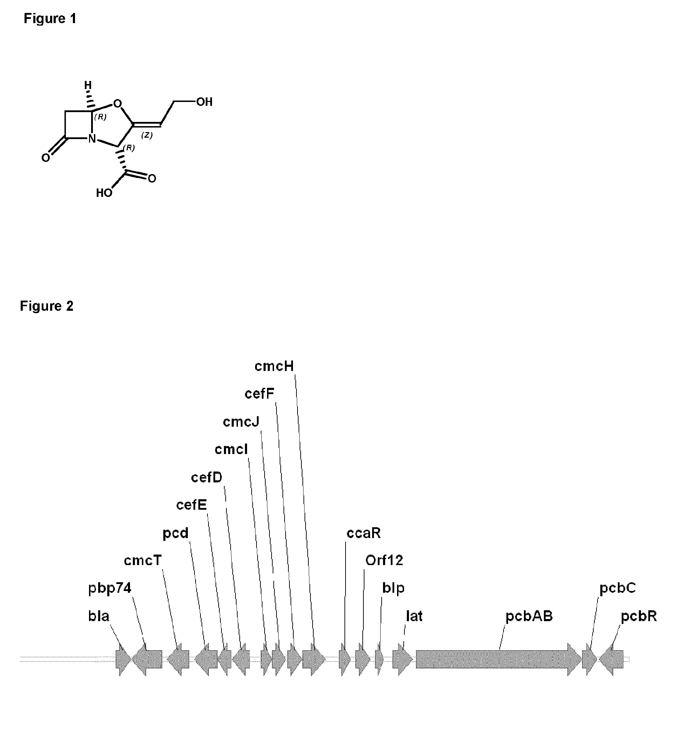

Figure 1 shows the molecular structure of clavulanic acid. Clavulanic acid or (2R,5R,Z)-30(2 hydroxyethylidene) -7- oxo-4-oxa-I- azabicyclo [3.2.0] heptane-2- carboxylic acid is produced by several microorganisms, namely e.g. by *Streptomyces clavuligerus* ATCC 27064 (for instance described in US4110165, or in BE827926), *Streptomyces jumonjinensis* (GB 1563103), *Streptomyces katsurahamanus* IFO 13716 FERM 3944 (described in JP83009679B) and *Streptomyces sp.* P6621 FERM 2804 (described in JP55162993A), although *S. clavuligerus* ATCC 27064 (equivalent to *S*. *clavuligerus* NRRL 3585) and its improved relatives are by far the most widely used and most studied in connection with clavulanic acid production.

Microorganisms, especially Streptomycetes, are producers of a large variety of secondary metabolites such as antibiotics, often of several different natural products simultaneously. There is considerable interest in being able to manipulate the absolute and relative amounts of these antibiotics produced and, accordingly, there have been a large number of studies investigating the metabolic and genetic mechanisms of these pathways (see, e.g. Demain, 1990). Like other Streptomycetes, *S. clavuligerus* produces other natural products apart from clavulanic acid. Holomycin, clavams other than CA, tunicamycin, penicillins *etc.,* have been identified in *S. clavuligerus* culture broths (Medema *et al.,* 2010). Recently, with help of the whole genome sequencing and bioinformatic tools, potential genetic clusters for 48 different secondary metabolites have been found in the genome of *S. clavuligerus* (Medema *et al.* 2010). Most notably, cephamycin C, belonging to the β-lactam antibiotic family that includes penicillin G and cephalosporin C, is produced in concert with CA by *S*. *clavuligerus,* as well as its bioactive intermediates: Penicillin N and O-carbamoyldeacetylcephalosporin C (Song *et al.,* 2010).

The primary metabolic precursors of clavulanic acid are D-glyceraldehyde-3-phosphate (G3P) (Khaleeli *et al.,* 1999) and L-arginine (Valentine *et al.,* 1993). The biosynthetic pathway is well characterized today (Saudagar *et al.,* 2008) with exception of the late steps inverting clavaminic acid 3S,5S stereochemistry to 3R,5R clavaldehyde and subsequently 3R,5R oriented clavulanic acid. These steps remain to be elucidated in full (Arulanantham *et al.,* 2005).

The gene clusters that specify biosynthesis of clavulanic acid and cephamycin C have been shown to reside immediately adjacent to one another (Ward and Hodgson, 2006). In each cluster, there is a pathway-specific regulator including CcaR for cephamycin C biosynthesis and ClaR for clavulanic acid biosynthesis. Both gene products are positive regulators for the corresponding clusters. However, while ClaR specifically regulates clavulanic acid production, CcaR regulates both cephamycin C and clavulanic acid production. Experimental evidence suggests that CcaR regulates clavulanic acid levels by affecting directly claR expression (Paradkar *et al.,* 1998; Perez-Llarena *et al.,* 1997; and Perez-Redondo *et al.,* 1998).

In addition, biochemical and regulatory related clavams such as alanyl-clavam, clavam-2-carboxylate and others are produced by *S*. *clavuligerus,* sharing early synthetic genes with CA biosynthesis, albeit a paralogous copy of the early genes leading to clavaminic acid was identified in the *S. clavuligerus* genome (Tahlan *et al.,* 2004). The common intermediate of CA and antipodal clavams, clavaminic acid, makes the accumulation of the non CA clavams a metabolic burden for the microorganism at the expense of CA titers. Inevitably the CA - cephamycin C cluster regulon has its impact also on antipodal clavam synthesis although interconnections are poorly understood especially due to different behavior when cultivated on various media (Tahlan *et al.,* 2004; Tahlan *et al.,* 2007).

Until now, traditional strain improvement with regard to clavulanic acid production has been carried out. This traditional strain improvement, which can be used to obtain high-titer industrial fermentation strains, largely depends on random mutagenesis and selection techniques. Development of new generation of high producing strains, however, with this approach often takes 5 years or more (Nielsen, 1997). A significant drawback is the introduction of a limited spectrum of base-pair substitutions that do not readily solve the specific rate limitations of biosynthetic pathways (Baltz, 1998) in addition to accumulation of unwanted silent mutations which come to attention as limitations only when the production titers reach a plateau at certain titers. Further biochemical analyses show that these improved strains still retain the capability to produce undesired products, mainly cephamycin C and antipodal clavams (Saudagar *et al.,* 2009).

In the past decade, as molecular genetics techniques have become increasingly sophisticated, the ability to modify existing pathways or create non-native pathways has advanced rapidly. Progress in genetics, transcriptional analysis, proteomics, metabolic reconstructions and metabolic flux analysis offer genetic engineering as an alternative approach for strain improvement in a targeted manner (Baltz, 2001). Duplication, replacement or deletion of specific genes can be achieved by inserting molecular tool targeting the desired gene(s) in the chromosome by homologous recombination or by site-specific integration. Targets for genetic engineering are usually the genes thought to be involved in rate limiting steps, the genes involved in undesired pathways and global or specific pathway regulators.

Specifically, one approach to enhance the CA production in *S. clavuligerus* is to inactivate pathways that compete for key precursors, intermediates, cofactors and energy supply within the primary metabolism. One such example was reported on rational strain improvement for enhanced CA production by genetic engineering of the glycolytic pathway in *S. clavuligerus.* Two genes (*gap1* and *gap2),* whose protein products are distinct glyceraldehyde-3-phosphate dehydrogenases (GAPDHs), were inactivated in *S. clavuligerus* by targeted gene disruption. A doubled production of CA was consistently obtained when *gap1* was disrupted, and reversed by complementation (Li and Townsend, 2006; WO2007/030772).

Another approach undertaken showed that the gene dosage constructs of the biosynthetic genes *ceaS2* and cas2 resulted in recombinant strains with 60% and 100% higher clavulanic acid production, respectively, compared to the wild-type strain in S. *clavuligerus* (Perez-Redondo *et al.,* 1999. Disruption of negative regulatory gene(s), or increased expression of positive regulatory gene(s), can also result in the elevated production of secondary metabolites. Paradkar *et al.* observed a 2- to 3-fold increase in CA production by introducing additional copies of positive regulatory genes in the wild-type (Paradkar *et al.,* 1998; Perez-Llarena *et al.,* 1997; and Perez-Redondo *et al.,* 1998).

The third approach is to inactivate secondary methabolyte biosynthesis pathways that compete for key precursors, intermediates, co-factors and energy supply. The inactivation of the clavam pathway, which shares the common intermediate clavaminic acid with the clavulanic acid pathway, has been shown to give an elevated yield of clavulanic acid in *S*. *clavuligerus* (Paradkar et al., 2001; WO2004/092389; EP0970198).

In this aspect, several studies performed on S. *clavuligerus* deal with biosynthesis of cephamycin C and related molecules. These β-lactam antibiotics are synthesized from the tripeptide precursor d-(L-α-aminoadipyl)-L-cysteinyl-D-valine (ACV) (Martin *et al.,* 1999). Biosynthesis of cephamycin C in *Streptomyces clavuligerus* involves the initial conversion of lysine to α-aminoadipic acid. The recognized first dedicated step in the biosynthesis of both penicillins and cephalosporins in S. *clavuligerus* involves the enzyme L-lysine ε-aminotransferase (LAT). The second step involves piperideine-6-carboxylate dehydrogenase (PCD), the product of *S*. *clavuligerus pcd* gene, providing α-aminoadipic acid as one of the substrates for the tripeptide synthetase. The nucleotide sequence and the derived amino acid sequence of *S*. *clavuligerus lat* and *pcd* genes are known (see for instance Tobin *et al.,* 1991; Tahlan *et al.,* 2004). In Figure 2, a graphic representation of the *S*. *clavuligerus* cephamycin C gene cluster is depicted.

LAT and PCD, catalyzing the first and the second step in cephamycin C biosynthesis, respectively have been subject to genetic modification in an attempt to abolish cephamycin C synthesis in a CA producing microorganism. In 1987, a spontaneous mutant strain of *S*. *clavuligerus* (NPI) was described, which did not produce cephamycin C (Mahro *et al*., 1987). Later, it was demonstrated that the activity of the the enzymes LAT, ACVS and IPNS can be recovered by transforming said mutant with a fragment of DNA encoding the entire *lat* gene and the upstream half of the *pcbAB* gene (Yu *et al.,* 1994). However, there was no teaching of any effect in this mutant in relation to the production of clavulanic acid or other clavams.

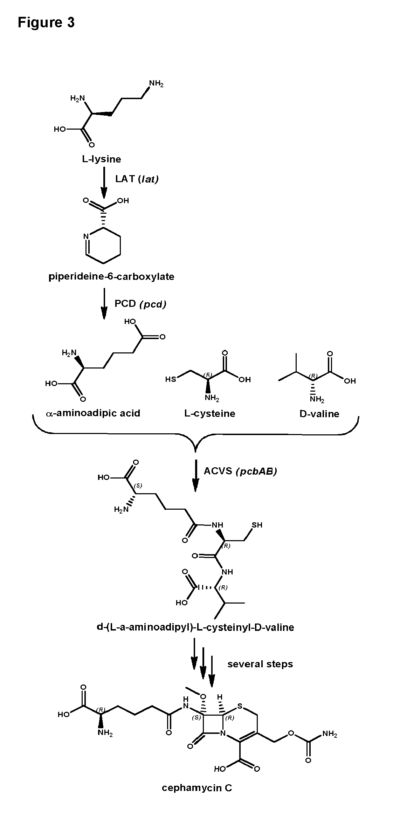

The inactivation of *pcd,* the gene encoding the second step enzyme PCD, resulted in positive effects on CA production in *S. clavuligerus pcd* deletion mutant (see, e.g. WO2005/075659). The authors, however, observed no change in cephamycin C production. This observation was confirmed later by an independent group, which observed some reduction in cephamycin C production in *S*. *clavuligerus pcd* deletion mutant, but not complete abolishment (Dylan *et*. *al.,* 2007). The *pcd* deletion therefore does not solve the given problem of elimination of cephamycin C accumulation in CA production processes. An important teaching of the above publications, nonetheless, is the fact that the α-aminoadipic acid, a substrate for further steps in cephamycin C biosynthesis, can also be provided by another, redundant pathway, independent of the genes *lat* and *pcd* in the cephamycin C cluster. This hypothesis can be followed due to the fact that no residual PCD activity was found in *S*. *clavuligerus pcd* deletion mutant, and cephamycin C was found accumulating all the same (Dylan *et al.,* 2007). In Figure 3, the initial steps of cephamycin C biosynthesis in *S*. *clavuligerus* are depicted.

Further with this respect, cephamycin C production was blocked in wild-type cultures of the CA-producing organism *S*. *clavuligerus* by targeted disruption of the gene *(*/*at)* encoding lysine ε-aminotransferase. Nonetheless, production of CA increased in the *lat* mutants derived from the wild-type strain by 2.0 to 2.5-fold (Pradkar et al., 2001; W09641886). In addition, for the first time the effect of such genetic manipulation was evaluated in a high-producer strain SB198, showing a 10% improvement compared to parent strain. However, the strain subjected to evaluation was in fact a double mutant, with an additional disruption in a *cvm1* gene, a gene involved in antipodal clavam biosynthesis. The authors conclude that there is no information available as to which change (disruption of *lat* or disruption of *cvm1*) contributed to improved levels of CA production. Although an effort to confirm that the disruption of *lat* gene is indeed responsible for complete abolishment of cephamycin was taken by feeding experiments providing external α-aminoadipic acid to the *lat* disruption strain cultures showing partial restoration of cephamycin C synthesis, an incomplete understanding weather *lat* disruption is the sole reason for cephamycin C abolishment remains. The reason is that the authors themselves found polar effects on expression of the neighboring gene to the *lat,* the *pcbAB* gene, which could easily result, at least in part, in the observed phenotype (Paradkar *et al*., 2001).

Given these findings (Paradkar *et al.,* 2001), and the observations from studies on the role of the *pcd* gene (Dylan *et al.,* 2007), the question remains open whether the genes *lat* and *pcd,* supposedly responsible for the first two steps in the cephamycin C biosynthesis, are the sole pathway towards the precursor of cephamycin C, α-aminoadipic acid, in *S*. *clavuligerus.* Independently of the answer to this question, the first step which clearly differentiates the primary from the secondary metabolism on the biochemical level, despite presence of *lat* and *pcd* genes in the cephamycin C gene cluster, is the condensation of the 3 amino acids α-aminoadipic acid, L-cystein and D-valine into the tripeptide d-(L-α-aminoadipyl)-L-cysteinyl-D-valine, catalyzed by the ACVS enzyme encoded by the *pcbAB* gene of *S. clavuligerus.*

Therefore, despite the above-described attempts for improving clavulanic acid production, in particular the clavulanic acid production by using microorganisms, there is still a need for an improved production method.

### Summary of the invention

The present invention provides the following aspects, subject-matters and preferred embodiments, which respectively taken alone or in combination, contribute to solving the object of the present invention:
(1) A process for preparing clavulanic acid by using a microorganism, comprising the steps of:
   a) providing a microorganism being capable of producing clavulanic acid, wherein said microorganism is modified or treated in that its enzyme d-(L-α-aminoadipyl)-L-cysteinyl-D-valine synthetase (ACVS) is selectively reduced or abolished,
   b) cultivating said modified or treated microorganism or its descendant to produce clavulanic acid, and
   c) isolating clavulanic acid.
   Within the meaning of the present invention, the term "microorganism" denotes any entity of microscopic or submicroscopic size that is capable of carrying on living processes, for instance bacteria.
   Within the meaning of the present invention, the term "microorganisms being capable of producing clavulanic acid" denotes microorganisms that have the capability of clavulanic acid production. In general, microorganisms that have the capability of producing clavulanic acid are in possession of the respective necessary cellular machinery, such as proteins, in particular enzymes, and genes for producing clavulanic acid. With respect to clavulanic acid, enzymes typically involved in the biosynthesis thereof are for instance N²-(2-carboxyethyl)arginine synthase, β-lactam synthetase, clavaminate synthase, proclavaminate amidino hydrolase, and clavulanate dehydrogenase. The microorganism typically has a cephamycin C pathway or a portion thereof, and according to the present invention it is preferred that only the conversion of L-α-aminoadipic acid, L-cysteine and D-valine to d-(L-α-aminoadipyl)-L-cysteinyl-D-valine of this cephamycin C pathway is interfered. Particularly preferred, the invention generally provides that the modified or treated microorganism, having both a clavulanic acid pathway or a portion thereof and a cephamycin C pathway or a portion thereof, has d-(L-α-aminoadipyl)-L-cysteinyl-D-valine synthetase (ACVS) activity specifically reduced or abolished, and a formation or accumulation of cephamycin C is thus substantially reduced or, more preferably, abolished. Within this preferred embodiment of the invention, "specifically reduced or abolished" means that no other enzyme other than ACVS typically involved in the cephamycin C pathway, such as L-lysine ε-aminotransferase (LAT), piperideine-6-carboxylate dehydrogenase (PCD), d-(L-α-aminoadipyl)-L-cysteinyl-D-valine, and/or isopenicillin N synthase (IPNS), is actively (by treatment) reduced or abolished, making this embodiment distinct from an unspecific treatment or modification to interfere with the cephamycin C pathway. More specifically, within the meaning of the present invention, the term "selectively" preferably denotes that, due to the modification or treatment of the microorganism, mainly the activity of ACVS is reduced or abolished, i.e. the treatment or modification of the microorganism aims at reducing or abolishing the activity of ACVS without significantly reducing the activity of LAT and IPNS activity. This means that the activity of LAT and IPNS is preferably not reduced by the modifying or treating by more than for example 30%, 20%, 10% or 5% compared to the activity before said modifying or treating. In further embodiments, regarding the "selective" reduction of ACVS, it is not relevant whether, besides ACVS, the activity of enzymes other than LAT or IPNS is reduced or enhanced.
   Clavulanic acid or (2R,5R,Z)-30(2 hydroxyethylidene) -7- oxo-4-oxa-I- azabicyclo [3.2.0] heptane-2-carboxylic acid, is produced by several microorganisms, for example *Streptomyces clavuligerus* ATCC 27064 (US4110165, BE827926), *Streptomyces jumonjinensis* (GB 1563103), *Streptomyces katsurahamanus* IFO 13716 FERM 3944 (JP83009679B) and *Streptomyces sp.* P6621 FERM 2804 (JP55162993A), without being limited thereto. The afore-mentioned microorganisms can for example be used within the present invention.
   The invention is based on the finding that interfering with the conversion of L-α-aminoadipic acid, L-cysteine and D-valine to d-(L-α-aminoadipyl)-L-cysteinyl-D-valine effectively blocks cephamycin C synthesis and surprisingly results in a significant increase of clavulanic acid production in a microorganism. As mentioned according to a preferred embodiment, such interference is specific for ACVS, with no interference with one or more enzymes (apart from ACVS) involved in cephamycin C pathway. Typical enzymes that are involved in cephamycin C pathway are for instance listed above.
   Further within the meaning of the present invention, the term "modifying" shall denote any kind of manipulation in or on the microorganism, in particular a manipulation of its genome and/or structure, wherein, after the manipulation has stopped, the microorganism remains modified. In particular, said modification can be provided, or passed over, to the descendants of the microorganism. A preferred type of modification is a manipulation of the microorganism by genetic engineering.
   The term "treating" shall denote any kind of manipulation in or on the microorganism that temporarily influences the metabolism of the microorganism, in particular the function of the proteins (e.g. enzymes) and genes thereof, wherein, after the manipulation has stopped, the microorganism is not influenced by the treatment any more. Rather, within a certain time point after the manipulation has stopped, it returns to a state that corresponds to the state prior to said manipulation. In particular, treating a microorganism can be carried out by providing certain substances via the cultivation medium. Furthermore, the term "being treated" is used in respect to a process wherein the step of treating and cultivating is at least partly carried out simultaneously.
   Within the meaning of the present invention, the term "reducing" denotes a decrease of ACVS activity in the modified or treated microorganism, compared to the ACVS activity in the unmodified and untreated microorganism as reference. The highest activity of the reference microorganism corresponds to 100% (a method for determining ACVS activity is described below).
   In general, the activity of ACVS (or any other enzyme, such as IPNS, or LAT) can be determined by any suitable method that is known to a person skilled in the art. In particular, it is possible to determine the activity of the respective enzyme by its capability to convert a substrate to a product. Such a method is for instance described in detail in Jhang *et al.* (1989) with regard to ACVS and IPNS activity, and with regard to the activity of LAT in Kern *et al.* (1980). A further method to determine the activity of the respective enzymes is to determine the production of cephamycin C. A reduced activity of for instance ACVS blocks the cephamycin C synthesis, which, in turn, leads to a reduced cephamycin C production.
   Within the meaning of the present invention, a suitable decrease of the ACVS activity is to such an extent in the modified or treated microorganism that said decreased activity leads to an increased production of clavulanic acid by the modified or treated microorganism, compared to the clavulanic acid production by an unmodified and untreated microorganism as reference (a method for determining ACVS activity is described below). The highest amount of clavulanic acid produced by the reference microorganism per time (e.g. at 150 hours) can be taken as a 100% reference.
   Therefore, the microorganism can be treated or modified in such a way that the reduction in ACVS activity in the treated or modified microorganism results in an increase of the amount of clavulanic acid (CA) produced. The extent of reduction of ACVS activity suitable for increasing the amount of produced CA may depend on various factors, such as the genus or species of microorganism used, or the cultivation conditions. A possible extent of reduction of activity of ACVS may for instance be at least 10%, at least 20%, at least 30%, at least 40%, or at least 50%, Further preferred, such a reduction may for instance be at least 60%, at least 70%, preferably at least 80% or more preferably at least 90%. In an even further preferred embodiment, the activity of ACVS is essentially or completely abolished. Further, the increase in produced amount of CA can be at least 2%, at least 10% or at least 20%. An upper limit of said increase may e.g. be 200% or 300%.
   The reference microorganism is the same type of microorganism that is modified or treated, however with the difference that the reference microorganism is not treated or modified. A modified or treated microorganism can for instance be a modified or treated *S*. *clavuligerus.* In this case, a corresponding reference microorganism is an unmodified and untreated wild type microorganism *S*. *clavuligerus.*
   Within the meaning of the present invention, the term "descendant" denotes the progenitors of the originally modified or treated microorganism. Further, a descendant can for instance also be a spore of the microorganism.
   By applying the method according to the present invention, advantageously it is only required to reduce or abolish the activity of one enzyme (compared to reducing or abolishing the activity of several enzymes, or reducing or abolishing enzymes in the cephamycin C pathway unspecifically), thereby leading to an improvement of the process of clavulanic acid production e.g. in terms of working time, or costs/expenses needed. Furthermore, the presence of undesired products, i.e. products other than CA in the cell culture, can be significantly reduced (in particular substances such as cephamycin C that are produced by the microorganism by using a pathway that is based on ACVS activity), therefore further contributing to a decrease of costs, e.g. the costs incurred with isolation and purification of CA from the cultivation broth. Furthermore, the targeted reduction or abolishing of only ACVS activity may be less time consuming compared to random mutagenesis processes that aim at manipulating the activity of several enzymes. In general, such mutagenesis processes are followed by selection techniques selecting the mutant that exhibits the desired effect.
   As mentioned above, in step a) a microorganism is provided, wherein said microorganism is capable of producing clavulanic acid (CA), and wherein said microorganism is modified or treated in that its enzyme ACVS is selectively reduced or abolished. In one embodiment, said microorganism is modified or treated during step a), and in another embodiment of the present invention, which is the preferred one, said microorganism provided in step a) is already modified in step a). The latter embodiment is for instance referred to in item (21).
(2) The process according to item (1), wherein the microorganism has L-lysine ε-aminotransferase (LAT) and/or isopenicilling N synthase (IPNS) activity which respectively is not reduced or abolished, and/or wherein, among the enzymes involved in the biosynthesis of cephamycin C, activity of ACVS alone is reduced or abolished.
   Enzymes other than ACVS that are involved in the cephamycin C pathway are known to a person skilled in the art. Examples of such enzymes are for instance L-lysine ε-aminotransferase (LAT), piperideine-6-carboxylate dehydrogenase (PCD), d-(L-α-aminoadipyl)-L-cysteinyl-D-valine, or isopenicillin N synthase (IPNS). The activity of the enzymes that are involved in the cephamycin C pathway, such as the activity of the above enzymes, is not significantly influenced by the modification or treatment. Methods of determining the activity of said enzymes are described for instance above in item (i), or below in the methods part.
   Reference is further made to the description of the preferred embodiments under item (1) above.
(3) The process according to item (1) or (2), wherein the modified or treated microorganism is a result of any one of
   (i) modifying or treating the enzyme compound ACVS,
   (ii) reducing the level of the enzyme ACVS, or completely preventing expression of a nucleic acid encoding ACVS in said microorganism, and
   (iii) modifying or treating the nucleic acid encoding the enzyme ACVS,
      respectively to reduce or abolish activity of ACVS.
(4) The process according to item (3), wherein
   (i) modifying or treating the enzyme compound ACVS comprises applying a component that partially or completely inhibits enzyme activity of ACVS,
   (ii) reducing the level of enzyme ACVS, or completely preventing expression of a nucleic acid encoding ACVS in said microorganism, comprises modifying regulatory elements of enzyme ACVS, for instance transcription and/or translation factors, or promoters, of enzyme ACVS, and
   (iii) modifying or treating the nucleic acid encoding the enzyme ACVS comprises introducing a mutation into said nucleic acid.
(5) The process according to item (4), wherein the component in (i) is a protein or peptide that interacts with said ACVS enzyme, preferably the component is an antibody; or the component reacts with the enzyme ACVS by modifying covalent bonds in said enzyme ACVS.
(6) The process according to item (4), wherein in (ii) reducing the level of enzyme ACVS, or completely preventing expression of a nucleic acid encoding ACVS in said microorganism comprises manipulating cis- or trans-located transcriptional and/or translational factors, such as transcriptional promoters, transcriptional terminators, ribosome binding site, initiation codons, termination codons, or translational attenuators.
(7) The process according to item (4), wherein in (iii) modifying or treating the nucleic acid encoding the enzyme ACVS comprises introducing mutation into the nucleic acid encoding the enzyme ACVS to that it cannot express, or that it can only express reduced levels of, the enzyme ACVS, preferably wherein introducing a mutation comprises deletion, insertion, substitution, and/or point mutation.
   In a specific embodiment, the complete gene encoding the enzyme ACVS is deleted.
(8) The process according to item (4), wherein in (ii) reducing the level of enzyme ACVS, or completely preventing expression of a nucleic acid encoding ACVS in said microorganism, comprises the use of antisense compounds, preferably of antisense oligonucleotides.
(9) The process according to any of the preceding items, wherein the microorganism of step a) belongs to the genus *Streptomyces* or the descendants thereof, preferably the microorganism is selected from the group consisting of *Streptomyces clavuligerus, Streptomyces clavuligerus, Streptomyces jumonjinensis, Streptomyces katsurahamanus* or the descendants thereof, more preferably the microorganism is *Streptomyces clavuligerus,* even more preferred the microorganism of step a) is *Streptomyces clavuligerus A TCC 27064* or *Streptomyces clavuligerus K213.*
(10) The process according to any of the preceding items (3) - (9), wherein the nucleic acid encoding the enzyme ACVS is the *pcbAB* gene.
(11) The process according to item (10), wherein modifying the nucleic acid encoding the enzyme ACVS comprises partially or completely deleting the *pcbAB* gene.
(12) The process according to any of the preceding items, wherein the enzyme ACVS to be modified or treated is encoded by the *pcbAB* gene, preferably the enzyme ACVS to be modified or treated is encoded by a nucleic acid sequence that has a sequence identity of at least 70%, preferably at least 80%, more preferably at least 85%, even more preferably at least 90% and most preferably at least 95%, 97% or 98.5% to the nucleic acid as listed under SEQ. ID. NO: 3.
   In a further embodiment, the enzyme ACVS is encoded by a nucleic acid sequence as listed under SEQ. ID. NO: 3. SEQ. ID. NO: 3 represents the coding region of the nucleic acid sequence encoding ACVS.
   In particular, the invention is based on the surprising finding that interfering with the function of d-(L-α-aminoadipyl)-L-cysteinyl-D-valine synthetase (ACVS) encoded by the *pcbAB* gene in *S*. *clavuligerus* results in an increase of clavam production in said microorganism.
   SEQ. ID. NO: 2 represents the amino acid sequence that is encoded by the coding region as depicted in SEQ. ID. NO: 1. SEQ. ID. NO: 1 depicts a nucleic acid sequence comprising primer binding sites, and a coding region of the *pcbAB* gene of *S*. *clavuligerus.*
(13) The process according to any of the preceding items, wherein prior to step c) spores of said modified or treated microorganism are prepared, preferably said spores are prepared on a sporulation medium.
(14) The process according to item (13), wherein the spores, the microorganism or its descendant are used to inoculate a liquid medium.
(15) The process according to any of the preceding items, wherein step b) comprises the use of said modified or treated microorganism as seed microorganism and further comprises a fermentation process of said seed microorganism.
(16) The process according to any of the preceding items, wherein the modified microorganism of step a) is stored in a frozen stock prior to cultivation in step b) and, optionally, step b) comprises the preparation of seed medium culture frozen stock.
(17) The process according to item (15), wherein the fermentation process comprises the inoculation of a culture broth with the modified or treated microorganism, preferably the inoculation is carried out by aseptical transfer of the microorganism into a bioreactor comprising the culture broth.
(18) The process according to item (17), wherein the culture broth is inoculated with the vegetative culture of the modified or treated microorganism.
(19) The process according to any of items (15) to (18), wherein the fermentation process is carried out in a bioreactor, preferably under agitation and/or aeration, preferably under submerged aerobic conditions in an aqueous nutrient medium containing sources of carbon, nitrogen, phosphate and minerals.
   The preferred sources of carbon in the nutrient media can selected from dextrin, starch, glycerol, maltose, sucrose or oil as exemplified below. The preferred sources of nitrogen in the nutrient media are yeast extract, soymeal protein isolates or concentrates, soybean meal, bacterial peptone, casein hydrolysate, ammonium sulphate or any of the proteinogenic aminoacids individually or in a mixture. Inorganic/mineral salts such as calcium carbonate, sodium chloride, sodium or potassium phosphate, magnesium, manganese, zinc, iron and other salts may also be added to the medium.
(20) The process according to any of items (15) to (19), wherein the fermentation process is carried out at a pH in the range of about 5.8 to 7.3, preferably 6.1 to 7.1 and at a temperature in the range of from 19°C to 30°C, preferably of from 21 °C to 29°C.
   Preferably, the production cultures of the fermentation process are incubated for 80 to about 300 hours, more preferably for about 130 to 280 hours.
(21) A process for preparing clavulanic acid by using a modified microorganism, comprising the steps of:
   A) providing a modified microorganism being capable of producing clavulanic acid, wherein
      aA) the *pcbAB* gene of said microorganism is selectively modified in that it has reduced or abolished activity of d-(L-α-aminoadipyl)-L-cysteinyl-D-valine synthetase (ACVS), or
      bA) said microorganism has selectively reduced or abolished activity of d-(L-α-aminoadipyl)-L-cysteinyl-D-valine synthetase (ACVS),
   B) cultivating said modified microorganism or its descendant to produce clavulanic acid, and
   C) isolating clavulanic acid.
   With regard to the term "selectively", as well as with regard to (preferred) embodiments, reference is made to the definition elsewhere herein.
(22) The process according to item (21), wherein the modified microorganism has L-lysine ε-aminotransferase (LAT) and/or isopenicilling N synthase (IPNS) activity which respectively is not reduced or abolished, and/or wherein, among the enzymes involved in the biosynthesis of cephamycin C, activity of ACVS alone is reduced or abolished.
   Reference is further made to the description of (preferred) embodiments under items (1) and (2) above.
(23) The process according to items (21) or (22), wherein said modified microorganism is defined as in any one of items (36) to (42).
(24) The process according to any of items (21) to (23), wherein prior to step B) spores of said modified microorganism are prepared, preferably said spores are prepared on a sporulation medium.
(25) The process according to item (24), wherein the spores, the microorganism or its descendant are used to inoculate a liquid medium.
   With regard to the term "descendant", reference is made to the definition elsewhere herein.
(26) The process according to any of items (21) to (25), wherein step B) comprises the use of said modified microorganism as seed microorganism and further comprises a fermentation process of said seed microorganism.
(27) The process according to any of items (21) to (26), wherein the modified microorganism of step A) is stored in a frozen stock prior to cultivation in step B) and, optionally, step B) comprises the preparation of seed medium culture frozen stock.
(28) The process according to item (26) or (27), wherein the fermentation process comprises the inoculation of a culture broth, preferably the culture broth is a production medium, with the modified microorganism, preferably the inoculation is carried out by aseptical transfer of the microorganism into a bioreactor comprising the culture broth.
   The process step that takes place in a production medium is herein also referred to as "main fermentation process".
(29) The process according to item (28), wherein the culture broth is inoculated with the vegetative culture of the modified microorganism.
(30) The process according to any of items (26) to (29), wherein the fermentation process is carried out in a bioreactor, preferably under agitation and/or aeration, preferably under submerged aerobic conditions in an aqueous nutrient medium containing sources of carbon, nitrogen, phosphate and minerals.
   The preferred sources of carbon in the nutrient media can selected from dextrin, starch, glycerol, maltose, sucrose or oil as exemplified below. The preferred sources of nitrogen in the nutrient media are yeast extract, soymeal protein isolates or concentrates, soybean meal, bacterial peptone, casein hydrolysate, ammonium sulphate or any of the proteinogenic aminoacids individually or in a mixture. Inorganic/mineral salts such as calcium carbonate, sodium chloride, sodium or potassium phosphate, magnesium, manganese, zinc, iron and other salts may also be added to the medium.
(31) The process according to any of items (26) to (30), wherein the fermentation process is carried out at a pH in the range of about 5.8 to 7.3, preferably 6.1 to 7.1 and at a temperature in the range of from 19°C to 30°C, preferably of from 21 °C to 29°C.
   Preferably, the production cultures of the fermentation process are incubated for 80 to about 300 hours, more preferably for about 130 to 280 hours.
(32) A recombinant plasmid comprising a *pcbAB* gene being modified to encode partially or completely inactivated enzyme ACVS.
(33) The recombinant plasmid according to item (32), wherein the *pcbAB* gene has been modified by introducing a mutation, preferably by introducing deletion, insertion, substitution and/or point mutation, to thereby partially or completely inactivate the encoded enzyme ACVS.
   In a preferred embodiment, the complete gene encoding ACVS is deleted.
(34) The recombinant plasmid according to items (32) and (33), wherein the enzyme ACVS to be modified or treated is encoded by the *pcbAB* gene, preferably the enzyme ACVS to be modified or treated is encoded by a nucleic acid sequence that has a sequence identity of at least 70%, preferably at least 80%, more preferably at least 85%, even more preferably at least 90% and most preferably at least 95%, 97% or 98.5% to the nucleic acid encoding ACVS as depicted in SEQ. ID. NO: 3.
(35) The recombinant plasmid according to any of items (32) to (34), wherein the *pcbAB* gene comprises the nucleic acid sequence as depicted in SEQ. ID. NO: 3.
(36) A modified microorganism comprising the recombinant plasmid according to any of items (32) to (35).
(37) A microorganism that is modified or treated by selectively reducing or abolishing activity of its d-(L-α-aminoadipyl)-L-cysteinyl-D-valine synthetase (ACVS).
   With regard to the term "selectively", reference is made to the definition elsewhere herein.
(38) The microorganism according to item (37), wherein the microorganism is modified or treated by applying step a) as defined in any of items (1) to (12).
(39) The microorganism according to item (36) to (38), which has L-lysine ε-aminotransferase (LAT) and/or IPNS enzyme activity which is not reduced or abolished, and/or wherein, among the enzymes involved in the biosynthesis of cephamycin C, activity of ACVS alone is reduced or abolished.
(40) The microorganism according to any of items (37) to (39) comprising the recombinant plasmid according to any of items (21) to (23).
(41) The microorganism according to any of items (36) to (40), having the modified *pcbAB* gene in its genome.
(42) The microorganism according to any of items (36) to (40), wherein said microorganism belongs to the genus *Streptomyces* or the descendants thereof, preferably the microorganism is selected from the group consisting of *Streptomyces clavuligerus, Streptomyces clavuligerus, Streptomyces jumonjinensis, Streptomyces katsurahamanus* or the descendants thereof, more preferably the microorganism is *Streptomyces clavuligerus,* even more preferred the microorganism is *Streptomyces clavuligerus* ATCC 27064 or *Streptomyces clavuligerus* K213.
(43) Use of the recombinant plasmid according to any of items (32) to (35) for modifying a microorganism.
(44) Use of the microorganism according to any of items (36) to (42) for the production of clavulanic acid.
(45) The process according to any of items (1) to (31), wherein, in a further step, the prepared clavulanic acid, which optionally is converted to a pharmaceutically acceptable salt or derivative thereof, is mixed with a suitable pharmaceutically acceptable carrier or excipient to prepare a pharmaceutical composition containing clavulanic acid.

### Detailed description of the invention

The present invention is now described in more detail by preferred embodiments and examples, which are however presented for illustrative purpose only and shall not be understood as limiting the scope of the present invention in any way.

All patent applications, patents and literature references cited herein are hereby incorporated by reference in their entirety. In practicing the present invention, many conventional techniques in molecular biology, microbiology, and recombinant DNA are used. These techniques are well known and are explained in, for example, Short Protocols in Molecular Biology, Third Edition, 1995 (F. M. Ausubel ed.), John Wiley & Sons, Inc.; Sambrook and Russell, 2001, Molecular Cloning: A Laboratory Manual, Third Edition, Cold Spring Harbor Laboratory Press, Cold Spring Harbor, N.Y.; Kieser et al., 2000, Practical Streptomyces Genetics, The John Innes Foundation, Norwich, GB and Spreptomyces: Molecular biology and Biotechnology, 2011 (P. Dyson ed.) Caister Academic Press, Norfolk, UK.

The present invention provides an improved process for the production of clavulanic acid (CA), with the proviso that a microorganism, which is capable of producing CA and which produces d-(L-α-aminoadipyl)-L-cysteinyl-D-valine synthetase (ACVS) is modified or treated in order to selectively reduce or abolish the ACVS activity. It has been surprisingly found that when using said microorganisms, the selective reduction or abolishing of ACVS activity is particularly advantageous for producing CA. The process according to the present invention can for instance be improved with regard to process time and costs. By selectively targeting the ACVS activity only, additional steps of (further) manipulating for instance the genome of the microorganism can be prevented, therefore saving time and costs that may be involved by such additional steps.

Additionally, by applying the process according to the present invention, the produced product can have an improved quality. This improved quality can further contribute to time and cost savings, as (further) purification and isolation steps that otherwise might have been necessary can be reduced or do not have to be carried out any more. In particular, the presence of undesired metabolites, such as cephamycin C, can be diminished, or even completely abolished. This is all the more advantageous if the clavulanic acid is intended to be used in the field of pharmaceutics, e.g. in human medicine: In order to meet the respective quality standards in this field, less time and money has to be spent compared to the time and money that has to be spent when using conventional production processes. Furthermore, it has been found that by applying the process according to the present invention, the yield of clavulanic acid can be improved. It is all the more surprising that even if strains are used that have a history in strain development (also know as high-producer strains, that for instance have many years of random mutagenesis and selection cycles), the yield in CA can be improved.

Without wishing to be bound by any theory, it is assumed that improvements of the inventive process can be attributed to the fact that due to the reduction or abolishing of ACVS activity, the synthesis of side products such as cephamycin C, clavams, or others, is reduced, or even abolished. Therefore, the metabolites and energy needed to produce these competing compounds, which are normally eating up the resources at the expense at the product CA, are reduced. Therefore, more energy and nutrient flow is directed to the biosynthesis of CA, which, in turn, leads to an improved yield in CA.

It has now surprisingly been found out that the reduction or abolishing of ACVS activity and subsequently reduction of the amount of cephamycin C that is produced by the microorganism, or even the complete absence of cephamycin leads to an increase of the yield of CA although aminoadipic acid is still produced at an unchanged rate.

Facing the fact that the biochemical pathways that are involved in the production of primary and secondary metabolites are more or less inter-regulated and connected to each other, this is all the more surprising to a person skilled in the art, as the reduction or abolishment of cephamycin C by selectively reducing or abolishing the ACVS activity in the specific microorganism can not be expected to result in improved clavulanic acid titers as, for instance, the synthesis of α-aminoadipic acid is not interfered with. In fact, it has been found that α-aminoadipic acid is accumulating extracellularly in S. *clavuligerus* cultures, and this extracellular pool is not reduced later in the cultivation time despite the observation that intracellular pool of α-aminoadipic acid is decreased already after the early stage of cultivation (Madduri *et al.,* 1991). In this case, a person skilled in the art would expect that, even with the inactivation of the ACVS activity (for instance by inactivation of ACVS gene *(pcbAB),* and reduction or, preferably, abolishment of cephamycin C production), the nutrients and energy would still be used for synthesis of α-aminoadipic acid at the expense of CA titers, and the extracellularly accumulated α-aminoadipic acid would be lost to the microorganism as a nutrient source. Despite the above, it has now surprisingly been found that reducing or abolishing the activity of AVCS in a specific microorganism reduces or preferably completely abolishes synthesis of cephamycin C and, in addition, also improves production of clavulanic acid despite its preserved ability to synthesize α-aminoadipic acid.

Thus, the present invention relates to a process for preparing clavulanic acid by using a microorganism, comprising the steps of:
a) providing a microorganism being capable of producing clavulanic acid, wherein said microorganism is modified or treated in that its enzyme d-(L-α-aminoadipyl)-L-cysteinyl-D-valine synthetase (ACVS) is selectively reduced or abolished,
b) cultivating said modified or treated microorganism or its descendant to produce clavulanic acid, and
c) isolating clavulanic acid.

In step (a), a microorganism is chosen, wherein the microorganism can be a commercially available microorganism or a microorganism that has been genetically engineered to have desired properties such as for example a high rate of reproduction.

A preferred microorganism for carrying out the present invention is a microorganism belonging to the group of clavulanic acid producing organisms. A suitable microorganism may therefore be selected from *Streptomyces clavuligerus* ATCC 27064, *Streptomyces clavuligerus* NRRL 3585, *Streptomyces jumonjinensis, Streptomyces katsurahamanus* IFO 13716 FERM 3944, *Streptomyces sp.* P6621 FERM 2804 and the descendants of said microorganisms. In a preferred aspect of the invention, *Streptomyces clavuligerus* ATCC 27064, *Streptomyces clavuligerus* NRRL 3585 and their descendants may be used. *Streptomyces clavuligerus* ATCC 27064 and its descendants are particularly suitable.

Additionally preferred, the microorganism has L-lysine ε-aminotransferase (LAT) and/or isopenicilling N synthase (IPNS) activity which respectively is not reduced and/or abolished in step (a), and/or wherein, among the enzymes involved in the biosynthesis of cephamycin C, activity of ACVS alone is reduced or abolished.

Additionally preferred, the microorganism of step a) belongs to the genus *Streptomyces,* preferably the microorganism is selected from the group consisting of *Streptomyces clavuligerus, Streptomyces jumonjinensis, Streptomyces katsurahamanus,* or the descendants thereof, more preferably the microorganism is *Streptomyces clavuligerus,* even more preferred the microorganism of step a) is *Streptomyces clavuligerus ATCC 27064* or *Streptomyces clavuligerus K213.*

In order to identify microorganisms other than the above that are suitable within the present invention, potential microorganisms can be tested to determine their capability of producing clavulanic acid and ACVS (see methods described elsewhere herein).

In step (a), the microorganism is modified or treated in order to selectively reduce or abolish activity of said d-(L-α-aminoadipyl)-L-cysteinyl-D-valine synthetase (ACVS).

In general, the ACVS activity is reduced or abolished by interfering with the function of the enzyme ACVS which catalyses the conversion of L-α-aminoadipic acid, L-cysteine and D-valine to d-(L-α-aminoadipyl)-L-cysteinyl-D-valine or by preventing production of ACVS. Interfering with the function of the enzyme may be on the protein or nucleic acid level. It is to be understood that in the context of the present invention, a complete inactivation of the enzyme or gene function is preferred, but not necessary. Also a partial inactivation of the function will result in an increased amount of produced CA, albeit not to the extent as in case of a complete inactivation. Said interfering with the ACVS can end if the treatment with the interfering agent ends and for example "fresh", or new, ACVS having full activity is produced by the microorganism and replenishes the active enzyme level. If the microorganism is modified in step (a), the ACVS activity will not increase after modification has finished.

In step (b), said modified or treated microorganism or its descendant is cultivated to - finally - produce clavulanic acid. Cultivation of the microorganism can be carried out by methods known to a person skilled in art. Cultivation processes of *Streptomyces clavuligerus* are for example described in WO0005397, US6100052, WO 9739137, and others.

Preferably, the cultivation step (b) comprises a main fermentation process and optionally a step of producing a culture of seed microorganisms prior to the main fermentation process. The main fermentation process is carried out in a production medium. The seed microorganisms are present in a seed medium.

Preferably the main fermentation process in step (b) is carried out in a bioreactor, in particular under agitation and/or aeration. In a preferred embodiment, the process for the production of clavulanic acid is carried out under submerged aerobic conditions in an aqueous nutrient medium (production medium), containing sources of assimilable carbon, nitrogen, phosphate and minerals.

The main fermentation process can be carried out according to any suitable method that is known to a person skilled in the art. In general, the main fermentation process comprises the inoculation of production medium with a culture of the microorganism of step a), in particular a culture of seed microorganism. Inoculation can for example be carried by aseptical transfer into the bioreactor (also referred to herein as "reactor"). The seed culture can for example be prepared from a frozen stock, in particular a seed medium culture frozen stock. A seed medium culture frozen stock refers to a microorganism grown in a seed medium and stored as a frozen vial to be used later as an inoculum for further cultivation. It is preferred to employ the vegetative culture of the microorganism for inoculation. Further, the seed culture can also be prepared from spores of the modified or treated microorganism. The spores are prepared according to any suitable method that is known to a person skilled in the art, for instance the modified or treated microorganism is cultivated on a sporulation medium. The obtained spores are used to inoculate a liquid medium, for example a seed medium. After cultivation, the culture broth can be stored as a frozen stock by freezing below -20°C in an appropriate vial.

The addition of nutrient medium (production medium) in the main fermentation process into the bioreactor can be carried out once or more, batch-wise or in a continuous way. Addition of nutrient medium (production medium) can be carried out before and/or during the fermentation process. The preferred sources of carbon in the nutrient media can be selected from dextrin, starch, glycerol, maltose, sucrose or oil as exemplified below. The preferred sources of nitrogen in the nutrient media are yeast extract, soymeal protein isolates or concentrates, soybean meal, bacterial peptone, casein hydrolysate, ammonium sulphate or any of the proteinogenic aminoacids individually or in a mixture. Inorganic/mineral salts such as calcium carbonate, sodium chloride, sodium or potassium phosphate, magnesium, manganese, zinc, iron and other salts may also be added to the medium.

The main fermentation process in step (b) can be carried out at a pH in the range of about 5.8 to 7.3 and temperature in the range of 19 to 30 °C. Preferably, the pH is in the range of about 6.1 to 7.1 and the temperature is preferably in the range of about 21 to 29 °C. Preferably, the production cultures are incubated for 80 to about 300 hours, more preferably for about 130 to 280 hours.

The production of clavulanic acid may be performed in aerobic conditions with agitation and aeration of production medium. Agitation and aeration of the culture mixture may be accomplished in a variety of ways, which are known to a person skilled in the art. The agitation of production medium may be provided by a propeller or similar mechanical device and varied to various extents according to fermentation conditions and scale. The aeration rate can be varied in the range of 1.0 to 2.5 VVM (gas volume flow per unit of liquid volume per minute (volume per volume per minute)) with respect to the working volume of the bioreactor.

Further, known additives for the fermentative process may be added, in particular in the main fermentation process. To prevent excessively foaming of the culture medium, anti-foaming agents may be added, such as silicone oil, fatty oil, plant oil and the like. Particularly, a silicone-based anti-foaming agent may be added during the fermentation process to prevent excessively foaming of the culture medium.

The isolation of clavulanic acid in step (c) can be carried out by using suitable techniques that are known to a person skilled in the art. Clavulanic acid from the fermentation broth can be separated and purified by conventional methods commonly used for recovery of biologically active substances. A number of methods used for recovery of clavulanic acid from fermentation froth and subsequent conversion to pharmaceutically acceptable salts of clavulanic acid is for example described in Saudagar *et al.,* 2008.

In the process according to the present invention, modifying or treating the microorganism is selected from (i) modifying or treating the enzyme compound ACVS, (ii) reducing the level of the enzyme ACVS, or completely preventing expression of a nucleic acid encoding the enzyme ACVS in said microorganism, and (iii) modifying or treating the nucleic acid encoding the enzyme ACVS, respectively to reduce or abolish activity of ACVS.

There are several different methodical approaches known in the art describing interference with function (or activity, respectively) of proteins/enzymes as in step (i) above (i.e. modifying or treating the enzyme compound ACVS). For instance, compounds or chemicals may be used that specifically inhibit the protein function, such as specific antibodies or other proteins or peptides that bind to or chemically modify the protein to be (partially or completely) inactivated. Within the meaning of the present invention, the term "inactivated" always refers to a partial or complete inactivation of the protein function or activity, respectively. Also, chemical modification of the protein structure affecting its function may be performed. As mentioned above, said inhibition may be only partial, thus leaving some residual protein activity. Thus, by treating the microorganism with proteins, peptides, antibodies, or any other agents, e.g. inorganic compounds, that bind to or react with the ACVS, a reduced or abolished ACVS activity may be resulted.

A further way of modifying or treating a microorganism, as referred to in step (ii) above, is to interfere with or destabilize nucleic acids such as DNA or the messenger RNA encoding said enzyme, thereby reducing or even abolishing expression of ACVS and thus reducing or even abolishing ACVS activity by reducing the level of ACVS which is present in the microorganism. For instance, antisense compounds, particularly antisense oligonucleotides, may be used to inhibit the expression of nucleic acid molecules. These antisense compounds specifically hybridize with the nucleic acids and prevent them from fulfilling their function, i.e. being transcribed into mRNA in case of DNA or translated into a protein in case of mRNA. It is known to a person skilled in the art how the sites of the coding region of the protein whose activity is intended to be reduced or abolished have to be determined. In general, any region in the coding region can be used to design antisense RNA for silencing.

Another approach of modifying or treating a microorganism, as referred to in step (ii) above, can be undertaken by manipulating expression levels of said protein by interfering with transcriptional and/or translational factors regardless the factors being *cis* or *trans.* Non limiting examples of such factors are transcriptional promoters or parts thereof, transcriptional terminators and other cis located transcriptional signals. An equally successful approach would be to manipulate translational cis factors such as ribosome binding site, initiation and termination codons, translational attenuator and other translational *cis* elements. Another method is to manipulate the function of *trans* located transcription and translation factors, such as specific transcriptional regulators and translation initiation factors. Alternatively, as referred to in step (iii) above, the nucleic acid encoding the enzyme ACVS can be modified or treated. Thereby, the function of the nucleic acids may be modified by way of mutation of the nucleic acid sequence thereby resulting in a protein with no or reduced activity. By introducing mutations to the sequence, either the translation of the protein may be completely blocked or a mutated protein with corresponding mutations in its amino acid sequence will result. Such a protein will either be completely inactive or will display a reduced functional activity. It is well within the general knowledge of a person skilled in the art to select for those mutations that will have the desired effect. Merely established standard technology that is known to a person skilled in the art is required to generate such mutated nucleic acids and to select for mutations suitable in the context of the present invention. As used herein, the term "mutation" encompasses any change in the nucleic acid sequence such as deletions, insertions, substitutions and point mutations. Single nucleotides may be changed, but also larger portions of the sequence or the complete sequence may be affected. In a preferred embodiment of the invention, such mutation is introduced into the gene *pcbAB* encoding ACVS according to the nucleic sequence SEQ. ID. NO. 1, preferably into the coding region of *pcbAB* as depicted in SEQ. ID. NO: 3. In an even more preferred embodiment, the gene encoding ACVS is completely deleted, and most preferred, SEQ. ID. NO: 3 is completely deleted.

Methods of inactivating genes in microorganisms, e.g. as referred to in step (iii) above, are well known in the art, for example, a method is described in the handbook "Practical *Streptomyces* genetics" (Kieser *et al.,* 2000, Practical *Streptomyces* genetics, A Laboratory Manual. ISBNO-7084-0623-8). A similar but more preferred method which includes use of both positive and negative selection markers was reported recently (Dubeau, *et al.,* 2009). Both methods are based on homologous recombination mechanisms which, in the outcome of double recombination, results in exchange of the target sequence in the genome for the sequence introduced externally and positioned between the homologous regions. Therefore, preferably homologous sequences flanking the region to be modified in the genome are used. These regions are selected according to the desired effect. For instance for deletion, the sequences are positioned distant to each other for the fragment length which is targeted for deletion. The above mentioned homologous sequences can be suitably obtained by conventional cloning methods (such as PCR, polymerase chain reaction) based on the published sequence.

By applying the above methods, the ACVS activity can selectively be reduced or abolished, which results in an increased CA production per time compared to the CA production of an unmodified or untreated microorganism. For example, the increase in produced amount of CA per time (e.g. 100 hours, 150 hours or 200 hours) can be at least 2%, at least 10%, at least 20%, at least 50% or at least 65%. An upper limit of said increase may e.g. be 200% or 300%. The value "100%" corresponds to the relative amounts of clavulanic acid and cephamycin C given as % wherein the highest amounts produced by the unmodified and untreated reference organism are nominated as 100%.

Enzymes other than ACVS that are involved in the cephamycin C pathway are known to a person skilled in the art. Examples of such enzymes are for instance L-lysine ε-aminotransferase (LAT), piperideine-6-carboxylate dehydrogenase (PCD), d-(L-α-aminoadipyl)-L-cysteinyl-D-valine, or isopenicillin N synthase. The activity of the enzymes that are involved in the cephamycin C pathway, such as the activity of the above enzymes, is not significantly influenced by the modification or treatment.

Therefore, following from the above, in a preferred embodiment,
(i) modifying or treating the enzyme compound ACVS comprises applying a component that partially or completely inhibits enzyme activity of ACVS,
(ii) reducing the level of enzyme ACVS, or completely preventing expression of ACVS in said microorganism, comprises modifying regulatory elements of enzyme ACVS, for instance transcription and/or translation factors, or promoters, of enzyme ACVS, and
(iii) modifying or treating the nucleic acid encoding the enzyme ACVS comprises introducing a mutation into said nucleic acid.

In a preferred embodiment, the component in (i) is a protein or peptide that interacts with said ACVS enzyme, preferably the component is an antibody; or the component reacts with the enzyme ACVS by modifying covalent bonds in said enzyme ACVS. Additionally preferred, reducing the level of enzyme ACVS in step (ii), or completely preventing expression of a nucleic acid encoding ACVS in said microorganism, comprises introducing a mutation into the nucleic acid encoding the enzyme ACVS to that it cannot express, or that it can only express reduced levels of, the enzyme ACVS. Introducing a mutation into the nucleic acid encoding the enzyme ACVS comprises deletions, insertions, substitutions, and/or point mutations. The introduction of mutations is a standard technique that is known to a person skilled in the art.

In a further preferred embodiment, modifying or treating the nucleic acid encoding the enzyme ACVS in step (iii) comprises the use of antisense compounds, preferably of antisense oligonucleotides. The generation of antisense oligotechniques, in particular the technique of "silencing" nucleic acid molecules is known to a person skilled in the art. In general, any site in the coding region of the gene *pcbAB* encoding ACVS can be used to design antisense compounds such as antisense oligonucleotides.

Further preferred, modifying the nucleic acid encoding the enzyme ACVS comprises partially or completely deleting the *pcbAB* gene. Particularly preferred, a method is provided wherein the conversion of L-α-aminoadipic acid, L-cysteine and D-valine to d-(L-α-aminoadipyl)-L-cysteinyl-D-valine is interfered with by mutating the *pcbAB* gene. In a preferred embodiment, said mutation is disrupting or eliminating the *pcbAB* gene or a part thereof.

In a further preferred embodiment, the enzyme ACVS produced by the microorganism of step a) (i.e. prior to a modification and/or treatment) is encoded by a nucleic acid sequence that has a sequence identity of at least 70%, preferably at least 80%, more preferably at least 85%, even more preferably at least 90% and most preferably at least 95%, 97% or 98.5% to the nucleic acid as listed under SEQ. ID. NO: 3. In a further embodiment, the enzyme ACVS produced by the microorganism of step a) is encoded by a nucleic acid sequence that comprises to the coding region of the nucleic acid as listed under SEQ. ID. NO: 3.

Further preferred, prior to step c), spores of said modified or treated microorganism, preferably a strain of *S*. *clavuligerus* as defined elsewhere herein, are prepared. The spores can be prepared according to any suitable method that is known to a person skilled in the art, preferably said spores are prepared on a sporulation medium, e.g. on a sporulation medium as described herein. Said spores, the microorganism or its descendant can then be used to inoculate a liquid medium.

Further preferred, step b) comprises the use of said modified or treated, preferably genetically modified, microorganism, as seed microorganism and further comprises a fermentation process of said seed microorganism.

Additionally preferred, the microorganism of step a) is stored in a frozen stock, preferably by freezing below -20°C in an appropriate vial, prior to cultivation in step b). Optionally, step b) comprises the preparation of seed medium culture frozen stock.

In principle, the cultivation of seed microorganism can be carried out under the conditions (e.g. pH and temperature) similar to the main fermentation process (described under step b)).

Preferably, the production of seed microorganism (which can for instance be used in the main fermentation process) for the production of clavulanic acid starts from a frozen stock of said modified microorganism. In this respect, the process according to the present invention comprises the preparation of frozen stock of modified microorganism, preferably of *Streptomyces clavuligerus,* and optionally seed medium culture frozen stock of modified microorganism. This preparation of inoculum may be carried out using methods known in the state of art. Preferably this frozen stock of modified strain of microorganism is used to produce a vegetative or seed culture by inoculation to a seed medium. The production of vegetative culture of described microorganism should start with inoculation of a relatively small quantity of seed medium with the frozen stock.

Additionally preferred, the fermentation process comprises the inoculation of a culture broth with the modified or treated microorganism, preferably with the vegetaticve culture of said microorganism. The inoculation can be carried out by any suitable method that is known to a person skilled in the art; in a preferred embodiment, the inoculation is carried out by aseptical transfer of the microorganism into a bioreactor comprising the culture broth.

Additionally preferred, the fermentation process is carried out in a bioreactor, preferably under agitation and/or aeration, preferably under submerged aerobic conditions in an aqueous nutrient medium containing sources of carbon, nitrogen, phosphate and minerals. The preferred sources of carbon in the nutrient media can be selected from dextrin, starch, glycerol, maltose, sucrose or oil as exemplified below. The preferred sources of nitrogen in the nutrient media are yeast extract, soymeal protein isolates or concentrates, soybean meal, bacterial peptone, casein hydrolysate, ammonium sulphate or any of the proteinogenic aminoacids individually or in a mixture. Inorganic/mineral salts such as calcium carbonate, sodium chloride, sodium or potassium phosphate, magnesium, manganese, zinc, iron and other salts may also be added to the medium.

The fermentation process can be carried out at a pH in the range of about 5.8 to 7.3, preferably 6.1 to 7.1, and at a temperature in the range of from 19°C to 30°C, preferably of from 21 °C to 29°C.

The production culture of the fermentation process can be incubated for 80 to about 300 hours, preferably for about 130 to 280 hours.

Furthermore, the invention relates to a process for preparing clavulanic acid by using a modified microorganism, comprising the steps of:
A) providing a modified microorganism being capable of producing clavulanic acid, wherein
   aA) the *pcbAB* gene of said microorganism is modified in that it has selectively reduced or abolished activity of d-(L-α-aminoadipyl)-L-cysteinyl-D-valine synthetase (ACVS), or
   bA) the microorganism has selectively reduced or abolished activity of d-(L-α-aminoadipyl)-L-cysteinyl-D-valine synthetase (ACVS),
B) cultivating said modified microorganism or its descendant to produce clavulanic acid, and
C) isolating clavulanic acid.

The term "modified microorganisms" and preferred modified microorganisms are described above. Modified microorganisms that are used in step (A) have a higher capacity of producing CA compared to their unmodified and untreated form.

Additionally preferred, the modified microorganism has L-lysine ε-aminotransferase (LAT) and/or isopenicillin N synthase (IPNS) activity which respectively is not reduced or abolished, and/or wherein, among the enzymes involved in the biosynthesis of cephamycin C, activity of ACVS alone is reduced or abolished. With regard to (preferred) embodiments and definitions, reference is made to the description elsewhere herein.

In a preferred embodiment, the modified microorganism is derived from *Streptomyces clavuligerus* ATCC 27064, *Streptomyces clavuligerus* NRRL 3585, *Streptomyces jumonjinensis, Streptomyces katsurahamanus* IFO 13716 FERM 3944, and *Streptomyces sp.* P6621 FERM 2804 by modification as described herein. In this case, a "modified microorganism" can be identified (by testing activity of ACVS, LAT and IPNS) as a microorganism having a reduced ACVS activity compared to the respective unmodified microorganism from the group of *Streptomyces clavuligerus* ATCC 27064, *Streptomyces clavuligerus* NRRL 3585, *Streptomyces jumonjinensis, Streptomyces katsurahamanus* IFO 13716 FERM 3944, and *Streptomyces sp.* P6621 FERM 2804, while not having a significantly reduced LAT and/or IPNS activity. "Significantly" means that the activity of LAT and/or IPNS activity is not reduced by more than 30%, or more than 20%, or more than 10% or more than 5%. Preferably, the activity of LAT and/or IPNS is not reduced. Thus, it is determined from which microorganism a potential "modified microorganism" is derived from and then a comparison between the potential modified microorganism and the reference microorganism is made.

Other preferred modified microorganisms are described above.

In a further preferred embodiment, prior to step B) spores of said modified microorganism are prepared. The preparation of spores is described elsewhere herein. Said spores, the microorganism or its descendant are used to inoculate a liquid medium, which is also described elsewhere herein.

Additionally preferred, step B) comprises the use of said modified microorganism as seed microorganism and further comprises a fermentation process of said seed microorganism. In a preferred embodiment, the fermentation process comprises the inoculation of a culture broth with the modified microorganism, preferably the inoculation is carried out by aseptical transfer of the modified microorganism into a bioreactor comprising the culture broth. In a preferred embodiment, the culture broth is inoculated with the vegetative culture of the modified microorganism.

The modified microorganism of step A) can be stored in a frozen stock prior to cultivation in step B) and, optionally, step B) comprises the preparation of a seed medium culture frozen stock.

Further preferred, the fermentation process is carried out as described above. With regard to the media used, in particular with regard to the preferred sources of carbon and nitrogen in the nutrient media, as well as with regard to the inorganic/mineral salts, reference is made to the specification above.

Furthermore, the production cultures of the fermentation process can be incubated for 80 to about 300 hours, more preferably for about 130 to 280 hours.

In further aspects, the invention provides a recombinant plasmid containing a mutated *pcbAB* gene with partially or completely inactivated function and a microorganism having both a clavulanic acid pathway or a portion thereof and a cephamycin C pathway or a portion thereof and containing such a recombinant plasmid.

Thus, the invention relates to a recombinant plasmid comprising a *pcbAB* gene being modified to encode partially or completely inactivated enzyme ACVS. With regard to the term "modified", as well as with regard to the aspect how a gene can be modified, reference is made to the description above. Therefore, the *pcbAB* gene has preferably been modified by introducing a mutation, preferably by introducing deletion, insertion, substitution and/or point mutation, to thereby partially or completely inactivate the encoded enzyme ACVS.

Additionally preferred, the *pcbAB* gene comprises a nucleic acid sequence that has a sequence identity of at least 70%, preferably at least 80%, more preferably at least 85%, even more preferably at least 90% and most preferably at least 95%, 97%, 98.5% or even 100% to the coding region of the nucleic acid as listed under SEQ. ID. NO: 3.

Furthermore, the present invention provides genetically modified microorganisms having both a clavulanic acid pathway or a portion thereof and a cephamycin C pathway or a portion thereof wherein the conversion of conversion of L-α-aminoadipic acid, L-cysteine and D-valine to d-(L-α-aminoadipyl)-L-cysteinyl-D-valine is interfered with by mutating the *pcbAB* gene as described above and wherein the production of clavulanic acid is increased. In a preferred embodiment said microorganism belongs to *Streptomyces,* more preferably the said microorganism is *Streptomyces clavuligerus.*

The invention also relates to a modified microorganism comprising the recombinant plasmid as described above.

The invention further refers to a microorganism that is modified or treated by selectively reducing or abolishing activity of said d-(L-α-aminoadipyl)-L-cysteinyl-D-valine synthetase (ACVS), preferably the microorganism is modified or treated by applying step b) as defined above. Additionally preferred, said microorganism has L-lysine ε-aminotransferase (LAT) and/or IPNS enzyme activity which is not significantly reduced or abolished and/or wherein, among the enzymes involved in the biosynthesis of cephamycin C, activity of ACVS alone is reduced or abolished. Additionally preferred, the microorganism comprises the modified *pcbAB* gene as described elsewhere herein, and/or said microorganism belongs to the genus *Streptomyces* or the descendants thereof. Preferably the microorganism is selected from the group consisting of *Streptomyces clavuligerus, Streptomyces clavuligerus, Streptomyces jumonjinensis, Streptomyces katsurahamanus* or the descendants thereof, more preferably the microorganism is *Streptomyces clavuligerus,* even more preferred the microorganism is *Streptomyces clavuligerus* ATCC 27064 or *Streptomyces clavuligerus* K213.

The invention also refers to the use of the recombinant plasmid according described herein for modifying a microorganism. Additionally preferred, the microorganism described herein is used for the production of clavulanic acid.

It is additionally preferred in the process described herein to carry out a further step, wherein the prepared clavulanic acid is converted to a pharmaceutically acceptable salt or derivative thereof.

### Methods

### 1. Production of clavulanic acid

The production of clavulanic acid can be determined and quantified by any suitable method that is known to a person skilled in the art, such as chromatographic methods, preferably HPLC (high performance liquid chromatography) or HPLC-MS (mass spectroscopy).

The capacity of a microorganism regarding the production of clavulanic acid can be compared with the capacity of another microorganism by cultivating both microorganisms in same amounts under same conditions and determining the amount of produced CA.

### 2. Production of A C VS (or any other protein or enzyme)

Production of ACVS (or any other protein or enzyme) can be determined by any suitable method that is known to a person skilled in the art. For instance, a suitable method is determining the expression of ACVS mRNA by Northern blot, quantitative polymerase chain reaction (qPCR), or by using the second generation sequencing technologies coupled with reverse transcription techniques. Further, the presence of the enzyme itself can be identified by suitable methods that are known to a person skilled in the art, such as Western blotting, or by determination of *in vitro* activity of ACVS. The presence of ACVS can be confirmed by identification of any cephalosporin/cephamycin type antibiotic produced by an organism.

### 3. Determining the activity of ACVS (or of another enzyme)

The activity of AVCS (or any other enzyme) can be determined for instance by setting up an *in vitro* method, wherein an isolated substrate for ACVS (or for any other enzyme whose activity is to be determined) is, together with co-factors that may be necessary, brought together with a cell free lysate of the microorganism. The activity is then compared to the activity of the respective enzyme of the reference microorganism. The method is described in detail for instance in Kern *et al.* (1980) with regard to LAT activity, and in Jhang *et al.* (1989) with regard to ACVS and IPNS activity.

A (reduced) activity of ACVS can also be determined by measuring the production of cephamycin C. Reducing the activity of ACVS blocks the cephamycin C synthesis, which provides that the modifying or treating of the microorganism results in reduced cephamycin C production.

### Description of the figures

**Figure 1.** Figure 1 shows the molecular structure of clavulanic acid (CA).
**Figure 2.** Figure 2 is a graphic representation of the *S*. *clavuligerus* cephamycin C gene cluster.
**Figure 3.** Figure 3 depicts the initial steps in cephamycin C biosynthesis in *S*. *clavuligerus,* and some of the enzymes involved.
**Figure 4**. Figure 4 depicts the genetic map of the pKONC/pcbAB_L1_D1, the disruption plasmid for *pcbAB.*
**Figure 5**. LC-MS chromatograms of the cultures of **(A)** *S*. *clavuligerus* ATCC 27064 and **(B)** *S*.
*clavuligerus* ATCC 27064 *ΔpcbAB* after 144h of cultivation in the DCM8 medium. From top to bottom the chromatograms are as follows: MS ESI- total ion count, MS ESI- 197.5-198.5 representing clavulanic acid, MS ESI-444.5-445.5 representing cephamycin C and MS ESI- 155.6-156.5 representing clavam-2-carboxylate.

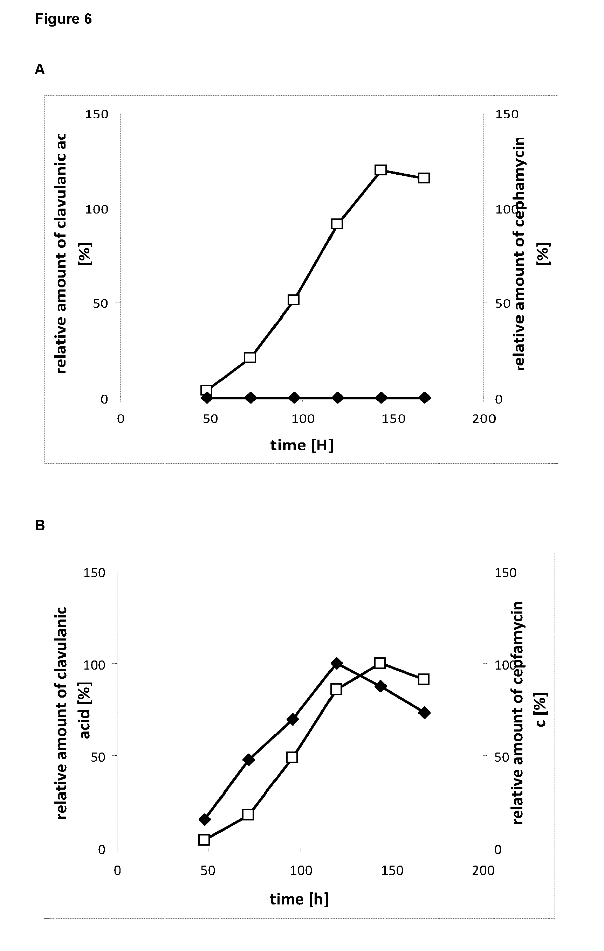

**Figure 6****.** Production of cephamycin C (solid diamonds) and clavulanic acid (empty rectangles) by **(B)** cultures of S. *clavuligerus* K213 and **(A)** S. *clavuligerus* K213 ΔpcbAB as function of cultivation time.

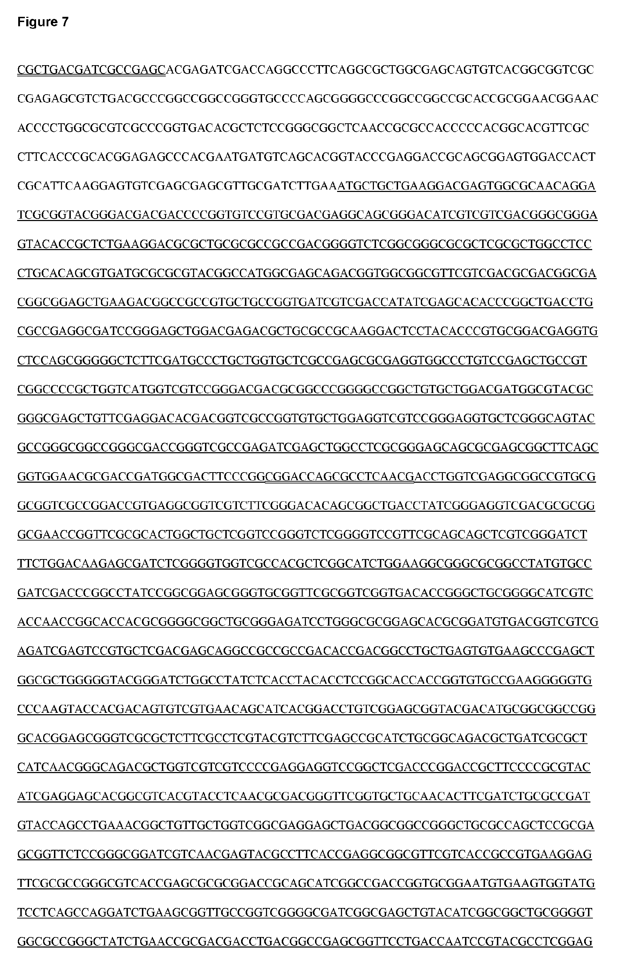

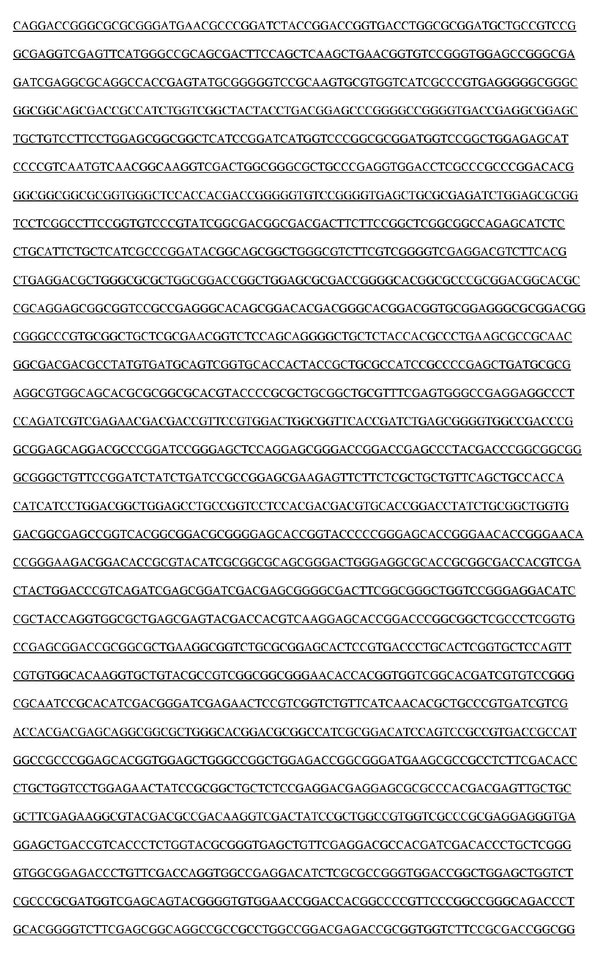

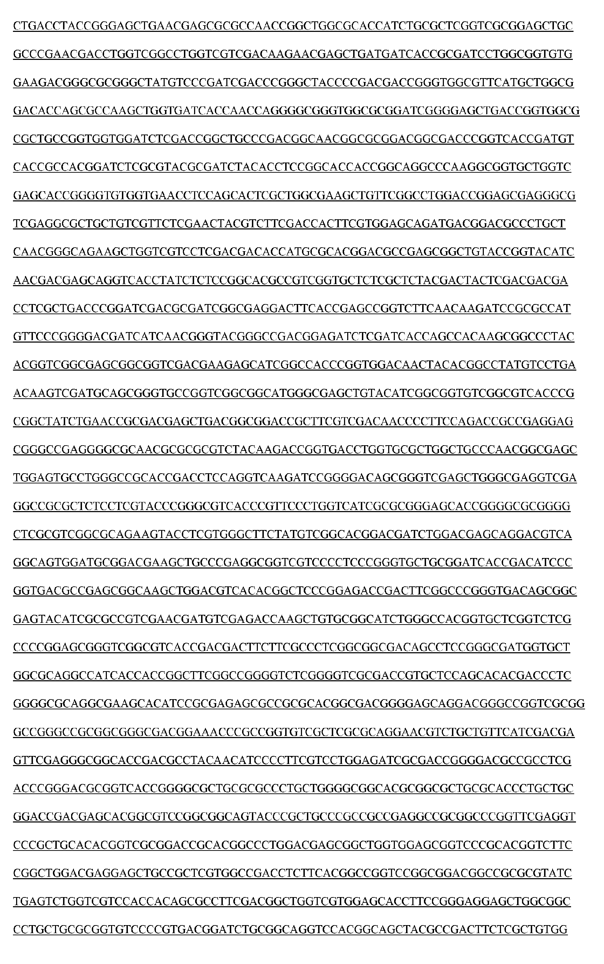

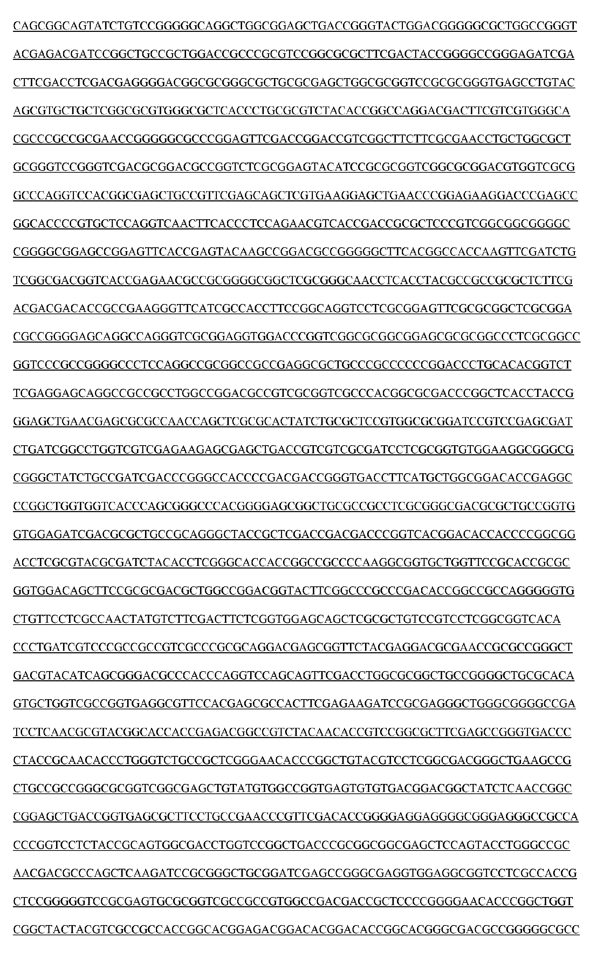

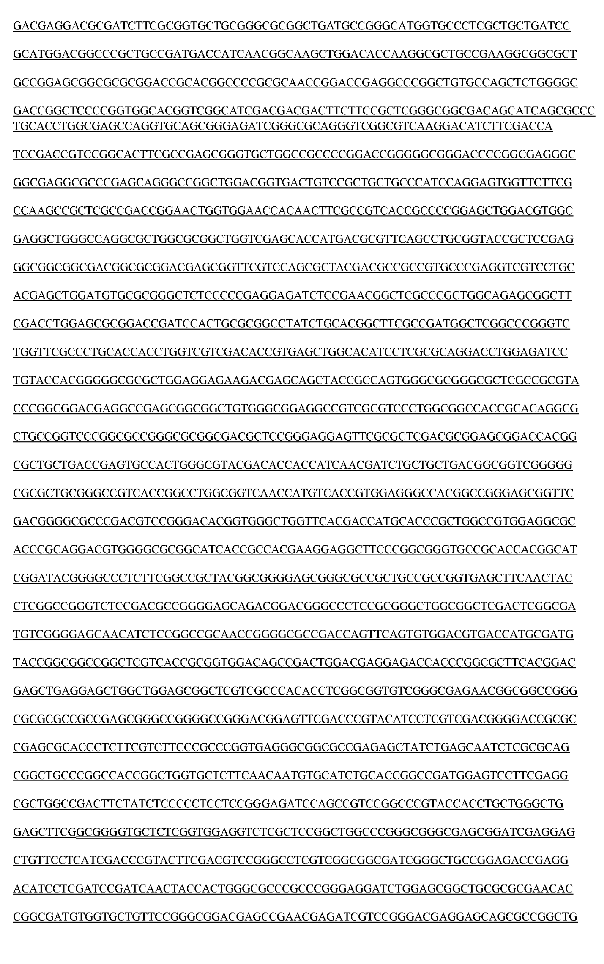

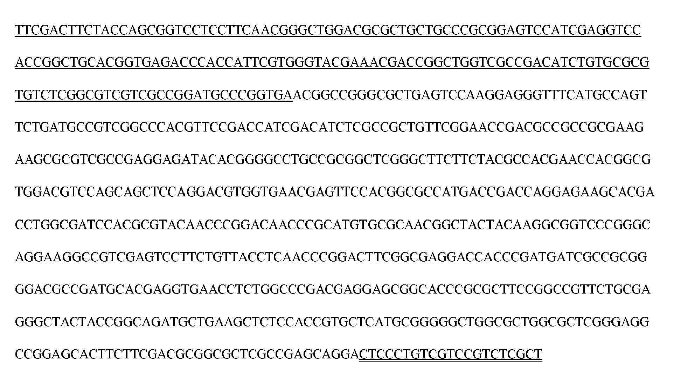

**Figure 7.** Figure 7 depicts the nucleic acid sequence comprising the gene *pcbAB* from the genome of *S. clavuligerus,* primer binding sites and coding region. Underlined is the coding region of the *pcbAB* gene, double underlined are the locations of annealing of the primers used for amplification of the homologous flanking regions in pKONC/pcbAB_L1_D1.

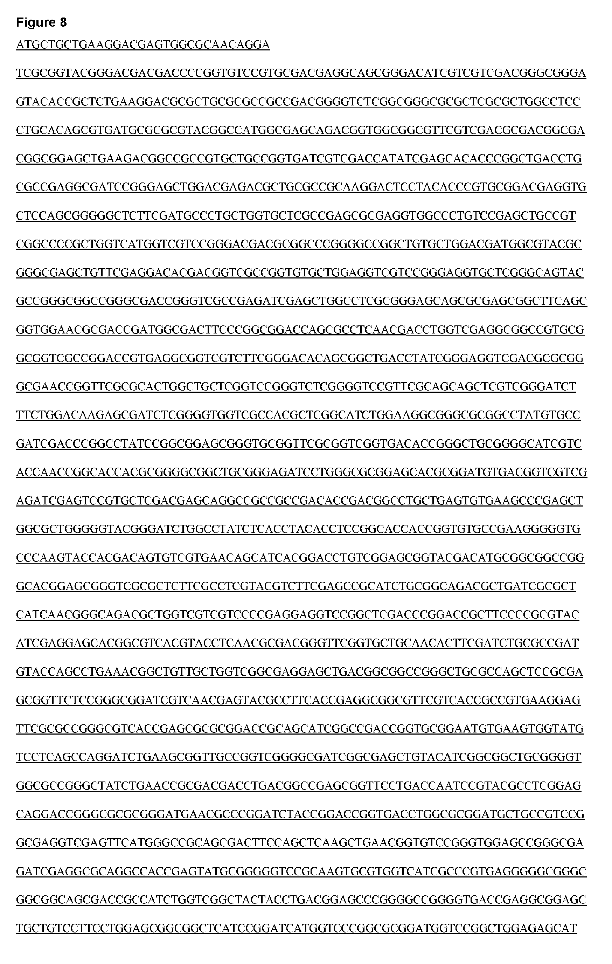

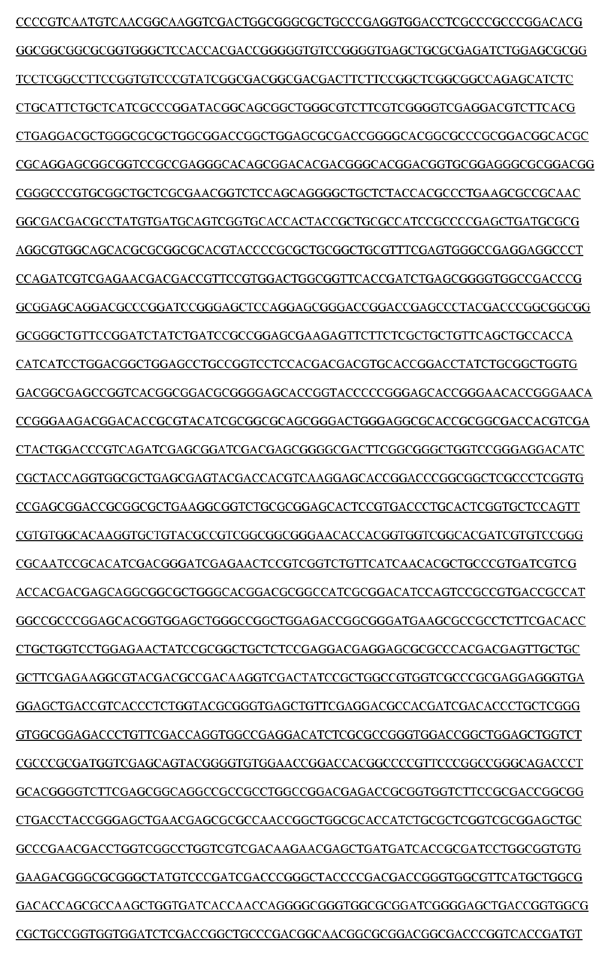

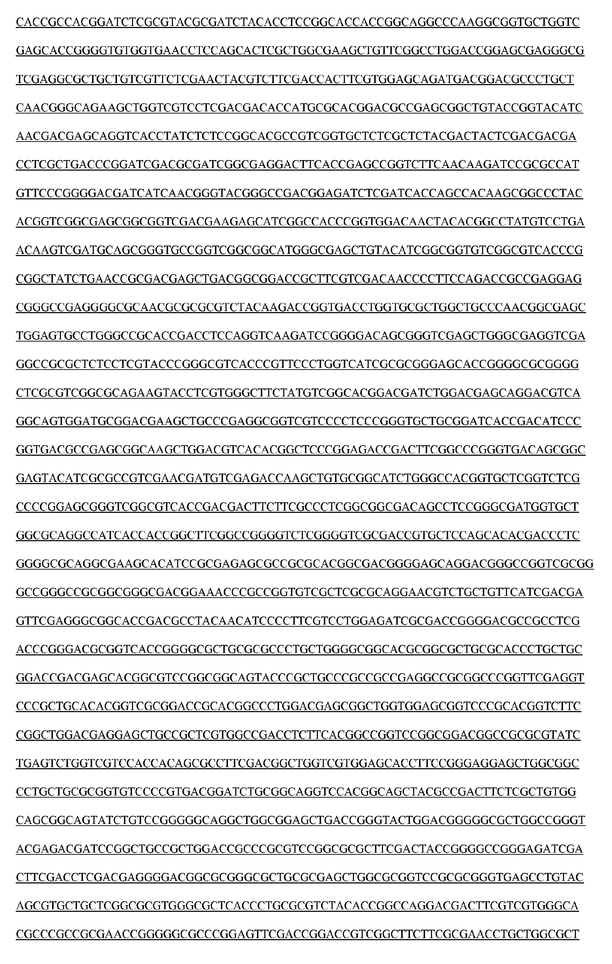

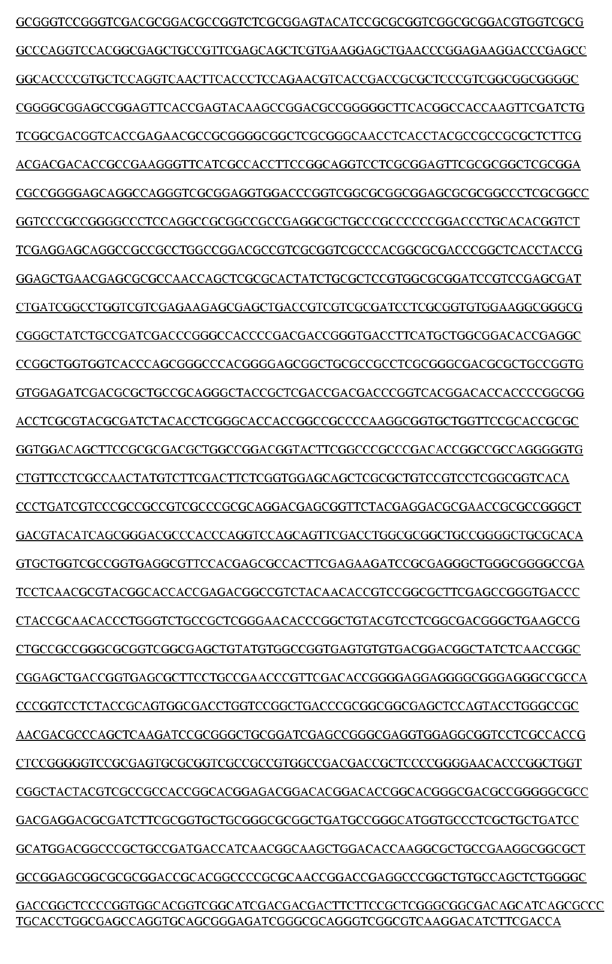

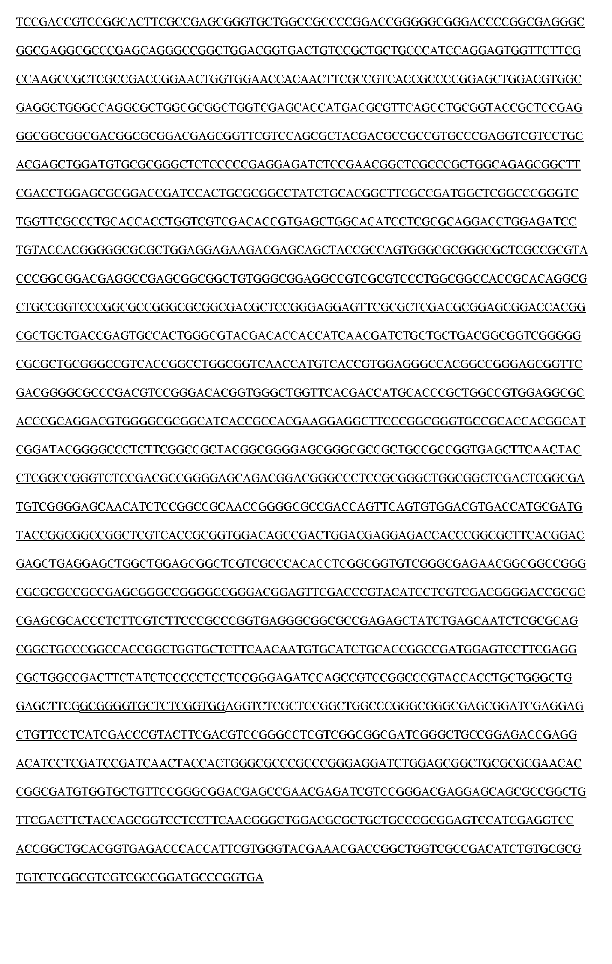

**Figure 8****.** Coding region of the *pcbAB* gene of *S*. *clavuligerus.* Underlined is the coding region of the *pcbAB* gene, double underlined are the locations of annealing of the primers used for amplification of the homologous flanking regions in pKONC/pcbAB_L1_D1.

### Examples

### Example 1. Maintenance and general manipulation with S. clavuligerus

Media and growth conditions used for general manipulation and maintenance of *S*. *clavuligerus* are listed. Specific procedures are given at appropriate examples below.

### SNK-t medium

SNK-t medium is a liquid medium used for general cultivation of *S*. *clavuligerus* where CA production is not necessary. Said medium was used for obtaining S. *clavuligerus* biomass for purpose of DNA isolation, re-cultivation in selection procedures as well as a seed phase for testing purposes. The composition of the SNK-t medium is presented in Table 1.

**Table 1. The composition of the SNK-t medium.**

| **Material** | **Amount** |
|---|---|
| Bacto Soytone (DIFCO BD) | 20 g |
| NaCl | 5 g |
| K₂HPO₄ | 2.5 g |
| Glycerol (Sigma) | 24 g |
| soya oil (GEA) | 5 g |
| Ultra-pure water | To a final volume of 500 mL |

The pH was adjusted to 6.8 using 5M HCl. Medium was sterilized at 121±2°C, 120±1 kPa for 15 minutes. After sterilization, 500 ml sterilized and cooled solution of maltose (DIFCO BD) - 20 g/L was added to a cooled medium.

SNK-t was used with culture conditions as follows:
- Deep-well microtiter plate (square well): 500 µl of medium, inoculation with 2% suspension of *S. clavuligerus* spores or, alternatively, another liquid culture or frozen culture. Incubation was carried out on a rotary shaker at 28°C and 260 rpm (2.5 cm excenter) for 24-48 hours under aerobic conditions.
- 100 ml shake flask: 15 ml of medium, inoculation with 2% suspension of *S. clavuligerus* spores or, alternatively, another liquid culture or frozen culture. Incubation was carried out on a rotary shaker at 28°C and 260 rpm (2.5 cm excenter) for 24-48 hours under aerobic conditions.
- 250 ml shake flask: 50 ml of medium, inoculation with 2% suspension of *S. clavuligerus* spores or, alternatively, another liquid culture or frozen culture. Incubation was carried out on a rotary shaker at 28°C and 260 rpm (2.5 cm excenter) for 24-48 hours under aerobic conditions.

### KKA-2L medium

KKA-2L medium is a solid medium used as general medium for spore preparation, sub-cultivation of the strains, imposing and /or maintaining selection pressures, isolation of single colony derived strains, etc. The composition of the KKA-2L medium is presented in Table 2 and the composition of the mineral solution is presented in Table 3.

**Table 2. The composition of the KKA-2L medium.**

| **Material** | **Amount** |
|---|---|
| Corn dextrine (ROQUETTE) | 10 g |
| K₂HPO₄ | 1 g |
| MgSO₄ × 7H₂O | 1 g |
| NaCl | 1 g |
| (NH₄)₂SO₄ × 7H₂O | 2 g |
| CaCO₃ | 4 g |
| Mineral solution | 1 mL |
| Bacto agar (DIFCO BD) | 20 g |
| Tap water | To a final volume of 1 Liter |

**Table 3. The composition of the Mineral solution.**

| **Material** | **Amount** |
|---|---|
| 5M HCl | 10 ml |
| CaCl₂ × 2H₂O | 25 g |
| NaCl | 12 g |
| MgCl₂ × 6H₂O | 66 g |
| KCI | 100 g |
| FeCl₃ × 6H₂O | 5 g |
| ZnCl₂ | 0.7 g |
| CuCl₂ × 2H₂O | 0.3 g |
| MnSO₄ × H₂O | 0.4 g |
| Ultra pure water | To a final volume of 1 Liter |

The pH is naturally around 7.0, there was no pH adjustment prior to sterilization. Sterilization was performed at 121±2°C, 220±10 kPa for 20 minutes.

### ISP-2 medium

ISP-2 medium is a solid medium used for plating of liquid culture dilutions. Characteristically it allows faster growth of the *S*. *clavuligerus* colonies.

**Table 4. The composition of the ISP-2 medium.**

| **Material** | **Amount** |
|---|---|
| Bacto Yeast Extract (DIFCO BD) | 4 g |
| Bacto Malt Extract (DIFCO BD) | 10 g |
| Dextrose (ROQUETTE) | 4 g |
| Bacto agar (DIFCO BD) | 18 g |
| Ultra-pure water | To a final volume of 1 Liter |

The pH was adjusted to 7.0 with 1 M NaOH prior to sterilization. Sterilization was performed at 121±2°C, 220±10 kPa for 20 minutes.

### Example 2. Construction of the pcbAB disruption plasmid

### Construction of pKONC

The plasmid pKONC was designed as a suicide plasmid for *Streptomyces.* Only *E. coli* replicative element was included in the plasmid (originating from pSET151) but no replicative element conferring replication in *Streptomyces.* Therefore, upon transformation and exposure to selection pressure, only those cells of *S*. *clavuligerus* form colonies that had the plasmid integrated into the chromosome. Two selection markers were included into the plasmid: *aac(3)IV* gene conferring apramycin resistance, originating from pSET152, a well known plasmid for use in *Streptomyces* (Kieser *et al.,* 2000) and *codA* counterselection marker gene originating from pMG302M, producing a very toxic 5-fluoro-uracyl in presence of 5-fluoro-cytosine (Dubeau *et al.,* 2009).

*codA* gene was amplified using PCR amplification of pMG392M plasmid DNA using a Eppendorf Mastercycler Gradient thermocycler. The PCR reaction was carried out with PCR Extender System (5PRIME) and the buffer provided by the manufacturer (10x tuning buffer with Magnesium) in the presence of 200 µM dNTP, 4% DMSO, 2 µM of each primer, approximately 50 ng of template DNA and 1 unit of enzyme in a final volume of 25 µl for 30 cycles.

The thermal profile started with denaturation step at 94°C for 10 min. The thermal cycle for the following 30 cycles was 94°C for 45 sec (denaturation step), 58°C for 45 sec (annealing step), and 72°C for 150 sec (extension step). The final elongation step was carried out at 72°C for 7 min.

Oligonucleotide primer pair used was:
SEQ. ID. NO: 4: codA_XbaI_R GCGCTCTAGAGCGCTTGTAGTCGATGGCCT
SEQ. ID. NO: 5: codA_Ndel_F GCGGCATATGGTTGACATCTTTTGCCGATTCTGG

The PCR-amplified product was analysed by agarose gel electrophoresis and its length agreed with the predicted length (1373 bp). The fragment was cloned into the pGEM-T easy PCR cloning vector (Promega) yielding plasmids pGEM/codA. The sequence analysis of the cloned PCR fragment confirmed its respective sequence as expected from the primer design procedure.

*aac(3)IV* gene was amplified using PCR amplification of pSET152 plasmid DNA (Kieser et al., 2000) using an Eppendorf Mastercycler Gradient thermocycler. The PCR reaction was carried out with PCR Extender System (5PRIME) and the buffer provided by the manufacturer (10x tuning buffer with magnesium) in the presence of 200 µM dNTP, 4% DMSO, 2 µM of each primer, approximately 50 ng of template DNA and 1 unit of enzyme in a final volume of 25 µl for 30 cycles.

The thermal profile started with denaturation step at 94°C for 10 min. The thermal cycle for the following 30 cycles was 94°C for 45 sec (denaturation step), 58°C for 45 sec (annealing step), and 72°C for 150 sec (extension step). The final elongation step was carried out at 72°C for 7 min.

Oligonucleotide primer pair used was:
SEQ. ID. NO: 6:
   Apr (aac3) Xbal_F CGCGTCTAGAGGATCTTCACCTAGATCCTTTTGGTTC
SEQ. ID. NO: 7:
   ApR(aac3)_EcoRl CCGGGAATTCCCCGATCCGCTCCACGTG

The PCR-amplified product was analysed by agarose gel electrophoresis and its length agreed with the predicted length (1011 bp). The fragment was cloned into the pGEM-T easy PCR cloning vector (Promega) yielding plasmids pGEM/Apr2. The sequence analysis of the cloned PCR fragment confirmed its respective sequence as expected from the primer design procedure.

The plasmid pGEM/CodA (as described above) was cleaved using restriction endonucleases Ndel and Xbal (both Promega) according to instruction of the manufacturer. The mixture was resolved on the agarose gel electrophoresis gel. The resulting 1368 bp fragment was purified from the agarose gel. In parallel the plasmid pGEM/Apr2 was cleaved with the restriction endonucleases Ndel and Xbal (both Promega) so that 3987 bp was isolated from the band cut out of an agarose electrophoresis band. The fragments were purified of the remaining agarose with help of Wizard® Gel and PCR Clean-up System (Promega).

The fragments were assembled in a DNA ligation reaction using T4 DNA Ligase (Promega) according to manufacturer's instructions. The ligation mixture was used to transform competent *E. coli* JM109 (Promega) and transformed clones were selected based on their resistance to 50 µg/mL ampicillin. The resulting clones were verified using restriction analysis with several distinct restriction endonucleases. The final construct was designated pGEM/codA_Apr2 with overall length of 5357 bp.

Plasmid pSET151 (Kieser *et al.,* 2000) was than cleaved using restriction endonuclease EcoRl and Ndel (both Promega) according to instruction of the manufacturer but under conditions of partial restriction. Fragments were separated on agarose gel electrophoresis. 5897 bp fragment containing the larger part of the plasmid was purified of the remaining agarose with help of Wizard® Gel and PCR Clean-up System (Promega). In parallel, the plasmid pGEM/coda_Apr2 was cleaved with the restriction endonucleases Ndel and EcoRl (both Promega) so that 2373 bp fragment containing *aac(3)IV and codA* genes was isolated from the band cut out of an agarose electrophoresis band. The fragment was purified of the remaining agarose with help of Wizard® Gel and PCR Clean-up System (Promega).

The fragments were assembled in a DNA ligation reaction using T4 DNA Ligase (Promega) according to manufacturer's instructions. The ligation mixture was used to transform competent *E. coli* JM109 (Promega) and transformed clones were selected based on their resistance to 50 µg/mL ampicillin. The resulting clones were verified using restriction analysis with several distinct restriction endonucleases. The final construct was designated pSET151/codA_Apr2 with overall length of 8270 bp.

Finally the plasmid pSET151/codA_Apr2 was cleaved using restriction endonuclease Dral (Promega) according to instruction of the manufacturer. Fragments were separated on agarose gel electrophoresis. 542/8 bp fragment containing the larger part of the plasmid was purified of the remaining agarose with help of Wizard® Gel and PCR Clean-up System (Promega) and self-ligated in a DNA ligation reaction using T4 DNA Ligase (Promega) according to manufacturer's instructions. The ligation mixture was used to transform competent *E. coli* JM109 (Promega) and transformed clones were selected based on their resistance to 25 µg/mL apramycin. The resulting clones were verified using restriction analysis with several distinct restriction endonucleases. The final construct was designated pKONC with overall length of 5428 bp which differs from pSET151/codA_Apr2 in loss of fragment containing genes *xylE, tsr* and *bla.*

### Construction of pKONC/pcbAB

### a) Preparation of Streptomyces clavuligerus genomic DNA

Spores of *Streptomyces clavuligerus* ATCC 27064 (wild type) were used to inoculate 50 ml of SNK-t medium in a 250 ml Erlenmeyer flask. Cultures were grown for 24 hours at 28°C with shaking (260 rpm). Mycelium was recovered by centrifugation and genomic DNA was prepared using Wizard® Genomic DNA Purification Kit (Promega) according to the instructions of the kit manufacturer. DNA was re-suspended in 100 µl TE buffer (Sambrook and Russell, 2000, Molecular Cloning: A Laboratory Manual, ISBN-978-087969577-4).

### b) PCR amplification of pcbAB fragments used for gene disruption

PCR amplification of DNA fragments: *S*. *clavuligerus* genomic DNA obtained was PCR amplified using an Eppendorf Mastercycler Gradient thermocycler. The PCR reaction was carried out with PCR Extender System (5PRIME) and the buffer provided by the manufacturer (10x tuning buffer with magnesium) in the presence of 200 µM dNTP, 4% DMSO, 2 µM of each primer, approximately 50 ng of template *S*. *clavuligerus* genomic DNA and 1 unit of enzyme in a final volume of 25 µL for 30 cycles.

The thermal profile started with denaturation step at 98°C for 120 sec followed by 95°C for 120 sec. The thermal cycle for the following 30 cycles was 95°C for 20 sec (denaturation step), 62°C for 15 sec (annealing step), and 72°C for 150 sec (extension step). The final elongation step was carried out at 72°C for 7 min.

For the amplification of the 5' flanking region of *pcbAB* the following oligonucleotide primer pair was used:
SEQ. ID. NO: 8: pcbAB_L1_F_EcoRI GCGCGAATTCCGCTGACGATCGCCGAGC
SEQ. ID. NO: 9: pcbAB_L1_R_BamHl GCGCGGATCCCGTTGAGGCGCTGGTCCG

For the amplification of the 3' flanking region of the *pcbAB* the following oligonucleotide primer pair was used:
SEQ. ID. NO: 10: pcbAB_D1_F_BamHl GCGCGGATCCGCGGGGTGCTCTCGGTGG
SEQ. ID. NO: 11: pcbAB_D1_R_Hindlll GCGCAAGCTTAGCGAGACGGACGACAGGGAG

The PCR-amplified products obtained were analysed by agarose gel electrophoresis and their length agreed with their corresponding predicted length.

The fragments were cloned was cloned into the pGEM-T easy PCR cloning vector (Promega) yielding plasmids pGEM/pcbAB_L1 containing 5' flanking fragment and pGEM/pcbAB_D1 containing 3' flanking fragment. The sequence analysis of the cloned PCR fragments confirmed its respective sequence as expected from the primer design procedure.

### c) Final assembly of pcbAB disruption plasmid

The plasmid pKONC (as described above) was cleaved using restriction endonucleases EcoRl and Hindlll (both Promega) according to instruction of the manufacturer. The mixture was resolved on the agarose gel electrophoresis gel. The resulting 5377 bp fragment was purified from the agarose gel. In parallel, the plasmid pGEM/pcbAB_L1 was cleaved with the restriction endonucleases EcoRl and BamHl (both Promega) so that 1028 bp 5' flanking fragment was excised from the plasmid and isolated from the band cut out of an agarose electrophoresis band. The fragment was purified of the remaining agarose with help of Wizard® Gel and PCR Clean-up System (Promega). The 1031 bp 3' fragment was excised from pGEM/pcbAB_{_}D1 using BamHl and Hindlll and purified as described above.

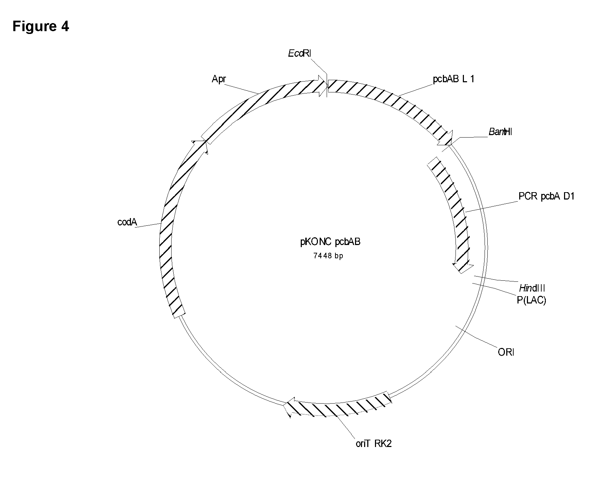

The fragments were assembled in a single step in a DNA ligation reaction using T4 DNA Ligase (Promega) according to manufacturer's instructions. The ligation mixture was used to transform competent *E*. *coli* JM109 (Promega) and transformed clones were selected based on their resistance to 25 ug/mL apramycin. The resulting clones were verified using restriction analysis with several distinct restriction endonucleases. The final construct was designated pKONC/pcbAB_L1-D1 with overall length of 7448 bp. Its physical map is illustrated in Figure 4 (the genetic map of the pKONC/pcbAB_L1_D1, the disruption plasmid for pcbAB).

### Example 3. Disruption of the pcbAB gene in the genomes of S. clavuligerus K213 and S. clavuligerus ATCC 27064

### Conjugal transfer of disruption plasmid to S. clavuligerus strains

Plasmid construct pKONC/pcbAB_L1_D1 was introduced by transformation into electrocompetent *E*. *coli* strain ET12567 containing the conjugation helper plasmid pUZ8002 (Paget et al., 1999 J. Bacteriol. 181: 204-211). The plasmid pUZ8002 contains all the necessary genes for construction of conjugative pilli, however it lacks the origin of transfer and, thus, remains in the host cell (Jones et al., 1997 Mol. Microbiol. 23:169-178). Conjugation procedure was done as described in Kieser *et al.,* 2000 using *S*. *clavuligerus* ATCC 27064 and S. *clavuligerus* K213 spores. KKA-2L agar plates were used for selection of exoconjugants and apramycin (Sigma) was used for selection at 60 µg/ml. Transformation with vector pKONC/pcbAB_L1_D1 yielded 12 exoconjugants resistant to apramycin. As the plasmid pKONC/pcbAB_L1_D1 is unable to replicate itself in *S*. *clavuligerus,* the exoconjugants must have had the said plasmid integrated into the genome. Only tops of exconjugant colonies were transferred in a dense spread to KKA-2L medium supplemented with 60 µg/ml apramycin (KKA-2L Apr) and grown at 25°C for 10 days. Exoconjugants were tested by a PCR method, described later in detail, and were shown that they all contain a single crossover genotype as expected.

### Selection of disruption mutants

Selection for stable secondary recombinant strains of *S*. *clavuligerus* with disrupted *pcbAB* gene was achieved by counterselection to *CodA* (Cytosine-deaminase) marker gene with Fluorocytosine (Sigma) and therefore loss of all genetic markers together with the pKONC/pcbAB_L1_D1 plasmid backbone. The approach is known by skilled persons as scarless gene deletion (Dubeau *et al.,* 2009).

Spores of *S*. *streptomyces* exconjugants of vector pKONC/pcbAB_L1_D1, grown at 25°C on KKA-2L medium supplemented with 60 µg/ml apramycin (KKA-2L Apr) were inoculated into liquid SNK-t medium with apramacin (60 µg/ml) and grown in deep-well microtiter plates at 28°C and 260 rpm to produce dense mycelium culture. After 24h, this culture was subcultivated into parallel wells of fresh deep-well microtiter plates, one containing SNK-t medium supplemented with 60 µg/L apramycin and the other containing SNK-t medium supplemented with 100 µg/L 5-fluoro-cytosine (FC) medium and grown at 25°C and 260 rpm until growth appeared in the wells containing 5-fluoro-cytosine. The cultures were subcultivated repeatedly, as soon as growth appeared in the SNK-t FC containing wells into parallel wells in a manner described above (the 1% inoculum used was always taken from the preceding generation in SNK-t FC wells).

The procedure was repeated for as little as five to as many as fifteen cycles of selection, until growth was no longer appearing in the wells with SNK-t supplemented with apramycin. The cultures ware then plated onto ISP-2 medium containing 100 µg/ml 5-fluoro-cytosine in appropriate dilutions yielding approximately 100 colonies per plate. After 5-10 days, the colonies were spread in parallel onto KKA-2L and KKA-2L Apr agar plates. A large majority of the transferred strains showed growth only on KKA-2L but not on KKA-2L Apr agar plates, indicating the loss of genetic markers from the genome and thereby successful secondary recombination. Approximately 30 independently obtained secondary recombinants were isolated for each of the parent strains; *S*. *clavuligerus* ATCC 27064 and *S. clavuligerus* K213.

### Conformation of disruption of pcbAB gene by genetic characterization

PCR was used as a method for conformation of correct integration and homologous recombination events during the selection pressure cycles. A set of conformation oligonucleotide primers were designed which distinguish between wild type, single crossover situation and the final double recombination situation in the locus of the I gene in the *S*. *clavuligerus* genome.

DNA templates ware prepared in accordance to the "colony PCR" approach. Specifically, the spores of the tested strains were cultivated in SNK-t medium at 25°C and 260 rpm for 48 h, followed by separation of the mycelium by filtration, and partial permeabilization of the mycelium using resuspension in TE buffer (Sambrook and Russell, 2000, Molecular Cloning: A Laboratory Manual, ISBN-978-087969577-4), supplemented with 2.5 µg/ml RNase. (Roche, 500 µg/mL) The mycelium as filtered off and the filtrate was used as a DNA template for the PCR reactions. The PCR reaction was carried out with PCR Extender System (5PRIME) on an Eppendorf Mastercycler Gradient thermocycler with the buffer provided by the manufacturer (10x Tuning buffer with Magnesium) in the presence of 200 µM dNTP, 4% DMSO, 2 µM of each primer, approximately 1 µL of template *S*. *clavuligerus* genomic DNA and 0.5 unit of enzyme in a final volume of 12.5 µl for 30 cycles.

The thermal profile started with denaturation step at 98°C for 3 min followed by 95°C for 120 sec. The thermal cycle for the following 30 cycles was 95°C for 20 sec (denaturation step), 61°C for 15 sec (annealing step), and 72°C for 3 min (extension step). The final elongation step was carried out at 72°C for 7 min. PCR products were analysed on agarose gel electrophoresis determining presence and length of PCR products.

The primer pairs used were the following:
SEQ. ID. NO: 12: pcbAB_conf_F3 AGCTGTTCGAGGACACGACGGTC
SEQ. ID. NO: 13: pcbAB_conf_R3 GACGGCATCAGAACTGGCATGAAA
SEQ. ID. NO: 12: pcbAB_conf_F3 AGCTGTTCGAGGACACGACGGTC
SEQ. ID. NO: 14: pcbAB_conf_R3_IN AGTCGACCTTGCCGTTGACATTGA
SEQ. ID. NO: 15: pcbAB_conf_znotraj_F ACTTCCAGCTCAAGCTGAACGGTGT SEQ. ID. NO: 16: pcbAB_conf_znotraj_R TCCTTGACGTGGTCGTACTCGCTC

**Table 5. The expected lengths of the conformation PCR products**

| | **Wild type** | **Single cross-over** | **Double cross-over** |
|---|---|---|---|
| pcbAB_conf_F3 | No product | 683 bp | 683 bp |
| pcbAB_conf_R3 | (10kb fragment) | | |
| pcbAB_conf_F3 | 1704 bp | 1704 bp | No product |
| pcbAB_conf_R3_IN | | | |
| pcbAB_conf_znotraj_F | 1414 bp | 1414 bp | No product |
| pcbAB_conf_znotraj_R | | | |

5 strains originating from *S. clavuligerus* ATCC 27064 and 3 strains originating from *S*. *clavuligerus* K213 had the expected PCR product profile. These strains were additionally tested for absence of genetic marker cytosine deaminase and marker for apramycin resistance using PCR and were all found negative. Other strains have reverted to the wild type genotype and were therefore discarded. These strains were additionally tested for absence of genetic marker cytosine deaminase and marker for apramycin resistance using PCR and were all found negative. One strain of each parent and designated accordingly: *S. clavuligerus* ATCC 27064 Δ*pcbAB* and *S. clavuligerus* K213 Δ*pcbAB.*

Both of these strains were further confirmed by southern hybridization.

DIG-labelled plasmid DNA probe: The plasmid pGEM/pcbAB_L1 was digested with EcoRl and BamHl restriction enzymes and the 1028 bp long fragment was purified with a Wizard® Gel and PCR Clean-up System (Promega). Approximately 1 µg of the eluted fragment was labelled over-night with digoxigenin-dUTP by random primed labelling technique using the DIG High Prime DNA Labelling and Detection Starter Kit I (Roche). Genomic DNA of S. *clavuligerus* wild type strains and the corresponding *pcbAB* disruption mutants as well as the intermediate primary recombinant strains was prepared as described earlier. Approximately 10 µg of each isolated genomic DNA was digested with the EcoRl (Promega) restriction enzyme. The DNA fragments were then separated by electrophoresis in a 1% agarose gel at 20 V for 12 hours, and transferred to a positively charged Hybond-N+ nylon membrane (GE Healthcare) using the alkali capillary blotting method (Southern, 1975) for 6 hours. Pre-hybridization and hybridization of DIG-labelled DNA probe to DNA immobilized on a nylon membrane was performed as suggested by the DIG High Prime DNA Labelling and Detection Starter Kit I manufacturer (Roche). Pre-hybridization was carried out in the supplied pre-hybridization solution for 1 hour at 50°C. For hybridization, 1 µg of denatured DNA (at 90°C for 10 minutes) was added to fresh pre-hybridization solution pre-warmed to 50°C. After overnight hybridization, membranes were washed as follows: two washes with 2 x SSC, 0.1 % SDS, at room temperature for 5 minutes, and two washes with 0.5 x SSC, 0.1% SDS at 55°C for 15 minutes. The hybridized probes were immunodetected with anti-digoxigenin-AP Fab fragments and visualized with the colorimetric substrates NBT/BCIP, as suggested by the DIG High Prime DNA Labelling and Detection Starter Kit I supplier. After analysis of positive fragments, a clear distinction could be observed between all 3 genotypes. The secondary recombinants derived by pKONC/pcbAB_L1_D1 vector were therefore confirmed by described Southern hybridization procedure.

### Example 4. Clavulanic acid and cephamycin C production of the pcbAB disruption mutants

### General characterization of the strains

The *pcbAB* disruption strains were first evaluated by morphological appearance on several different agar media. From the PCR confirmed disruption mutants, those with most similar appearance to the respective wild type strains were selected for further testing.

### Growth conditions, media and sampling methods

Spores of the tested strains obtained from 1 square cm of the confluent KKA-2L agar plate culture are used as inoculum for the seed phase. The seed phase is cultivated on a rotary shaker in 250ml shake flasks containing SNK-t medium at 28°C and 260 rpm (2.5 cm excenter). After 40-48 hours the culture is ready for transfer to the production medium DCM8. The production medium DCM8, 15 mL in 100 mL shake flask, is inoculated with 600 ml of the seed medium culture and incubated on a rotary shaker at 25°C and 260 rpm (2.5 cm excenter) for 7 days.

**Table 6. The composition of the DCM8 medium**

| **Material** | **Amount** |
|---|---|
| Maltose (DIFCO BD) | 60 g |
| Glycerol (Sigma) | 24 g |
| L-Arginine (Sigma) | 8 g |
| L-Aspartic acid (Sigma) | 4 g |
| L-Lysine Hidrochloride (Sigma) | 2 g |
| L-Valine (Sigma) | 1 g |
| L-Histidine (Sigma) | 1 g |
| K₂HPO₄ | 1.36 g |
| RPMI 1640 Vitamins solution (Sigma) | 10 mL |
| L-Cysteine (Sigma | 1.2 g |
| MOPS (Melford) | 42 g |
| Mineral solution | 15 mL |
| Ultrapure water | To a final volume of 1 Liter |

The pH is adjusted to 6.8 with 1 M NaOH prior to sterilization. Sterilization is performed at 121±2°C, 220±10 kPa for 15 minutes.

A large number of parallel shake flasks were inoculated so that each one flask was discarded after sampling while others were left incubating until the next sampling and so on. 5 independent production cultures were sampled and analyzed for each culture tested at each time point. The sampling was performed by pipetting 2x 2 ml of the culture broth into 2 mL Eppendorf tubes. The samples were immediately centrifuged for 10 min at 15000 G and 4°C. The cleared supernatants were decanted into a fresh vial and stored at -75°C until analysis. 10-fold dilutions (with ultrapure water) of the samples were used for the LC-MS analysis. Additional parameters were measured in these samples such carbon source concentration, amino acid consumption, pH etc., but these are not the subject of the said invention.

### Analytical methods

LC-MS analysis was performed with mass spectrometer Thermo LCQ FLEET (Ion trap) equipped with Waters Acquity UPLC system. Samples were prepared by centrifugation of the culture broths and subsequent filtration and 10-fold dilution of culture supernatants. The following chromatographic conditions were used: Column: Agilent Zorbax SB-Aq 50x4.6mm, 1.8 µm, flow: 0.5 ml/min, temperature: 35°C, λ=260 nm, 210 nm, 280 nm, eluent A: 18,2MΩ water, formic acid (pH 2.5), eluent B: 75 eluent A, 25 acetonitrile, v/v. Gradient: 0 - 1.88 min - 35% B, 1.92-2.72 min - 100% B, 2.76 - 5.2 min 35% B. Sample injection 5 µl. Ionization conditions: ESI -, Spray voltage: 4.0 kV, Capillary voltage: 14 V, Tube lens voltage: 79.5V, capillary temperature: 275°C, sheet gas 33 AU, Auxiliary gas 5 AU, full scan, normal mode 100-500 m/z, ESI +

Analysis showed an UV absorption peak at 1.49 min with maximum absorption at 258 nm, having [M-H]⁻ 444,80 as the major m/z at negative ionization and [M-H]⁺ 446,90 at positive ionization, corresponding to cephamycin C. The isotopic distribution agrees with the prediction obtained for molecular formula C₁₆H₂₂N₄O₉S. In addition, clavulanic acid was detected as an UV absorption peak at 2.24 min with maximum absorption at 221 nm, having [M-H]⁻ 198,10 as the major m/z at negative ionization and [M-H]⁺ 200,00 at positive ionization, corresponding to clavulanic acid. The isotopic distribution agrees with the prediction obtained for molecular formula C₈H₉NO₅.

Quantification of clavulanic acid was made by comparing the UV absorption peak area of clavulanic acid to the area of external standard. Quantification of cephamycin C was made by comparison of ion count peak of cephamycin C for m/z = 444.5-445.5 at negative ionization with ion count peak of Cephalosporin C for m/z = 414.5-415.5 at negative ionization, eluting at 2.6 min. The results are given as relative amounts of cephamycin C and clavulanic acid produced by *pcbAB* disruption mutants (*S*. *clavuligerus* ATCC 27064 Δ*pcbAB* and *S*. *clavuligerus* K213 Δ*pcbAB*) compared to production of corresponding wild type strains (*S. clavuligerus* ATCC 27064 and S. *clavuligerus* K213) cultivated under exact same conditions.

### Results

1. Production of Cephamycin C and clavulanic acid by cultures of *S*. *clavuligerus* ATCC 27064 and S. *clavuligerus* ATCC 27064 Δ*pcbAB* showed a significant difference in clavulanic acid productivity. The cephamycin C was not detected at all in *S*. *clavuligerus* ATCC 27064 Δ*pcbAB* and the final clavulanic acid titer obtained after 6 days of cultivation was 65 % higher than in the *S*. *clavuligerus* ATCC 27064, the wild type strain.

**Table 7. Relative amounts of clavulanic acid and cephamycin C given as % wherein the highest amounts produced by wild type strain (S. clavuligerus ATCC 27064) are nominated as 100%.**

| | *S. clavuligerus* ATCC 27064 | | *S*. *clavuligerus* ATCC 27064 *ΔpcbAB* | |
|---|---|---|---|---|
| | Clavulanic acid | Cephamycin C | Clavulanic acid | Cephamycin C |
| 72h | *27* | *61* | *37* | *n.d.** |
| 96h | *45* | *100* | *72* | *n.d.* |
| 120h | *85* | *96* | *153* | *n.d.* |
| 144h | *100* | *86* | *165* | *n.d* |

| | | | | |
|---|---|---|---|---|
| ** not detected* | | | | |

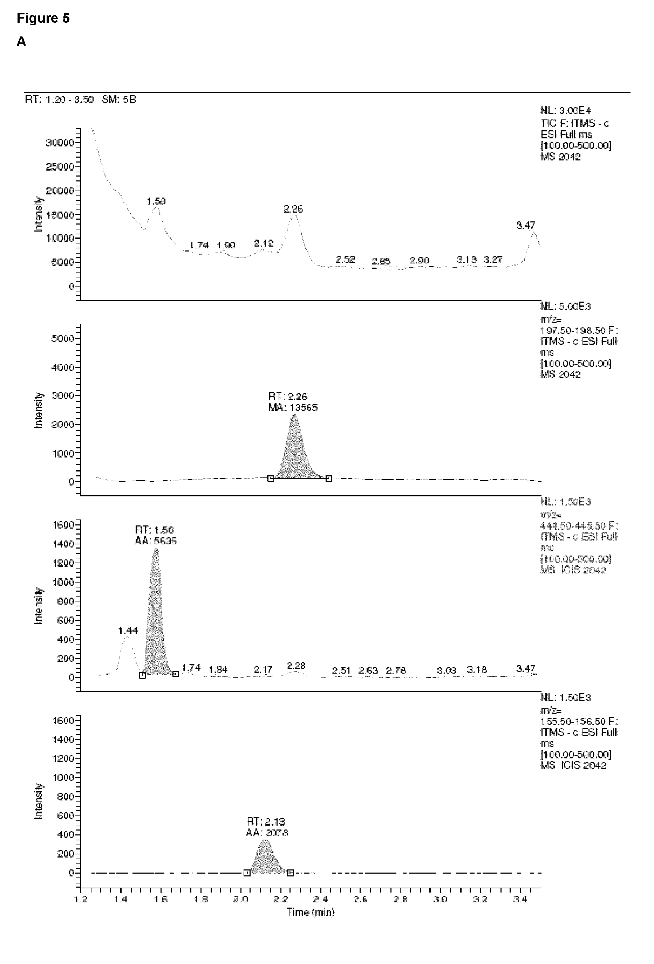

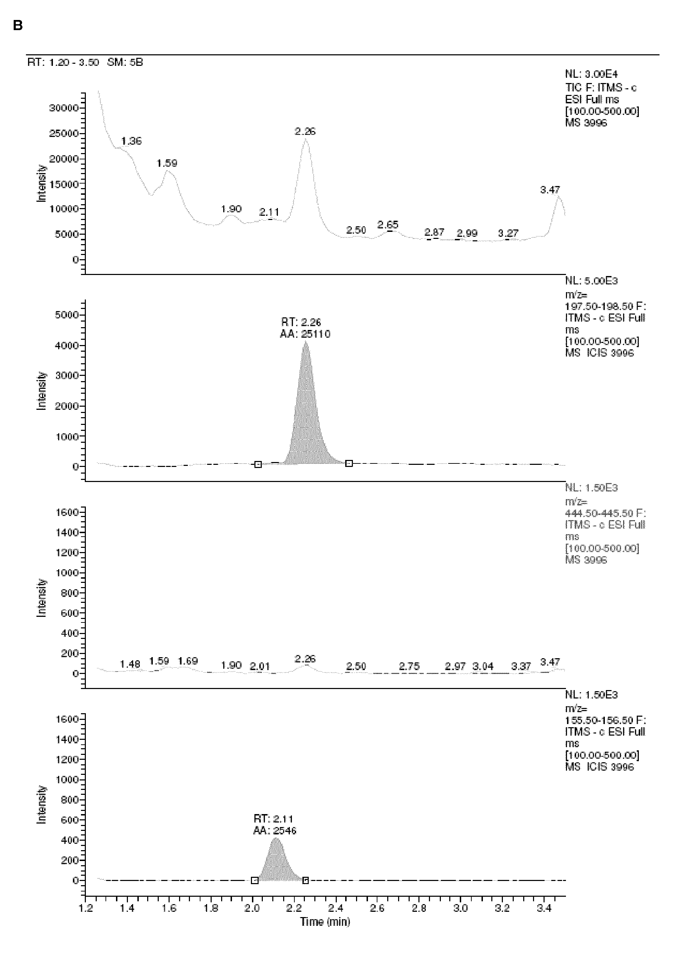

Figure 5 depicts the LC-MS chromatograms of the cultures of **(A) S. clavuligerus** ATCC 27064 and **(B)** *S*. *clavuligerus* ATCC 27064 Δ*pcbAB* after 144h of cultivation in the DCM8 medium.
2. Production of cephamycin C and clavulanic acid by cultures of *S*. *clavuligerus* K213 and *S*. *clavuligerus* K213 Δ*pcbAB* showed a significant difference in clavulanic acid productivity. The cephamycin C was not detected at all in *S*. *clavuligerus* K213 Δ*pcbAB* and the final clavulanic acid titer obtained after 6 days of cultivation was 20% higher than in the *S*. *clavuligerus* K213, the wild type strain. The effect is not as big as observed with the *S*. *clavuligerus* ATCC 27064, yet clear. Also see Figure 6.

**Table 8. Relative amounts of clavulanic acid and cephamycin C given as % wherein the highest amounts produced by wild type strain (S. clavuligerus K213) are nominated as 100%.**

| | *S. clavuligerus* K213 | | *S*. *clavuligerus* K213 □ *pcbAB* | |
|---|---|---|---|---|
| | Clavulanic acid | Cephamycin C | Clavulanic acid | Cephamycin C |
| 96h | *49* | *69* | *51* | *n.d.** |
| 120h | *86* | *100* | *91* | *n.d.* |
| 144h | *100* | *88* | *120* | *n.d.* |
| 168h | *91* | *74* | *116* | *n.d* |

| | | | | |
|---|---|---|---|---|
| **not detected* | | | | |

Figure 6 depicts the production of cephamycin C (solid diamonds) and clavulanic acid (empty rectangles) of *S*. *clavuligerus K213 (B)* and *S. clavuligerus* K213 Δ*pcbAB (A)* strain as function of cultivation time. As can be deduced from Figure 6, the mutant strain S. *clavuligerus* K213 Δ*pcbAB* dose not produce cephamycin C. However, the relative amount of clavulanic acid is higher compared to the amount of clavulanic acid produced by *S*. *clavuligerus K213.*

### References cited:

1. Reading, C. & Cole, M. Clavulanic acid: a beta-lactamase-inhiting beta-lactam from Streptomyces clavuligerus. Antimicrobial agents and chemotherapy 11, 852-7(1977).
2. Brown, A. et al. Naturally-occurring beta-lactamase inhibitors with antibacterial activity. The Journal of antibiotics 29, 668(1976).
3. Liras, P. & Rodriguez-Garcia, a Clavulanic acid, a beta-lactamase inhibitor: biosynthesis and molecular genetics. Applied microbiology and biotechnology 54, 467-75(2000).
4. Brown, A. Clavulanic acid, a novel β-lactamase inhibitor-a case study in drug discovery and development. Drug Des. Deliv 1, 1-25(1986).
5. Saudagar, P.S., Survase, S. a & Singhal, R.S. Clavulanic acid: a review. Biotechnology advances 26, 335-51 (2008).
6. Demain, A.L. Achievements in microbial technology. Biotechnology advances 8, 291-301 (1990). (Demain, 1990) "Biosynthesis and regulation of beta-lactam antibiotics." In : 50 years of Penicillin applications, history and trends.
7. Medema, M.H. et al. The Sequence of a 1.8-Mb Bacterial Linear Plasmid Reveals a Rich Evolutionary Reservoir of Secondary Metabolic Pathways. Genome Biology and Evolution 2010, 212-224(2010).
8. Song, J.Y., Jensen, S.E. & Lee, K.J. Clavulanic acid biosynthesis and genetic manipulation for its overproduction. Applied microbiology and biotechnology 88, 659-69(2010).
9. Khaleeli, N., Li, R. & Townsend, C.A. Origin of the beta-Lactam Carbons in Clavulanic Acid from an Unusual Thiamine Pyrophosphate-Mediated Reaction. Biochemistry 9223-9224(1999).
10. Valentine, B.P. et al. Evidence that arginine is a later metabolic intermediate than ornithine in the biosynthesis of clavulanic acid by Streptomyces clavuligerus. J. Chem. Soc., Chem. Commun. 1210-1211 (1993).at <http://xlink.rsc.org/?doi=C39930001210>
11. Arulanantham, H. et al. ORF17 from the clavulanic acid biosynthesis gene cluster catalyzes the ATP-dependent formation of N-glycyl-clavaminic acid. The Journal of biological chemistry 281, 279-87(2006).
12. Ward, J.M. & Hodgson, J.E. The biosynthetic genes for clavulanic acid and cephamycin production occur as a "super-cluster" in three Streptomyces. FEMS Microbiology Letters 110, 239-242(1993).
13. Paradkar, a S., Aidoo, K. a & Jensen, S.E. A pathway-specific transcriptional activator regulates late steps of clavulanic acid biosynthesis in Streptomyces clavuligerus. Molecular microbiology 27, 831-43(1998).
14. Pérez-Redondo, R. et al. The claR gene of Streptomyces clavuligerus, encoding a LysR-type regulatory protein controlling clavulanic acid biosynthesis, is linked to the clavulanate-9-aldehyde reductase (car) gene. Gene 211, 311-321(1998).
15. Pérez-Llarena, F.J. et al. A regulatory gene (ccaR) required for cephamycin and clavulanic acid production in Streptomyces clavuligerus: amplification results in overproduction of both beta-lactam compounds. Journal of bacteriology 179, 2053-9(1997).
16. Tahlan, K., Anders, C. & Jensen, S.E. The paralogous pairs of genes involved in clavulanic acid and clavam metabolite biosynthesis are differently regulated in Streptomyces clavuligerus. Journal of bacteriology 186, 6286(2004).
17. Tahlan, K. et al. 5S clavam biosynthetic genes are located in both the clavam and paralog gene clusters in Streptomyces clavuligerus. Chemistry & biology 14, 131-42(2007).
18. Nielsen, J. Physiological Engineering Aspects Of Penicillium Chrysogenum. 288(1997).
19. Baltz, R.H. New genetic methods to improve secondary metabolite production in Streptomyces. Journal of Industrial Microbiology and Biotechnology 20, 360-363(1998).
20. Baltz, R.H. Genetic methods and strategies for secondary metabolite yield improvement in actinomycetes. Antonie van Leeuwenhoek 79, 251-9(2001).
21. Li, R. & Townsend, C. a Rational strain improvement for enhanced clavulanic acid production by genetic engineering of the glycolytic pathway in Streptomyces clavuligerus. Metabolic engineering 8, 240-52(2006).
22. Miller, M.T. et al. The catalytic cycle of beta -lactam synthetase observed by x-ray crystallographic snapshots. Proceedings of the National Academy of Sciences of the United States of America 99, 14752-7(2002).
23. Paradkar, A. et al. Applications of gene replacement technology to Streptomyces clavuligerus strain development for clavulanic acid production. Applied and environmental microbiology 67, 2292(2001).
24. Pérez-Redondo, R. et al. Deletion of the pyc gene blocks clavulanic acid biosynthesis except in glycerol-containing medium: evidence for two different genes in formation of the C3 unit. Journal of bacteriology 181, 6922-8(1999).
25. Paradkar, A.S. et al. Applications of Gene Replacement Technology to Streptomyces clavuligerus Strain Development for Clavulanic Acid Production. Society 67, 2292-2297(2001).
26. Tobin, M. et al. Localization of the lysine epsilon-aminotransferase (lat) and delta-(L-alpha-aminoadipyl)-L-cysteinyl-D-valine synthetase (pcbAB) genes from Streptomyces clavuligerus and production of lysine epsilon-aminotransferase activity in Escherichia coli. Journal of bacteriology 173, 6223-6229(1991).
27. Kern B. A. et al. L-Lysine ε-Aminotransferase Involved in Cephamycin C Synthesis in Streptomyces lactamdurans. Antimicrobial Agents And Chemotherapy 17(4), 679-685(1980)
28. Yu, H. et al. Possible involvement of the lysine -aminotransferase gene (lat) in the expression of the genes encoding ACV synthetase (pcbAB) and isopenicillin N synthase (pcbC) in Streptomyces clavuligerus. Microbiology 140, 3367-3377(1994).
29. Dylan, A. et al. pcd mutants of Streptomyces clavuligerus still produce cephamycin C. Journal of bacteriology 189, 5867-74(2007).
30. Jhang et al. Phosphate regulation of ACV synthetase and cephalosporin biosynthesis in Streptomyces clavuligerus. FEMS Microbiol Lett 57, 145-150(1989)
31. Saudagar P. S. et al.,Clavulanic acid: a review. Biotechnology advances 26, 335-51 (2008)
32. Dubeau M.-P. et al., Cytosine Deaminase as a Negative Selection Marker for Gene Disruption and Replacement in the Genus Streptomyces and Other Actinobacteria. Applied and Environmental Microbiology 75, 1211-1214(2009)
33. Martin J. F. et al. Penicillin and cephalosporin biosynthesis: Mechanism of carbon catabolite regulation of penicillin production, Antonie van Leeuwenhoek 75: 21-31, (1999)
34. Kieser et al. Practical Streptomyces genetics, A laboratory Manual (2000) ISBNO-7084-0623-8

### Patents cited:

WO0005397
US6100052
WO 9739137
US4110165
BE827926
GB1563103
JP83009679B
JP55162993A
WO2005/075659
WO9641886
WO2007/030772
WO2004/092389
EP0970198

## Claims

1. A process for preparing clavulanic acid by using a microorganism, comprising the steps of:
a) providing a microorganism being capable of producing clavulanic acid, wherein said microorganism is modified or treated in that its enzyme d-(L-α-aminoadipyl)-L-cysteinyl-D-valine synthetase (ACVS) is selectively reduced or abolished,
b) cultivating said modified or treated microorganism or its descendant to produce clavulanic acid, and
c) isolating clavulanic acid.

2. The process according to claim 1, wherein in step a) a microorganism is provided, wherein
aA) the nucleic acid encoding the enzyme ACVS of said microorganism is selectively modified in that it has reduced or abolished activity of ACVS, or
bA) said microorganism has selectively reduced or abolished activity of ACVS.

3. The process according to claim 1 or 2, wherein the modified or treated microorganism is a result of any one of
(i) modifying or treating the enzyme compound ACVS,
(ii) reducing the level of enzyme ACVS, or completely preventing expression of a nucleic acid encoding ACVS in said microorganism, or
(iii) modifying or treating the nucleic acid encoding the enzyme ACVS.

4. The process according to any of claims 1 to 3, wherein
(i) modifying or treating the enzyme compound ACVS comprises applying a component that partially or completely inhibits enzyme activity of ACVS,
(ii) reducing the level of enzyme ACVS, or completely preventing expression of a nucleic acid encoding ACVS in said microorganism, comprises modifying regulatory elements of enzyme ACVS, and
(iii) modifying or treating the nucleic acid encoding the enzyme ACVS comprises introducing a mutation into said nucleic acid,
respectively to reduce or abolish activity of ACVS.

5. The process according to any of the preceding claims, wherein said microorganism belongs to the genus *Streptomyces* or the descendants thereof, preferably the microorganism is selected from the group consisting of *Streptomyces clavuligerus, Streptomyces clavuligerus, Streptomyces jumonjinensis, Streptomyces katsurahamanus* or the descendants thereof, more preferably the microorganism is *Streptomyces clavuligerus,* even more preferred the microorganism of step a) is *Streptomyces clavuligerus A TCC 27064* or *Streptomyces clavuligerus K213.*

6. The process according to any of the preceding claims, wherein the enzyme ACVS to be modified or treated is encoded by the *pcbAB* gene, preferably the enzyme ACVS to be modified or treated is encoded by a nucleic acid sequence that has a sequence identity of at least 70%, preferably at least 80%, more preferably at least 85%, even more preferably at least 90% and most preferably at least 95%, 97% or 98.5% to the nucleic acid sequence as listed under SEQ. ID. NO: 3.

7. A recombinant plasmid comprising a *pcbAB* gene being modified to encode partially or completely inactivated enzyme ACVS.

8. The recombinant plasmid according to claim 7, wherein the *pcbAB* gene has been modified by introducing a mutation, preferably by introducing deletion, insertion, substitution and/or point mutation.

9. A modified microorganism comprising the recombinant plasmid according to claim 7 or 8.

10. A microorganism that is modified or treated by selectively reducing or abolishing activity of its d-(L-α-aminoadipyl)-L-cysteinyl-D-valine synthetase (ACVS).

11. The microorganism according to claim 9 or 10, which has L-lysine ε-aminotransferase (LAT) and/or IPNS enzyme activity which is not reduced or abolished, and/or wherein, among the enzymes involved in the biosynthesis of cephamycin C, activity of ACVS alone is reduced or abolished.

12. The microorganism according to any of claims 9 to 11, wherein said microorganism belongs to the genus *Streptomyces* or the descendants thereof, preferably the microorganism is selected from the group consisting of *Streptomyces clavuligerus, Streptomyces clavuligerus, Streptomyces jumonjinensis, Streptomyces katsurahamanus* or the descendants thereof, more preferably the microorganism is *Streptomyces clavuligerus,* even more preferred the microorganism is *Streptomyces clavuligerus ATCC 27064* or *Streptomyces clavuligerus K213.*

13. Use of the recombinant plasmid according to claim 7 or 8 for modifying a microorganism.

14. Use of the microorganism according to any of claims 9 to 12 for the production of clavulanic acid.

15. The process according to any of claims 1 to 6, wherein, in a further step, the prepared clavulanic acid, which optionally is converted to a pharmaceutically acceptable salt or derivative thereof, is mixed with a suitable pharmaceutically acceptable carrier or excipient to prepare a pharmaceutical composition containing clavulanic acid.


SEQUENCE LISTING

<110> Lek Pharmaceuticals d.d.

<120> Process for production of clavulanic acid

<130> L 227

<160> 16

<170> BiSSAP 1.0

<210> 1
<211> 12169
<212> DNA
<213> Artificial Sequence

<220>
<221> source
<222> 1..12169
<223> /mol_type="DNA"
/note="Nucleic acid sequence comprising the gene pcbAB from the g
enome of Streptomyces clavuligerus, primer binding sites and codi
ng region"
/organism="Artificial Sequence"

<220>
<221> CDS
<222> 320..11578
<223> /transl_table=11
/translation="MLLKDEWRNRIAVRDDDPGVRATRQRDIVVDGREYTALKDALRAADGVSAG
ALALASLHSVMRAYGHGEQTVAAFVDATATAELKTAAVLPVIVDHIEHTRLTCAEAIRELDETLR
RKDSYTRADEVLQRGLFDALLVLAEREVALSELPSAPLVMVVRDDAARGRLCWTMAYAGELFEDT
TVAGVLEVVREVLGQYAGRPGDRVAEIELASREQRERLQRWNATDGDFPADQRLNDLVEAAVRRS
PDREAVVFGTQRLTYREVDARANRFAHWLLGPGLGVRSQQLVGIFLDKSDLGVVATLGIWKAGAA
YVPIDPAYPAERVRFAVGDTGLRGIVTNRHHAGRLREILGAEHADVTVVEIESVLDEQAAADTDG
LLSVKPELALGVRDLAYLTYTSGTTGVPKGVPKYHDSVVNSITDLSERYDMRRPGTERVALFASY
VFEPHLRQTLIALINGQTLVVVPEEVRLDPDRFPAYIEEHGVTYLNATGSVLQHFDLRRCTSLKR
LLLVGEELTAAGLRQLRERFSGRIVNEYAFTEAAFVTAVKEFAPGVTERADRSIGRPVRNVKWYV
LSQDLKRLPVGAIGELYIGGCGVAPGYLNRDDLTAERFLTNPYASEQDRARGMNARIYRTGDLAR
MLPSGEVEFMGRSDFQLKLNGVRVEPGEIEAQATEYAGVRKCVVIAREGAGGGSDRHLVGYYLTE
PGAGVTEAELLSFLERRLIRIMVPARMVRLESIPVNVNGKVDWRALPEVDLARPDTGGGAVGSTT
TGGVRGELREIWSAVLGLPVSRIGDGDDFFRLGGQSISCILLIARIRQRLGVFVGVEDVFTLRTL
GALADRLERDRGTAPADGTPQERRSAEGTADTTGTDGAEGADGGPVRLLANGLQQGLLYHALKRR
NGDDAYVMQSVHHYRCAIRPELMREAWQHARRTYPALRLRFEWAEEALQIVENDDRSVDWRFTDL
SGVADPAEQDARIRELQERDRTEPYDPAAGGLFRIYLIRRSEEFFSLLFSCHHIILDGWSLPVLH
DDVHRTYLRLVDGEPVTADAGSTGTPGSTGNTGNTGKTDTAYIAAQRDWEAHRGDHVDYWTRQIE
RIDERGDFGGLVREDIRYQVALSEYDHVKEHRTRRLALGAERTAALKAVCAEHSVTLHSVLQFVW
HKVLYAVGGGNTTVVGTIVSGRNPHIDGIENSVGLFINTLPVIVDHDEQAALGTDAAIADIQSAV
TAMAARSTVELGRLETGGMKRRLFDTLLVLENYPRLLSEDEERAHDELLRFEKAYDADKVDYPLA
VVAREEGEELTVTLWYAGELFEDATIDTLLGVAETLFDQVAEDISRRVDRLELVSPAMVEQYGVW
NRTTAPFPAGQTLHGVFERQAAAWPDETAVVFRDRRLTYRELNERANRLAHHLRSVAELRPNDLV
GLVVDKNELMITAILAVWKTGAGYVPIDPGYPDDRVAFMLADTSAKLVITNQGRVARIGELTGGA
LPVVDLDRLPDGNGADGDPVTDVTATDLAYAIYTSGTTGRPKAVLVEHRGVVNLQHSLAKLFGLD
RSEGVEALLSFSNYVFDHFVEQMTDALLNGQKLVVLDDTMRTDAERLYRYINDEQVTYLSGTPSV
LSLYDYSTTTSLTRIDAIGEDFTEPVFNKIRAMFPGTIINGYGPTEISITSHKRPYTVGERRSTK
SIGHPVDNYTAYVLNKSMQRVPVGGMGELYIGGVGVTRGYLNRDELTADRFVDNPFQTAEERAEG
RNARVYKTGDLVRWLPNGELECLGRTDLQVKIRGQRVELGEVEAALSSYPGVTRSLVIAREHRGA
GLASAQKYLVGFYVGTDDLDEQDVRQWMRTKLPEAVVPSRVLRITDIPVTPSGKLDVTRLPETDF
GPGDSGEYIAPSNDVETKLCGIWATVLGLAPERVGVTDDFFALGGDSLRAMVLAQAITTGFGRGL
GVATVLQHTTLGAQAKHIRESAAHGDGEQDGPVAGRAAGDGNPPVSLAQERLLFIDEFEGGTDA
YNIPFVLEIATGDAASTRDAVTGALRALLGRHAALRTLLRTDEHGVRRQYPLPAAEAAARFEVPL
HTVADRTALDERLVERSRTVFRLDEELPLVADLFTAGPADGRAYLSLVVHHSAFDGWSWSTFREE
LAALLRGVPVTDLRQVHGSYADFSLWQRQYLSGGRLAELTGYWTGALAGYETIRLPLDRPRPARF
DYRGREIDFDLDEGTARALRELARSARVSLYSVLLGAWALTLRVYTGQDDFVVGTPAANRGRPEF
DRTVGFFANLLALRVRVDADAGLAEYIRAVGADVVAAQVHGELPFEQLVKELNPEKDPSRHPVLQ
VNFTLQNVTDRAPVGGGAGAEPEFTEYKPDAGGFTATKFDLSATVTENAAGRLAGNLTYAAALFD
DDTAEGFIATFRQVLAEFARLADAGEQARVAEVDPVGAAERAALAAGPAGALQAAAAEALPAPRT
LHTVFEEQAAAWPDAVAVAHGATRLTYRELNERANQLAHYLRSVARIRPSDLIGLVVEKSELTVV

AILAVWKAGAGYLPIDPGHPDDRVTFMLADTEARLVVTQRAHGERLRRLAGDALPVVEIDALPQG
YRSTDDPVTDTTPADLAYAIYTSGTTGRPKAVLVPHRAVDSFRATLAGRYFGPPDTGRQGVLFLA
NYVFDFSVEQLALSVLGGHTLIVPPPSPAQDERFYEDANRAGLTYISGTPTQVQQFDLARLPGLR
TVLVAGEAFHERHFEKIREGWAGPILNAYGTTETAVYNTVRRFEPGDPYRNTLGLPLGNTRLYVL
GDGLKPLPPGAVGELYVAGECVTDGYLNRPELTGERFLPNPFDTGEEGREGRHPVLYRSGDLVRL
TRGGELQYLGRNDAQLKIRGLRIEPGEVEAVLATAPGVRECAVAAVADDRSPGNTRLVGYYVAAT
GTETDTDTGTGDAGGADEDAIFAVLRARLMPGMVPSLLIRMDGPLPMTINGKLDTKALPKAALPE
RRADRTAPRNRTEARLCQLWGDRLPGGTVGIDDDFFRSGGDSISALHLASQVQREIGRRVGVKDI
FDHPTVRHFAERVLAAPDRGRDPGEGGEAPEQGRLDGDCPLLPIQEWFFAKPLADRNWWNHNFAV
TAPELDVARLGQALARLVEHHDAFSLRYRSEGGGDGADERFVQRYDAAVPEVVLHELDVRGLSPE
EISERLARWQSGFDLERGPIHCAAYLHGFADGSARVWFALHHLVVDTVSWHILAQDLEILYHGGA
LEEKTSSYRQWARALAAYPADEAERRLWAEAVASLAATAQALPVPAPGAATLREEFALDAERTTA
LLTECHWAYDTTINDLLLTAVGGALRAVTGLAVNHVTEGHGRERFDGAPDVRDTVGWFTTMHPL
AVEAHPQDVGRGITATKEASRRVPHHGIGYGALFGRYGGERAPLPPVSFNYLGRVSDAGEQTDGP
SAGWRLDSAMSGSNISGRNRGADQFSVDVTMRCTGGRLVTAVDSRLDEETTRRFTDELRSWLERL
VAHTSAVSGENGGRARAAERAGAGTEFDPYILVDGDRAERTLFVFPPGEGGAESYLSNLAQRLPG
HRLVLFNNVHLHRPMESFEALADFYLPLLREIQPSGPYHLLGWSFGGVLSVEVSLRLARAGERIE
ELFLIDPYFDVRASSAAIGLPETEDILDPINYHWAPAREDLERLRANTGDVVLFRADEPNEIVRD
EEQRRLFDFYQRSSFNGLDALLPAESIEVHRLHGETHHSWVRNDRLVADICARVSASSPDAR"

<220>
<221> primer_bind
<222> 1..18

<220>
<221> primer_bind
<222> 12149..12169

<220>
<221> primer_bind
<222> 1011..1028

<220>
<221> primer_bind
<222> 11139..11156

<400> 1
cgctgacgat cgccgagcac gagatcgacc aggcccttca ggcgctggcg agcagtgtca 60

cggcggtcgc cgagagcgtc tgacgcccgg ccggccgggt gccccagcgg ggcccggccg 120

gccgcaccgc ggaacggaac acccctggcg cgtcgcccgg tgacacgctc tccgggcggc 180

tcaaccgcgc cacccccacg gcacgttcgc cttcacccgc acggagagcc cacgaatgat 240

gtcagcacgg tacccgagga ccgcagcgga gtggaccact cgcattcaag gagtgtcgag 300

cgagcgttgc gatcttgaaa tgctgctgaa ggacgagtgg cgcaacagga tcgcggtacg 360

ggacgacgac cccggtgtcc gtgcgacgag gcagcgggac atcgtcgtcg acgggcggga 420

gtacaccgct ctgaaggacg cgctgcgcgc cgccgacggg gtctcggcgg gcgcgctcgc 480

gctggcctcc ctgcacagcg tgatgcgcgc gtacggccat ggcgagcaga cggtggcggc 540

gttcgtcgac gcgacggcga cggcggagct gaagacggcc gccgtgctgc cggtgatcgt 600

cgaccatatc gagcacaccc ggctgacctg cgccgaggcg atccgggagc tggacgagac 660

gctgcgccgc aaggactcct acacccgtgc ggacgaggtg ctccagcggg ggctcttcga 720

tgccctgctg gtgctcgccg agcgcgaggt ggccctgtcc gagctgccgt cggccccgct 780

ggtcatggtc gtccgggacg acgcggcccg gggccggctg tgctggacga tggcgtacgc 840

gggcgagctg ttcgaggaca cgacggtcgc cggtgtgctg gaggtcgtcc gggaggtgct 900

cgggcagtac gccgggcggc cgggcgaccg ggtcgccgag atcgagctgg cctcgcggga 960

gcagcgcgag cggcttcagc ggtggaacgc gaccgatggc gacttcccgg cggaccagcg 1020
cctcaacgac ctggtcgagg cggccgtgcg gcggtcgccg gaccgtgagg cggtcgtctt 1080
cgggacacag cggctgacct atcgggaggt cgacgcgcgg gcgaaccggt tcgcgcactg 1140
gctgctcggt ccgggtctcg gggtccgttc gcagcagctc gtcgggatct ttctggacaa 1200
gagcgatctc ggggtggtcg ccacgctcgg catctggaag gcgggcgcgg cctatgtgcc 1260
gatcgacccg gcctatccgg cggagcgggt gcggttcgcg gtcggtgaca ccgggctgcg 1320
gggcatcgtc accaaccggc accacgcggg gcggctgcgg gagatcctgg gcgcggagca 1380
cgcggatgtg acggtcgtcg agatcgagtc cgtgctcgac gagcaggccg ccgccgacac 1440
cgacggcctg ctgagtgtga agcccgagct ggcgctgggg gtacgggatc tggcctatct 1500
cacctacacc tccggcacca ccggtgtgcc gaagggggtg cccaagtacc acgacagtgt 1560
cgtgaacagc atcacggacc tgtcggagcg gtacgacatg cggcggccgg gcacggagcg 1620
ggtcgcgctc ttcgcctcgt acgtcttcga gccgcatctg cggcagacgc tgatcgcgct 1680
catcaacggg cagacgctgg tcgtcgtccc cgaggaggtc cggctcgacc cggaccgctt 1740
ccccgcgtac atcgaggagc acggcgtcac gtacctcaac gcgacgggtt cggtgctgca 1800
acacttcgat ctgcgccgat gtaccagcct gaaacggctg ttgctggtcg gcgaggagct 1860
gacggcggcc gggctgcgcc agctccgcga gcggttctcc gggcggatcg tcaacgagta 1920
cgccttcacc gaggcggcgt tcgtcaccgc cgtgaaggag ttcgcgccgg gcgtcaccga 1980
gcgcgcggac cgcagcatcg gccgaccggt gcggaatgtg aagtggtatg tcctcagcca 2040
ggatctgaag cggttgccgg tcggggcgat cggcgagctg tacatcggcg gctgcggggt 2100
ggcgccgggc tatctgaacc gcgacgacct gacggccgag cggttcctga ccaatccgta 2160
cgcctcggag caggaccggg cgcgcgggat gaacgcccgg atctaccgga ccggtgacct 2220
ggcgcggatg ctgccgtccg gcgaggtcga gttcatgggc cgcagcgact tccagctcaa 2280
gctgaacggt gtccgggtgg agccgggcga gatcgaggcg caggccaccg agtatgcggg 2340
ggtccgcaag tgcgtggtca tcgcccgtga gggggcgggc ggcggcagcg accgccatct 2400
ggtcggctac tacctgacgg agcccggggc cggggtgacc gaggcggagc tgctgtcctt 2460
cctggagcgg cggctcatcc ggatcatggt cccggcgcgg atggtccggc tggagagcat 2520
ccccgtcaat gtcaacggca aggtcgactg gcgggcgctg cccgaggtgg acctcgcccg 2580
cccggacacg ggcggcggcg cggtgggctc caccacgacc gggggtgtcc ggggtgagct 2640
gcgcgagatc tggagcgcgg tcctcggcct tccggtgtcc cgtatcggcg acggcgacga 2700
cttcttccgg ctcggcggcc agagcatctc ctgcattctg ctcatcgccc ggatacggca 2760
gcggctgggc gtcttcgtcg gggtcgagga cgtcttcacg ctgaggacgc tgggcgcgct 2820
ggcggaccgg ctggagcgcg accggggcac ggcgcccgcg gacggcacgc cgcaggagcg 2880
gcggtccgcc gagggcacag cggacacgac gggcacggac ggtgcggagg gcgcggacgg 2940
cgggcccgtg cggctgctcg cgaacggtct ccagcagggg ctgctctacc acgccctgaa 3000
gcgccgcaac ggcgacgacg cctatgtgat gcagtcggtg caccactacc gctgcgccat 3060

ccgccccgag ctgatgcgcg aggcgtggca gcacgcgcgg cgcacgtacc ccgcgctgcg 3120
gctgcgtttc gagtgggccg aggaggccct ccagatcgtc gagaacgacg accgttccgt 3180
ggactggcgg ttcaccgatc tgagcggggt ggccgacccg gcggagcagg acgcccggat 3240
ccgggagctc caggagcggg accggaccga gccctacgac ccggcggcgg gcgggctgtt 3300
ccggatctat ctgatccgcc ggagcgaaga gttcttctcg ctgctgttca gctgccacca 3360
catcatcctg gacggctgga gcctgccggt cctccacgac gacgtgcacc ggacctatct 3420
gcggctggtg gacggcgagc cggtcacggc ggacgcgggg agcaccggta cccccgggag 3480
caccgggaac accgggaaca ccgggaagac ggacaccgcg tacatcgcgg cgcagcggga 3540
ctgggaggcg caccgcggcg accacgtcga ctactggacc cgtcagatcg agcggatcga 3600
cgagcggggc gacttcggcg ggctggtccg ggaggacatc cgctaccagg tggcgctgag 3660
cgagtacgac cacgtcaagg agcaccggac ccggcggctc gccctcggtg ccgagcggac 3720
cgcggcgctg aaggcggtct gcgcggagca ctccgtgacc ctgcactcgg tgctccagtt 3780
cgtgtggcac aaggtgctgt acgccgtcgg cggcgggaac accacggtgg tcggcacgat 3840
cgtgtccggg cgcaatccgc acatcgacgg gatcgagaac tccgtcggtc tgttcatcaa 3900
cacgctgccc gtgatcgtcg accacgacga gcaggcggcg ctgggcacgg acgcggccat 3960
cgcggacatc cagtccgccg tgaccgccat ggccgcccgg agcacggtgg agctgggccg 4020
gctggagacc ggcgggatga agcgccgcct cttcgacacc ctgctggtcc tggagaacta 4080
tccgcggctg ctctccgagg acgaggagcg cgcccacgac gagttgctgc gcttcgagaa 4140
ggcgtacgac gccgacaagg tcgactatcc gctggccgtg gtcgcccgcg aggagggtga 4200
ggagctgacc gtcaccctct ggtacgcggg tgagctgttc gaggacgcca cgatcgacac 4260
cctgctcggg gtggcggaga ccctgttcga ccaggtggcc gaggacatct cgcgccgggt 4320
ggaccggctg gagctggtct cgcccgcgat ggtcgagcag tacggggtgt ggaaccggac 4380
cacggccccg ttcccggccg ggcagaccct gcacggggtc ttcgagcggc aggccgccgc 4440
ctggccggac gagaccgcgg tggtcttccg cgaccggcgg ctgacctacc gggagctgaa 4500
cgagcgcgcc aaccggctgg cgcaccatct gcgctcggtc gcggagctgc gcccgaacga 4560
cctggtcggc ctggtcgtcg acaagaacga gctgatgatc accgcgatcc tggcggtgtg 4620
gaagacgggc gcgggctatg tcccgatcga cccgggctac cccgacgacc gggtggcgtt 4680
catgctggcg gacaccagcg ccaagctggt gatcaccaac caggggcggg tggcgcggat 4740
cggggagctg accggtggcg cgctgccggt ggtggatctc gaccggctgc ccgacggcaa 4800
cggcgcggac ggcgacccgg tcaccgatgt caccgccacg gatctcgcgt acgcgatcta 4860
cacctccggc accaccggca ggcccaaggc ggtgctggtc gagcaccggg gtgtggtgaa 4920
cctccagcac tcgctggcga agctgttcgg cctggaccgg agcgagggcg tcgaggcgct 4980
gctgtcgttc tcgaactacg tcttcgacca cttcgtggag cagatgacgg acgccctgct 5040
caacgggcag aagctggtcg tcctcgacga caccatgcgc acggacgccg agcggctgta 5100

ccggtacatc aacgacgagc aggtcaccta tctctccggc acgccgtcgg tgctctcgct 5160
ctacgactac tcgacgacga cctcgctgac ccggatcgac gcgatcggcg aggacttcac 5220
cgagccggtc ttcaacaaga tccgcgccat gttcccgggg acgatcatca acgggtacgg 5280
gccgacggag atctcgatca ccagccacaa gcggccctac acggtcggcg agcggcggtc 5340
gacgaagagc atcggccacc cggtggacaa ctacacggcc tatgtcctga acaagtcgat 5400
gcagcgggtg ccggtcggcg gcatgggcga gctgtacatc ggcggtgtcg gcgtcacccg 5460
cggctatctg aaccgcgacg agctgacggc ggaccgcttc gtcgacaacc ccttccagac 5520
cgccgaggag cgggccgagg ggcgcaacgc gcgcgtctac aagaccggtg acctggtgcg 5580
ctggctgccc aacggcgagc tggagtgcct gggccgcacc gacctccagg tcaagatccg 5640
gggacagcgg gtcgagctgg gcgaggtcga ggccgcgctc tcctcgtacc cgggcgtcac 5700
ccgttccctg gtcatcgcgc gggagcaccg gggcgcgggg ctcgcgtcgg cgcagaagta 5760
cctcgtgggc ttctatgtcg gcacggacga tctggacgag caggacgtca ggcagtggat 5820
gcggacgaag ctgcccgagg cggtcgtccc ctcccgggtg ctgcggatca ccgacatccc 5880
ggtgacgccg agcggcaagc tggacgtcac acggctcccg gagaccgact tcggcccggg 5940
tgacagcggc gagtacatcg cgccgtcgaa cgatgtcgag accaagctgt gcggcatctg 6000
ggccacggtg ctcggtctcg ccccggagcg ggtcggcgtc accgacgact tcttcgccct 6060
cggcggcgac agcctccggg cgatggtgct ggcgcaggcc atcaccaccg gcttcggccg 6120
gggtctcggg gtcgcgaccg tgctccagca cacgaccctc ggggcgcagg cgaagcacat 6180
ccgcgagagc gccgcgcacg gcgacgggga gcaggacggg ccggtcgcgg gccgggccgc 6240
ggcgggcgac ggaaacccgc cggtgtcgct cgcgcaggaa cgtctgctgt tcatcgacga 6300
gttcgagggc ggcaccgacg cctacaacat ccccttcgtc ctggagatcg cgaccgggga 6360
cgccgcctcg acccgggacg cggtcaccgg ggcgctgcgc gccctgctgg ggcggcacgc 6420
ggcgctgcgc accctgctgc ggaccgacga gcacggcgtc cggcggcagt acccgctgcc 6480
cgccgccgag gccgcggccc ggttcgaggt cccgctgcac acggtcgcgg accgcacggc 6540
cctggacgag cggctggtgg agcggtcccg cacggtcttc cggctggacg aggagctgcc 6600
gctcgtggcc gacctcttca cggccggtcc ggcggacggc cgcgcgtatc tgagtctggt 6660
cgtccaccac agcgccttcg acggctggtc gtggagcacc ttccgggagg agctggcggc 6720
cctgctgcgc ggtgtccccg tgacggatct gcggcaggtc cacggcagct acgccgactt 6780
ctcgctgtgg cagcggcagt atctgtccgg gggcaggctg gcggagctga ccgggtactg 6840
gacgggggcg ctggccgggt acgagacgat ccggctgccg ctggaccgcc cgcgtccggc 6900
gcgcttcgac taccgggccc gggagatcga cttcgacctc gacgagggga cggcgcgggc 6960
gctgcgcgag ctggcgcggt ccgcgcgggt gagcctgtac agcgtgctgc tcggcgcgtg 7020
ggcgctcacc ctgcgcgtct acaccggcca ggacgacttc gtcgtgggca cgcccgccgc 7080
gaaccggggg cgcccggagt tcgaccggac cgtcggcttc ttcgcgaacc tgctggcgct 7140
gcgggtccgg gtcgacgcgg acgccggtct cgcggagtac atccgcgcgg tcggcgcgga 7200

cgtggtcgcg gcccaggtcc acggcgagct gccgttcgag cagctcgtga aggagctgaa 7260
cccggagaag gacccgagcc ggcaccccgt gctccaggtc aacttcaccc tccagaacgt 7320
caccgaccgc gctcccgtcg gcggcggggc cggggcggag ccggagttca ccgagtacaa 7380
gccggacgcc gggggcttca cggccaccaa gttcgatctg tcggcgacgg tcaccgagaa 7440
cgccgcgggg cggctcgcgg gcaacctcac ctacgccgcc gcgctcttcg acgacgacac 7500
cgccgaaggg ttcatcgcca ccttccggca ggtcctcgcg gagttcgcgc ggctcgcgga 7560
cgccggggag caggccaggg tcgcggaggt ggacccggtc ggcgcggcgg agcgcgcggc 7620
cctcgcggcc ggtcccgccg gggccctcca ggccgcggcc gccgaggcgc tgcccgcccc 7680
ccggaccctg cacacggtct tcgaggagca ggccgccgcc tggccggacg ccgtcgcggt 7740
cgcccacggc gcgacccggc tcacctaccg ggagctgaac gagcgcgcca accagctcgc 7800
gcactatctg cgctccgtgg cgcggatccg tccgagcgat ctgatcggcc tggtcgtcga 7860
gaagagcgag ctgaccgtcg tcgcgatcct cgcggtgtgg aaggcgggcg cgggctatct 7920
gccgatcgac ccgggccacc ccgacgaccg ggtgaccttc atgctggcgg acaccgaggc 7980
ccggctggtg gtcacccagc gggcccacgg ggagcggctg cgccgcctcg cgggcgacgc 8040
gctgccggtg gtggagatcg acgcgctgcc gcagggctac cgctcgaccg acgacccggt 8100
cacggacacc accccggcgg acctcgcgta cgcgatctac acctcgggca ccaccggccg 8160
ccccaaggcg gtgctggttc cgcaccgcgc ggtggacagc ttccgcgcga cgctggccgg 8220
acggtacttc ggcccgcccg acaccggccg ccagggggtg ctgttcctcg ccaactatgt 8280
cttcgacttc tcggtggagc agctcgcgct gtccgtcctc ggcggtcaca ccctgatcgt 8340
cccgccgccg tcgcccgcgc aggacgagcg gttctacgag gacgcgaacc gcgccgggct 8400
gacgtacatc agcgggacgc ccacccaggt ccagcagttc gacctggcgc ggctgccggg 8460
gctgcgcaca gtgctggtcg ccggtgaggc gttccacgag cgccacttcg agaagatccg 8520
cgagggctgg gcggggccga tcctcaacgc gtacggcacc accgagacgg ccgtctacaa 8580
caccgtccgg cgcttcgagc cgggtgaccc ctaccgcaac accctgggtc tgccgctcgg 8640
gaacacccgg ctgtacgtcc tcggcgacgg gctgaagccg ctgccgccgg gcgcggtcgg 8700
cgagctgtat gtggccggtg agtgtgtgac ggacggctat ctcaaccggc cggagctgac 8760
cggtgagcgc ttcctgccga acccgttcga caccggggag gaggggcggg agggccgcca 8820
cccggtcctc taccgcagtg gcgacctggt ccggctgacc cgcggcggcg agctccagta 8880
cctgggccgc aacgacgccc agctcaagat ccgcgggctg cggatcgagc cgggcgaggt 8940
ggaggcggtc ctcgccaccg ctccgggggt ccgcgagtgc gcggtcgccg ccgtggccga 9000
cgaccgctcc ccggggaaca cccggctggt cggctactac gtcgccgcca ccggcacgga 9060
gacggacacg gacaccggca cgggcgacgc cgggggcgcc gacgaggacg cgatcttcgc 9120
ggtgctgcgg gcgcggctga tgccgggcat ggtgccctcg ctgctgatcc gcatggacgg 9180
cccgctgccg atgaccatca acggcaagct ggacaccaag gcgctgccga aggcggcgct 9240

gccggagcgg cgcgcggacc gcacggcccc gcgcaaccgg accgaggccc ggctgtgcca 9300
gctctggggc gaccggctcc ccggtggcac ggtcggcatc gacgacgact tcttccgctc 9360
gggcggcgac agcatcagcg ccctgcacct ggcgagccag gtgcagcggg agatcgggcg 9420
cagggtcggc gtcaaggaca tcttcgacca tccgaccgtc cggcacttcg ccgagcgggt 9480
gctggccgcc ccggaccggg ggcgggaccc cggcgagggc ggcgaggcgc ccgagcaggg 9540
ccggctggac ggtgactgtc cgctgctgcc catccaggag tggttcttcg ccaagccgct 9600
cgccgaccgg aactggtgga accacaactt cgccgtcacc gccccggagc tggacgtggc 9660
gaggctgggc caggcgctgg cgcggctggt cgagcaccat gacgcgttca gcctgcggta 9720
ccgctccgag ggcggcggcg acggcgcgga cgagcggttc gtccagcgct acgacgccgc 9780
cgtgcccgag gtcgtcctgc acgagctgga tgtgcgcggg ctctcccccg aggagatctc 9840
cgaacggctc gcccgctggc agagcggctt cgacctggag cgcggaccga tccactgcgc 9900
ggcctatctg cacggcttcg ccgatggctc ggcccgggtc tggttcgccc tgcaccacct 9960
ggtcgtcgac accgtgagct ggcacatcct cgcgcaggac ctggagatcc tgtaccacgg 10020
gggcgcgctg gaggagaaga cgagcagcta ccgccagtgg gcgcgggcgc tcgccgcgta 10080
cccggcggac gaggccgagc ggcggctgtg ggcggaggcc gtcgcgtccc tggcggccac 10140
cgcacaggcg ctgccggtcc cggcgccggg cgcggcgacg ctccgggagg agttcgcgct 10200
cgacgcggag cggaccacgg cgctgctgac cgagtgccac tgggcgtacg acaccaccat 10260
caacgatctg ctgctgacgg cggtcggggg cgcgctgcgg gccgtcaccg gcctggcggt 10320
caaccatgtc accgtggagg gccacggccg ggagcggttc gacggggcgc ccgacgtccg 10380
ggacacggtg ggctggttca cgaccatgca cccgctggcc gtggaggcgc acccgcagga 10440
cgtggggcgc ggcatcaccg ccacgaagga ggcttcccgg cgggtgccgc accacggcat 10500
cggatacggg gccctcttcg gccgctacgg cggggagcgg gcgccgctgc cgccggtgag 10560
cttcaactac ctcggccggg tctccgacgc cggggagcag acggacggcc cctccgcggg 10620
ctggcggctc gactcggcga tgtcggggag caacatctcc ggccgcaacc ggggcgccga 10680
ccagttcagt gtggacgtga ccatgcgatg taccggcggc cggctcgtca ccgcggtgga 10740
cagccgactg gacgaggaga ccacccggcg cttcacggac gagctgagga gctggctgga 10800
gcggctcgtc gcccacacct cggcggtgtc gggcgagaac ggcggccggg cgcgcgccgc 10860
cgagcgggcc ggggccggga cggagttcga cccgtacatc ctcgtcgacg gggaccgcgc 10920
cgagcgcacc ctcttcgtct tcccgcccgg tgagggcggc gccgagagct atctgagcaa 10980
tctcgcgcag cggctgcccg gccaccggct ggtgctcttc aacaatgtgc atctgcaccg 11040
gccgatggag tccttcgagg cgctggccga cttctatctc cccctcctcc gggagatcca 11100
gccgtccggc ccgtaccacc tgctgggctg gagcttcggc ggggtgctct cggtggaggt 11160
ctcgctccgg ctggcccggg cgggcgagcg gatcgaggag ctgttcctca tcgacccgta 11220
cttcgacgtc cgggcctcgt cggcggcgat cgggctgccg gagaccgagg acatcctcga 11280
tccgatcaac taccactggg cgcccgcccg ggaggatctg gagcggctgc gcgcgaacac 11340

cggcgatgtg gtgctgttcc gggcggacga gccgaacgag atcgtccggg acgaggagca 11400

gcgccggctg ttcgacttct accagcggtc ctccttcaac gggctggacg cgctgctgcc 11460

cgcggagtcc atcgaggtcc accggctgca cggtgagacc caccattcgt gggtacgaaa 11520

cgaccggctg gtcgccgaca tctgtgcgcg tgtctcggcg tcgtcgccgg atgcccggtg 11580

aacggccggg cgctgagtcc aaggagggtt tcatgccagt tctgatgccg tcggcccacg 11640

ttccgaccat cgacatctcg ccgctgttcg gaaccgacgc cgccgcgaag aagcgcgtcg 11700

ccgaggagat acacggggcc tgccgcggct cgggcttctt ctacgccacg aaccacggcg 11760

tggacgtcca gcagctccag gacgtggtga acgagttcca cggcgccatg accgaccagg 11820

agaagcacga cctggcgatc cacgcgtaca acccggacaa cccgcatgtg cgcaacggct 11880

actacaaggc ggtcccgggc aggaaggccg tcgagtcctt ctgttacctc aacccggact 11940

tcggcgagga ccacccgatg atcgccgcgg ggacgccgat gcacgaggtg aacctctggc 12000

ccgacgagga gcggcacccg cgcttccggc cgttctgcga gggctactac cggcagatgc 12060

tgaagctctc caccgtgctc atgcgggggc tggcgctggc gctcgggagg ccggagcact 12120

tcttcgacgc ggcgctcgcc gagcaggact ccctgtcgtc cgtctcgct 12169

<210> 2
<211> 3753
<212> PRT
<213> Artificial Sequence

<220>
<221> SOURCE
<222> 1..3753
<223> /mol_type="protein"
/note="[CDS]:320..11578 from SEQ ID NO 1"
/organism="Artificial Sequence"

<400> 2
Met Leu Leu Lys Asp Glu Trp Arg Asn Arg Ile Ala Val Arg Asp Asp
1               5                   10                  15
Asp Pro Gly Val Arg Ala Thr Arg Gln Arg Asp Ile Val Val Asp Gly
            20                  25                  30
Arg Glu Tyr Thr Ala Leu Lys Asp Ala Leu Arg Ala Ala Asp Gly Val
        35                  40                  45
Ser Ala Gly Ala Leu Ala Leu Ala Ser Leu His Ser Val Met Arg Ala
    50                  55                  60
Tyr Gly His Gly Glu Gln Thr Val Ala Ala Phe Val Asp Ala Thr Ala
65                  70                  75                  80
Thr Ala Glu Leu Lys Thr Ala Ala Val Leu Pro Val Ile Val Asp His
                85                  90                  95
Ile Glu His Thr Arg Leu Thr Cys Ala Glu Ala Ile Arg Glu Leu Asp
            100                 105                 110
Glu Thr Leu Arg Arg Lys Asp Ser Tyr Thr Arg Ala Asp Glu Val Leu
        115                 120                 125
Gln Arg Gly Leu Phe Asp Ala Leu Leu Val Leu Ala Glu Arg Glu Val
    130                 135                 140
Ala Leu Ser Glu Leu Pro Ser Ala Pro Leu Val Met Val Val Arg Asp
145                 150                 155                 160
Asp Ala Ala Arg Gly Arg Leu Cys Trp Thr Met Ala Tyr Ala Gly Glu
                165                 170                 175
Leu Phe Glu Asp Thr Thr Val Ala Gly Val Leu Glu Val Val Arg Glu
            180                 185                 190
Val Leu Gly Gln Tyr Ala Gly Arg Pro Gly Asp Arg Val Ala Glu Ile
        195                 200                 205

Glu Leu Ala Ser Arg Glu Gln Arg Glu Arg Leu Gln Arg Trp Asn Ala
        210                 215                 220
Thr Asp Gly Asp Phe Pro Ala Asp Gln Arg Leu Asn Asp Leu Val Glu
225                 230                 235                 240
Ala Ala Val Arg Arg Ser Pro Asp Arg Glu Ala Val Val Phe Gly Thr
                245                 250                 255
Gln Arg Leu Thr Tyr Arg Glu Val Asp Ala Arg Ala Asn Arg Phe Ala
            260                 265                 270
His Trp Leu Leu Gly Pro Gly Leu Gly Val Arg Ser Gln Gln Leu Val
        275                 280                 285
Gly Ile Phe Leu Asp Lys Ser Asp Leu Gly Val Val Ala Thr Leu Gly
    290                 295                 300
Ile Trp Lys Ala Gly Ala Ala Tyr Val Pro Ile Asp Pro Ala Tyr Pro
305                 310                 315                 320
Ala Glu Arg Val Arg Phe Ala Val Gly Asp Thr Gly Leu Arg Gly Ile
                325                 330                 335
Val Thr Asn Arg His His Ala Gly Arg Leu Arg Glu Ile Leu Gly Ala
            340                 345                 350
Glu His Ala Asp Val Thr Val Val Glu Ile Glu Ser Val Leu Asp Glu
        355                 360                 365
Gln Ala Ala Ala Asp Thr Asp Gly Leu Leu Ser Val Lys Pro Glu Leu
    370                 375                 380
Ala Leu Gly Val Arg Asp Leu Ala Tyr Leu Thr Tyr Thr Ser Gly Thr
385                 390                 395                 400
Thr Gly Val Pro Lys Gly Val Pro Lys Tyr His Asp Ser Val Val Asn
                405                 410                 415
Ser Ile Thr Asp Leu Ser Glu Arg Tyr Asp Met Arg Arg Pro Gly Thr
            420                 425                 430
Glu Arg Val Ala Leu Phe Ala Ser Tyr Val Phe Glu Pro His Leu Arg
        435                 440                 445
Gln Thr Leu Ile Ala Leu Ile Asn Gly Gln Thr Leu Val Val Val Pro
    450                 455                 460
Glu Glu Val Arg Leu Asp Pro Asp Arg Phe Pro Ala Tyr Ile Glu Glu
465                 470                 475                 480
His Gly Val Thr Tyr Leu Asn Ala Thr Gly Ser Val Leu Gln His Phe
                485                 490                 495
Asp Leu Arg Arg Cys Thr Ser Leu Lys Arg Leu Leu Leu Val Gly Glu
            500                 505                 510
Glu Leu Thr Ala Ala Gly Leu Arg Gln Leu Arg Glu Arg Phe Ser Gly
        515                 520                 525
Arg Ile Val Asn Glu Tyr Ala Phe Thr Glu Ala Ala Phe Val Thr Ala
    530                 535                 540
Val Lys Glu Phe Ala Pro Gly Val Thr Glu Arg Ala Asp Arg Ser Ile
545                 550                 555                 560
Gly Arg Pro Val Arg Asn Val Lys Trp Tyr Val Leu Ser Gln Asp Leu
                565                 570                 575
Lys Arg Leu Pro Val Gly Ala Ile Gly Glu Leu Tyr Ile Gly Gly Cys
            580                 585                 590
Gly Val Ala Pro Gly Tyr Leu Asn Arg Asp Asp Leu Thr Ala Glu Arg
        595                 600                 605
Phe Leu Thr Asn Pro Tyr Ala Ser Glu Gln Asp Arg Ala Arg Gly Met
    610                 615                 620
Asn Ala Arg Ile Tyr Arg Thr Gly Asp Leu Ala Arg Met Leu Pro Ser
625                 630                 635                 640
Gly Glu Val Glu Phe Met Gly Arg Ser Asp Phe Gln Leu Lys Leu Asn
                645                 650                 655
Gly Val Arg Val Glu Pro Gly Glu Ile Glu Ala Gln Ala Thr Glu Tyr
            660                 665                 670
Ala Gly Val Arg Lys Cys Val Val Ile Ala Arg Glu Gly Ala Gly Gly
        675                 680                 685
Gly Ser Asp Arg His Leu Val Gly Tyr Tyr Leu Thr Glu Pro Gly Ala
    690                 695                 700
Gly Val Thr Glu Ala Glu Leu Leu Ser Phe Leu Glu Arg Arg Leu Ile
705                 710                 715                 720
Arg Ile Met Val Pro Ala Arg Met Val Arg Leu Glu Ser Ile Pro Val
                725                 730                 735
Asn Val Asn Gly Lys Val Asp Trp Arg Ala Leu Pro Glu Val Asp Leu
            740                 745                 750
Ala Arg Pro Asp Thr Gly Gly Gly Ala Val Gly Ser Thr Thr Thr Gly

        755                 760                 765
Gly Val Arg Gly Glu Leu Arg Glu Ile Trp Ser Ala Val Leu Gly Leu
    770                 775                 780
Pro Val Ser Arg Ile Gly Asp Gly Asp Asp Phe Phe Arg Leu Gly Gly
785                 790                 795                 800
Gln Ser Ile Ser Cys Ile Leu Leu Ile Ala Arg Ile Arg Gln Arg Leu
                805                 810                 815
Gly Val Phe Val Gly Val Glu Asp Val Phe Thr Leu Arg Thr Leu Gly
            820                 825                 830
Ala Leu Ala Asp Arg Leu Glu Arg Asp Arg Gly Thr Ala Pro Ala Asp
        835                 840                 845
Gly Thr Pro Gln Glu Arg Arg Ser Ala Glu Gly Thr Ala Asp Thr Thr
    850                 855                 860
Gly Thr Asp Gly Ala Glu Gly Ala Asp Gly Gly Pro Val Arg Leu Leu
865                 870                 875                 880
Ala Asn Gly Leu Gln Gln Gly Leu Leu Tyr His Ala Leu Lys Arg Arg
                885                 890                 895
Asn Gly Asp Asp Ala Tyr Val Met Gln Ser Val His His Tyr Arg Cys
            900                 905                 910
Ala Ile Arg Pro Glu Leu Met Arg Glu Ala Trp Gln His Ala Arg Arg
        915                 920                 925
Thr Tyr Pro Ala Leu Arg Leu Arg Phe Glu Trp Ala Glu Glu Ala Leu
    930                 935                 940
Gln Ile Val Glu Asn Asp Asp Arg Ser Val Asp Trp Arg Phe Thr Asp
945                 950                 955                 960
Leu Ser Gly Val Ala Asp Pro Ala Glu Gln Asp Ala Arg Ile Arg Glu
                965                 970                 975
Leu Gln Glu Arg Asp Arg Thr Glu Pro Tyr Asp Pro Ala Ala Gly Gly
            980                 985                 990
Leu Phe Arg Ile Tyr Leu Ile Arg Arg Ser Glu Glu Phe Phe Ser Leu
        995                 1000                1005
Leu Phe Ser Cys His His Ile Ile Leu Asp Gly Trp Ser Leu Pro Val
    1010                1015                1020
Leu His Asp Asp Val His Arg Thr Tyr Leu Arg Leu Val Asp Gly Glu
1025                1030                1035                1040
Pro Val Thr Ala Asp Ala Gly Ser Thr Gly Thr Pro Gly Ser Thr Gly
                1045                1050                1055
Asn Thr Gly Asn Thr Gly Lys Thr Asp Thr Ala Tyr Ile Ala Ala Gln
            1060                1065                1070
Arg Asp Trp Glu Ala His Arg Gly Asp His Val Asp Tyr Trp Thr Arg
        1075                1080                1085
Gln Ile Glu Arg Ile Asp Glu Arg Gly Asp Phe Gly Gly Leu Val Arg
    1090                1095                1100
Glu Asp Ile Arg Tyr Gln Val Ala Leu Ser Glu Tyr Asp His Val Lys
1105                1110                1115                1120
Glu His Arg Thr Arg Arg Leu Ala Leu Gly Ala Glu Arg Thr Ala Ala
                1125                1130                1135
Leu Lys Ala Val Cys Ala Glu His Ser Val Thr Leu His Ser Val Leu
            1140                1145                1150
Gln Phe Val Trp His Lys Val Leu Tyr Ala Val Gly Gly Gly Asn Thr
        1155                1160                1165
Thr Val Val Gly Thr Ile Val Ser Gly Arg Asn Pro His Ile Asp Gly
    1170                1175                1180
Ile Glu Asn Ser Val Gly Leu Phe Ile Asn Thr Leu Pro Val Ile Val
1185                1190                1195                1200
Asp His Asp Glu Gln Ala Ala Leu Gly Thr Asp Ala Ala Ile Ala Asp
                1205                1210                1215
Ile Gln Ser Ala Val Thr Ala Met Ala Ala Arg Ser Thr Val Glu Leu
            1220                1225                1230
Gly Arg Leu Glu Thr Gly Gly Met Lys Arg Arg Leu Phe Asp Thr Leu
        1235                1240                1245
Leu Val Leu Glu Asn Tyr Pro Arg Leu Leu Ser Glu Asp Glu Glu Arg
    1250                1255                1260
Ala His Asp Glu Leu Leu Arg Phe Glu Lys Ala Tyr Asp Ala Asp Lys
1265                1270                1275                1280
Val Asp Tyr Pro Leu Ala Val Val Ala Arg Glu Glu Gly Glu Glu Leu
                1285                1290                1295
Thr Val Thr Leu Trp Tyr Ala Gly Glu Leu Phe Glu Asp Ala Thr Ile
            1300                1305                1310

Asp Thr Leu Leu Gly Val Ala Glu Thr Leu Phe Asp Gln Val Ala Glu
        1315                1320                1325
Asp Ile Ser Arg Arg Val Asp Arg Leu Glu Leu Val Ser Pro Ala Met
    1330                1335                1340
Val Glu Gln Tyr Gly Val Trp Asn Arg Thr Thr Ala Pro Phe Pro Ala
1345                1350                1355                1360
Gly Gln Thr Leu His Gly Val Phe Glu Arg Gln Ala Ala Ala Trp Pro
                1365                1370                1375
Asp Glu Thr Ala Val Val Phe Arg Asp Arg Arg Leu Thr Tyr Arg Glu
            1380                1385                1390
Leu Asn Glu Arg Ala Asn Arg Leu Ala His His Leu Arg Ser Val Ala
        1395                1400                1405
Glu Leu Arg Pro Asn Asp Leu Val Gly Leu Val Val Asp Lys Asn Glu
    1410                1415                1420
Leu Met Ile Thr Ala Ile Leu Ala Val Trp Lys Thr Gly Ala Gly Tyr
1425                1430                1435                1440
Val Pro Ile Asp Pro Gly Tyr Pro Asp Asp Arg Val Ala Phe Met Leu
                1445                1450                1455
Ala Asp Thr Ser Ala Lys Leu Val Ile Thr Asn Gln Gly Arg Val Ala
            1460                1465                1470
Arg Ile Gly Glu Leu Thr Gly Gly Ala Leu Pro Val Val Asp Leu Asp
        1475                1480                1485
Arg Leu Pro Asp Gly Asn Gly Ala Asp Gly Asp Pro Val Thr Asp Val
    1490                1495                1500
Thr Ala Thr Asp Leu Ala Tyr Ala Ile Tyr Thr Ser Gly Thr Thr Gly
1505                1510                1515                1520
Arg Pro Lys Ala Val Leu Val Glu His Arg Gly Val Val Asn Leu Gln
                1525                1530                1535
His Ser Leu Ala Lys Leu Phe Gly Leu Asp Arg Ser Glu Gly Val Glu
            1540                1545                1550
Ala Leu Leu Ser Phe Ser Asn Tyr Val Phe Asp His Phe Val Glu Gln
        1555                1560                1565
Met Thr Asp Ala Leu Leu Asn Gly Gln Lys Leu Val Val Leu Asp Asp
    1570                1575                1580
Thr Met Arg Thr Asp Ala Glu Arg Leu Tyr Arg Tyr Ile Asn Asp Glu
1585                1590                1595                1600
Gln Val Thr Tyr Leu Ser Gly Thr Pro Ser Val Leu Ser Leu Tyr Asp
                1605                1610                1615
Tyr Ser Thr Thr Thr Ser Leu Thr Arg Ile Asp Ala Ile Gly Glu Asp
            1620                1625                1630
Phe Thr Glu Pro Val Phe Asn Lys Ile Arg Ala Met Phe Pro Gly Thr
        1635                1640                1645
Ile Ile Asn Gly Tyr Gly Pro Thr Glu Ile Ser Ile Thr Ser His Lys
    1650                1655                1660
Arg Pro Tyr Thr Val Gly Glu Arg Arg Ser Thr Lys Ser Ile Gly His
1665                1670                1675                1680
Pro Val Asp Asn Tyr Thr Ala Tyr Val Leu Asn Lys Ser Met Gln Arg
                1685                1690                1695
Val Pro Val Gly Gly Met Gly Glu Leu Tyr Ile Gly Gly Val Gly Val
            1700                1705                1710
Thr Arg Gly Tyr Leu Asn Arg Asp Glu Leu Thr Ala Asp Arg Phe Val
        1715                1720                1725
Asp Asn Pro Phe Gln Thr Ala Glu Glu Arg Ala Glu Gly Arg Asn Ala
    1730                1735                1740
Arg Val Tyr Lys Thr Gly Asp Leu Val Arg Trp Leu Pro Asn Gly Glu
1745                1750                1755                1760
Leu Glu Cys Leu Gly Arg Thr Asp Leu Gln Val Lys Ile Arg Gly Gln
                1765                1770                1775
Arg Val Glu Leu Gly Glu Val Glu Ala Ala Leu Ser Ser Tyr Pro Gly
            1780                1785                1790
Val Thr Arg Ser Leu Val Ile Ala Arg Glu His Arg Gly Ala Gly Leu
        1795                1800                1805
Ala Ser Ala Gln Lys Tyr Leu Val Gly Phe Tyr Val Gly Thr Asp Asp
    1810                1815                1820
Leu Asp Glu Gln Asp Val Arg Gln Trp Met Arg Thr Lys Leu Pro Glu
1825                1830                1835                1840
Ala Val Val Pro Ser Arg Val Leu Arg Ile Thr Asp Ile Pro Val Thr
                1845                1850                1855
Pro Ser Gly Lys Leu Asp Val Thr Arg Leu Pro Glu Thr Asp Phe Gly

1860                1865                1870
Pro Gly Asp Ser Gly Glu Tyr Ile Ala Pro Ser Asn Asp Val Glu Thr
        1875                1880                1885
Lys Leu Cys Gly Ile Trp Ala Thr Val Leu Gly Leu Ala Pro Glu Arg
    1890                1895                1900
Val Gly Val Thr Asp Asp Phe Phe Ala Leu Gly Gly Asp Ser Leu Arg
1905                1910                1915                1920
Ala Met Val Leu Ala Gln Ala Ile Thr Thr Gly Phe Gly Arg Gly Leu
                1925                1930                1935
Gly Val Ala Thr Val Leu Gln His Thr Thr Leu Gly Ala Gln Ala Lys
            1940                1945                1950
His Ile Arg Glu Ser Ala Ala His Gly Asp Gly Glu Gln Asp Gly Pro
        1955                1960                1965
Val Ala Gly Arg Ala Ala Ala Gly Asp Gly Asn Pro Pro Val Ser Leu
    1970                1975                1980
Ala Gln Glu Arg Leu Leu Phe Ile Asp Glu Phe Glu Gly Gly Thr Asp
1985                1990                1995                2000
Ala Tyr Asn Ile Pro Phe Val Leu Glu Ile Ala Thr Gly Asp Ala Ala
                2005                2010                2015
Ser Thr Arg Asp Ala Val Thr Gly Ala Leu Arg Ala Leu Leu Gly Arg
            2020                2025                2030
His Ala Ala Leu Arg Thr Leu Leu Arg Thr Asp Glu His Gly Val Arg
        2035                2040                2045
Arg Gln Tyr Pro Leu Pro Ala Ala Glu Ala Ala Ala Arg Phe Glu Val
    2050                2055                2060
Pro Leu His Thr Val Ala Asp Arg Thr Ala Leu Asp Glu Arg Leu Val
2065                2070                2075                2080
Glu Arg Ser Arg Thr Val Phe Arg Leu Asp Glu Glu Leu Pro Leu Val
                2085                2090                2095
Ala Asp Leu Phe Thr Ala Gly Pro Ala Asp Gly Arg Ala Tyr Leu Ser
            2100                2105                2110
Leu Val Val His His Ser Ala Phe Asp Gly Trp Ser Trp Ser Thr Phe
        2115                2120                2125
Arg Glu Glu Leu Ala Ala Leu Leu Arg Gly Val Pro Val Thr Asp Leu
    2130                2135                2140
Arg Gln Val His Gly Ser Tyr Ala Asp Phe Ser Leu Trp Gln Arg Gln
2145                2150                2155                2160
Tyr Leu Ser Gly Gly Arg Leu Ala Glu Leu Thr Gly Tyr Trp Thr Gly
                2165                2170                2175
Ala Leu Ala Gly Tyr Glu Thr Ile Arg Leu Pro Leu Asp Arg Pro Arg
            2180                2185                2190
Pro Ala Arg Phe Asp Tyr Arg Gly Arg Glu Ile Asp Phe Asp Leu Asp
        2195                2200                2205
Glu Gly Thr Ala Arg Ala Leu Arg Glu Leu Ala Arg Ser Ala Arg Val
    2210                2215                2220
Ser Leu Tyr Ser Val Leu Leu Gly Ala Trp Ala Leu Thr Leu Arg Val
2225                2230                2235                2240
Tyr Thr Gly Gln Asp Asp Phe Val Val Gly Thr Pro Ala Ala Asn Arg
                2245                2250                2255
Gly Arg Pro Glu Phe Asp Arg Thr Val Gly Phe Phe Ala Asn Leu Leu
            2260                2265                2270
Ala Leu Arg Val Arg Val Asp Ala Asp Ala Gly Leu Ala Glu Tyr Ile
        2275                2280                2285
Arg Ala Val Gly Ala Asp Val Val Ala Ala Gln Val His Gly Glu Leu
    2290                2295                2300
Pro Phe Glu Gln Leu Val Lys Glu Leu Asn Pro Glu Lys Asp Pro Ser
2305                2310                2315                2320
Arg His Pro Val Leu Gln Val Asn Phe Thr Leu Gln Asn Val Thr Asp
                2325                2330                2335
Arg Ala Pro Val Gly Gly Gly Ala Gly Ala Glu Pro Glu Phe Thr Glu
            2340                2345                2350
Tyr Lys Pro Asp Ala Gly Gly Phe Thr Ala Thr Lys Phe Asp Leu Ser
        2355                2360                2365
Ala Thr Val Thr Glu Asn Ala Ala Gly Arg Leu Ala Gly Asn Leu Thr
    2370                2375                2380
Tyr Ala Ala Ala Leu Phe Asp Asp Asp Thr Ala Glu Gly Phe Ile Ala
2385                2390                2395                2400
Thr Phe Arg Gln Val Leu Ala Glu Phe Ala Arg Leu Ala Asp Ala Gly
                2405                2410                2415

Glu Gln Ala Arg Val Ala Glu Val Asp Pro Val Gly Ala Ala Glu Arg
                2420                2425                2430
Ala Ala Leu Ala Ala Gly Pro Ala Gly Ala Leu Gln Ala Ala Ala Ala
        2435                2440                2445
Glu Ala Leu Pro Ala Pro Arg Thr Leu His Thr Val Phe Glu Glu Gln
    2450                2455                2460
Ala Ala Ala Trp Pro Asp Ala Val Ala Val Ala His Gly Ala Thr Arg
2465                2470                2475                2480
Leu Thr Tyr Arg Glu Leu Asn Glu Arg Ala Asn Gln Leu Ala His Tyr
                2485                2490                2495
Leu Arg Ser Val Ala Arg Ile Arg Pro Ser Asp Leu Ile Gly Leu Val
            2500                2505                2510
Val Glu Lys Ser Glu Leu Thr Val Val Ala Ile Leu Ala Val Trp Lys
        2515                2520                2525
Ala Gly Ala Gly Tyr Leu Pro Ile Asp Pro Gly His Pro Asp Asp Arg
    2530                2535                2540
Val Thr Phe Met Leu Ala Asp Thr Glu Ala Arg Leu Val Val Thr Gln
2545                2550                2555                2560
Arg Ala His Gly Glu Arg Leu Arg Arg Leu Ala Gly Asp Ala Leu Pro
                2565                2570                2575
Val Val Glu Ile Asp Ala Leu Pro Gln Gly Tyr Arg Ser Thr Asp Asp
            2580                2585                2590
Pro Val Thr Asp Thr Thr Pro Ala Asp Leu Ala Tyr Ala Ile Tyr Thr
        2595                2600                2605
Ser Gly Thr Thr Gly Arg Pro Lys Ala Val Leu Val Pro His Arg Ala
    2610                2615                2620
Val Asp Ser Phe Arg Ala Thr Leu Ala Gly Arg Tyr Phe Gly Pro Pro
2625                2630                2635                2640
Asp Thr Gly Arg Gln Gly Val Leu Phe Leu Ala Asn Tyr Val Phe Asp
                2645                2650                2655
Phe Ser Val Glu Gln Leu Ala Leu Ser Val Leu Gly Gly His Thr Leu
            2660                2665                2670
Ile Val Pro Pro Pro Ser Pro Ala Gln Asp Glu Arg Phe Tyr Glu Asp
        2675                2680                2685
Ala Asn Arg Ala Gly Leu Thr Tyr Ile Ser Gly Thr Pro Thr Gln Val
    2690                2695                2700
Gln Gln Phe Asp Leu Ala Arg Leu Pro Gly Leu Arg Thr Val Leu Val
2705                2710                2715                2720
Ala Gly Glu Ala Phe His Glu Arg His Phe Glu Lys Ile Arg Glu Gly
                2725                2730                2735
Trp Ala Gly Pro Ile Leu Asn Ala Tyr Gly Thr Thr Glu Thr Ala Val
            2740                2745                2750
Tyr Asn Thr Val Arg Arg Phe Glu Pro Gly Asp Pro Tyr Arg Asn Thr
        2755                2760                2765
Leu Gly Leu Pro Leu Gly Asn Thr Arg Leu Tyr Val Leu Gly Asp Gly
    2770                2775                2780
Leu Lys Pro Leu Pro Pro Gly Ala Val Gly Glu Leu Tyr Val Ala Gly
2785                2790                2795                2800
Glu Cys Val Thr Asp Gly Tyr Leu Asn Arg Pro Glu Leu Thr Gly Glu
                2805                2810                2815
Arg Phe Leu Pro Asn Pro Phe Asp Thr Gly Glu Glu Gly Arg Glu Gly
            2820                2825                2830
Arg His Pro Val Leu Tyr Arg Ser Gly Asp Leu Val Arg Leu Thr Arg
        2835                2840                2845
Gly Gly Glu Leu Gln Tyr Leu Gly Arg Asn Asp Ala Gln Leu Lys Ile
    2850                2855                2860
Arg Gly Leu Arg Ile Glu Pro Gly Glu Val Glu Ala Val Leu Ala Thr
2865                2870                2875                2880
Ala Pro Gly Val Arg Glu Cys Ala Val Ala Ala Val Ala Asp Asp Arg
                2885                2890                2895
Ser Pro Gly Asn Thr Arg Leu Val Gly Tyr Tyr Val Ala Ala Thr Gly
            2900                2905                2910
Thr Glu Thr Asp Thr Asp Thr Gly Thr Gly Asp Ala Gly Gly Ala Asp
        2915                2920                2925
Glu Asp Ala Ile Phe Ala Val Leu Arg Ala Arg Leu Met Pro Gly Met
    2930                2935                2940
Val Pro Ser Leu Leu Ile Arg Met Asp Gly Pro Leu Pro Met Thr Ile
2945                2950                2955                2960
Asn Gly Lys Leu Asp Thr Lys Ala Leu Pro Lys Ala Ala Leu Pro Glu

                2965                2970                2975
Arg Arg Ala Asp Arg Thr Ala Pro Arg Asn Arg Thr Glu Ala Arg Leu
            2980                2985                2990
Cys Gln Leu Trp Gly Asp Arg Leu Pro Gly Gly Thr Val Gly Ile Asp
        2995                3000                3005
Asp Asp Phe Phe Arg Ser Gly Gly Asp Ser Ile Ser Ala Leu His Leu
    3010                3015                3020
Ala Ser Gln Val Gln Arg Glu Ile Gly Arg Arg Val Gly Val Lys Asp
3025                3030                3035                3040
Ile Phe Asp His Pro Thr Val Arg His Phe Ala Glu Arg Val Leu Ala
                3045                3050                3055
Ala Pro Asp Arg Gly Arg Asp Pro Gly Glu Gly Gly Glu Ala Pro Glu
            3060                3065                3070
Gln Gly Arg Leu Asp Gly Asp Cys Pro Leu Leu Pro Ile Gln Glu Trp
        3075                3080                3085
Phe Phe Ala Lys Pro Leu Ala Asp Arg Asn Trp Trp Asn His Asn Phe
    3090                3095                3100
Ala Val Thr Ala Pro Glu Leu Asp Val Ala Arg Leu Gly Gln Ala Leu
3105                3110                3115                3120
Ala Arg Leu Val Glu His His Asp Ala Phe Ser Leu Arg Tyr Arg Ser
                3125                3130                3135
Glu Gly Gly Gly Asp Gly Ala Asp Glu Arg Phe Val Gln Arg Tyr Asp
            3140                3145                3150
Ala Ala Val Pro Glu Val Val Leu His Glu Leu Asp Val Arg Gly Leu
        3155                3160                3165
Ser Pro Glu Glu Ile Ser Glu Arg Leu Ala Arg Trp Gln Ser Gly Phe
    3170                3175                3180
Asp Leu Glu Arg Gly Pro Ile His Cys Ala Ala Tyr Leu His Gly Phe
3185                3190                3195                3200
Ala Asp Gly Ser Ala Arg Val Trp Phe Ala Leu His His Leu Val Val
                3205                3210                3215
Asp Thr Val Ser Trp His Ile Leu Ala Gln Asp Leu Glu Ile Leu Tyr
            3220                3225                3230
His Gly Gly Ala Leu Glu Glu Lys Thr Ser Ser Tyr Arg Gln Trp Ala
        3235                3240                3245
Arg Ala Leu Ala Ala Tyr Pro Ala Asp Glu Ala Glu Arg Arg Leu Trp
    3250                3255                3260
Ala Glu Ala Val Ala Ser Leu Ala Ala Thr Ala Gln Ala Leu Pro Val
3265                3270                3275                3280
Pro Ala Pro Gly Ala Ala Thr Leu Arg Glu Glu Phe Ala Leu Asp Ala
                3285                3290                3295
Glu Arg Thr Thr Ala Leu Leu Thr Glu Cys His Trp Ala Tyr Asp Thr
            3300                3305                3310
Thr Ile Asn Asp Leu Leu Leu Thr Ala Val Gly Gly Ala Leu Arg Ala
        3315                3320                3325
Val Thr Gly Leu Ala Val Asn His Val Thr Val Glu Gly His Gly Arg
    3330                3335                3340
Glu Arg Phe Asp Gly Ala Pro Asp Val Arg Asp Thr Val Gly Trp Phe
3345                3350                3355                3360
Thr Thr Met His Pro Leu Ala Val Glu Ala His Pro Gln Asp Val Gly
                3365                3370                3375
Arg Gly Ile Thr Ala Thr Lys Glu Ala Ser Arg Arg Val Pro His His
            3380                3385                3390
Gly Ile Gly Tyr Gly Ala Leu Phe Gly Arg Tyr Gly Gly Glu Arg Ala
        3395                3400                3405
Pro Leu Pro Pro Val Ser Phe Asn Tyr Leu Gly Arg Val Ser Asp Ala
    3410                3415                3420
Gly Glu Gln Thr Asp Gly Pro Ser Ala Gly Trp Arg Leu Asp Ser Ala
3425                3430                3435                3440
Met Ser Gly Ser Asn Ile Ser Gly Arg Asn Arg Gly Ala Asp Gln Phe
                3445                3450                3455
Ser Val Asp Val Thr Met Arg Cys Thr Gly Gly Arg Leu Val Thr Ala
            3460                3465                3470
Val Asp Ser Arg Leu Asp Glu Glu Thr Thr Arg Arg Phe Thr Asp Glu
        3475                3480                3485
Leu Arg Ser Trp Leu Glu Arg Leu Val Ala His Thr Ser Ala Val Ser
    3490                3495                3500
Gly Glu Asn Gly Gly Arg Ala Arg Ala Ala Glu Arg Ala Gly Ala Gly
3505                3510                3515                3520

Thr Glu Phe Asp Pro Tyr Ile Leu Val Asp Gly Asp Arg Ala Glu Arg
                3525                3530                3535
Thr Leu Phe Val Phe Pro Pro Gly Glu Gly Gly Ala Glu Ser Tyr Leu
            3540                3545                3550
Ser Asn Leu Ala Gln Arg Leu Pro Gly His Arg Leu Val Leu Phe Asn
        3555                3560                3565
Asn Val His Leu His Arg Pro Met Glu Ser Phe Glu Ala Leu Ala Asp
    3570                3575                3580
Phe Tyr Leu Pro Leu Leu Arg Glu Ile Gln Pro Ser Gly Pro Tyr His
3585                3590                3595                3600
Leu Leu Gly Trp Ser Phe Gly Gly Val Leu Ser Val Glu Val Ser Leu
                3605                3610                3615
Arg Leu Ala Arg Ala Gly Glu Arg Ile Glu Glu Leu Phe Leu Ile Asp
            3620                3625                3630
Pro Tyr Phe Asp Val Arg Ala Ser Ser Ala Ala Ile Gly Leu Pro Glu
        3635                3640                3645
Thr Glu Asp Ile Leu Asp Pro Ile Asn Tyr His Trp Ala Pro Ala Arg
    3650                3655                3660
Glu Asp Leu Glu Arg Leu Arg Ala Asn Thr Gly Asp Val Val Leu Phe
3665                3670                3675                3680
Arg Ala Asp Glu Pro Asn Glu Ile Val Arg Asp Glu Glu Gln Arg Arg
                3685                3690                3695
Leu Phe Asp Phe Tyr Gln Arg Ser Ser Phe Asn Gly Leu Asp Ala Leu
            3700                3705                3710
Leu Pro Ala Glu Ser Ile Glu Val His Arg Leu His Gly Glu Thr His
        3715                3720                3725
His Ser Trp Val Arg Asn Asp Arg Leu Val Ala Asp Ile Cys Ala Arg
    3730                3735                3740
Val Ser Ala Ser Ser Pro Asp Ala Arg
3745                3750

<210> 3
<211> 11262
<212> DNA
<213> Streptomyces clavuligerus

<220>
<221> source
<222> 1..11262
<223> /mol_type="DNA"
      /note="coding region of pcbAB"
      /organism="Streptomyces clavuligerus"

<400> 3
atgctgctga aggacgagtg gcgcaacagg atcgcggtac gggacgacga ccccggtgtc      60

cgtgcgacga ggcagcggga catcgtcgtc gacgggcggg agtacaccgc tctgaaggac     120

gcgctgcgcg ccgccgacgg ggtctcggcg ggcgcgctcg cgctggcctc cctgcacagc     180

gtgatgcgcg cgtacggcca tggcgagcag acggtggcgg cgttcgtcga cgcgacggcg     240

acggcggagc tgaagacggc cgccgtgctg ccggtgatcg tcgaccatat cgagcacacc     300

cggctgacct gcgccgaggc gatccgggag ctggacgaga cgctgcgccg caaggactcc     360

tacacccgtg cggacgaggt gctccagcgg gggctcttcg atgccctgct ggtgctcgcc     420

gagcgcgagg tggccctgtc cgagctgccg tcggccccgc tggtcatggt cgtccgggac     480

gacgcggccc ggggccggct gtgctggacg atggcgtacg cgggcgagct gttcgaggac     540

acgacggtcg ccggtgtgct ggaggtcgtc cgggaggtgc tcgggcagta cgccgggcgg     600

ccgggcgacc gggtcgccga gatcgagctg gcctcgcggg agcagcgcga gcggcttcag     660

cggtggaacg cgaccgatgg cgacttcccg gcggaccagc gcctcaacga cctggtcgag     720

gcggccgtgc ggcggtcgcc ggaccgtgag gcggtcgtct tcgggacaca gcggctgacc     780

tatcgggagg tcgacgcgcg ggcgaaccgg ttcgcgcact ggctgctcgg tccgggtctc 840
ggggtccgtt cgcagcagct cgtcgggatc tttctggaca agagcgatct cggggtggtc 900
gccacgctcg gcatctggaa ggcgggcgcg gcctatgtgc cgatcgaccc ggcctatccg 960
gcggagcggg tgcggttcgc ggtcggtgac accgggctgc ggggcatcgt caccaaccgg 1020
caccacgcgg ggcggctgcg ggagatcctg ggcgcggagc acgcggatgt gacggtcgtc 1080
gagatcgagt ccgtgctcga cgagcaggcc gccgccgaca ccgacggcct gctgagtgtg 1140
aagcccgagc tggcgctggg ggtacgggat ctggcctatc tcacctacac ctccggcacc 1200
accggtgtgc cgaagggggt gcccaagtac cacgacagtg tcgtgaacag catcacggac 1260
ctgtcggagc ggtacgacat gcggcggccg ggcacggagc gggtcgcgct cttcgcctcg 1320
tacgtcttcg agccgcatct gcggcagacg ctgatcgcgc tcatcaacgg gcagacgctg 1380
gtcgtcgtcc ccgaggaggt ccggctcgac ccggaccgct tccccgcgta catcgaggag 1440
cacggcgtca cgtacctcaa cgcgacgggt tcggtgctgc aacacttcga tctgcgccga 1500
tgtaccagcc tgaaacggct gttgctggtc ggcgaggagc tgacggcggc cgggctgcgc 1560
cagctccgcg agcggttctc cgggcggatc gtcaacgagt acgccttcac cgaggcggcg 1620
ttcgtcaccg ccgtgaagga gttcgcgccg ggcgtcaccg agcgcgcgga ccgcagcatc 1680
ggccgaccgg tgcggaatgt gaagtggtat gtcctcagcc aggatctgaa gcggttgccg 1740
gtcggggcga tcggcgagct gtacatcggc ggctgcgggg tggcgccggg ctatctgaac 1800
cgcgacgacc tgacggccga gcggttcctg accaatccgt acgcctcgga gcaggaccgg 1860
gcgcgcggga tgaacgcccg gatctaccgg accggtgacc tggcgcggat gctgccgtcc 1920
ggcgaggtcg agttcatggg ccgcagcgac ttccagctca agctgaacgg tgtccgggtg 1980
gagccgggcg agatcgaggc gcaggccacc gagtatgcgg gggtccgcaa gtgcgtggtc 2040
atcgcccgtg agggggcggg cggcggcagc gaccgccatc tggtcggcta ctacctgacg 2100
gagcccgggg ccggggtgac cgaggcggag ctgctgtcct tcctggagcg gcggctcatc 2160
cggatcatgg tcccggcgcg gatggtccgg ctggagagca tccccgtcaa tgtcaacggc 2220
aaggtcgact ggcgggcgct gcccgaggtg gacctcgccc gcccggacac gggcggcggc 2280
gcggtgggct ccaccacgac cgggggtgtc cggggtgagc tgcgcgagat ctggagcgcg 2340
gtcctcggcc ttccggtgtc ccgtatcggc gacggcgacg acttcttccg gctcggcggc 2400
cagagcatct cctgcattct gctcatcgcc cggatacggc agcggctggg cgtcttcgtc 2460
ggggtcgagg acgtcttcac gctgaggacg ctgggcgcgc tggcggaccg gctggagcgc 2520
gaccggggca cggcgcccgc ggacggcacg ccgcaggagc ggcggtccgc cgagggcaca 2580
gcggacacga cgggcacgga cggtgcggag ggcgcggacg gcgggcccgt gcggctgctc 2640
gcgaacggtc tccagcaggg gctgctctac cacgccctga agcgccgcaa cggcgacgac 2700
gcctatgtga tgcagtcggt gcaccactac cgctgcgcca tccgccccga gctgatgcgc 2760
gaggcgtggc agcacgcgcg gcgcacgtac cccgcgctgc ggctgcgttt cgagtgggcc 2820

gaggaggccc tccagatcgt cgagaacgac gaccgttccg tggactggcg gttcaccgat 2880
ctgagcgggg tggccgaccc ggcggagcag gacgcccgga tccgggagct ccaggagcgg 2940
gaccggaccg agccctacga cccggcggcg ggcgggctgt tccggatcta tctgatccgc 3000
cggagcgaag agttcttctc gctgctgttc agctgccacc acatcatcct ggacggctgg 3060
agcctgccgg tcctccacga cgacgtgcac cggacctatc tgcggctggt ggacggcgag 3120
ccggtcacgg cggacgcggg gagcaccggt acccccggga gcaccgggaa caccgggaac 3180
accgggaaga cggacaccgc gtacatcgcg gcgcagcggg actggaggcg gcaccgcggc 3240
gaccacgtcg actactggac ccgtcagatc gagcggatcg acgagcgggg cgacttcggc 3300
gggctggtcc gggaggacat ccgctaccag gtggcgctga gcgagtacga ccacgtcaag 3360
gagcaccgga cccggcggct cgccctcggt gccgagcgga ccgcggcgct gaaggcggtc 3420
tgcgcggagc actccgtgac cctgcactcg gtgctccagt tcgtgtggca caaggtgctg 3480
tacgccgtcg gcggcgggaa caccacggtg gtcggcacga tcgtgtccgg gcgcaatccg 3540
cacatcgacg ggatcgagaa ctccgtcggt ctgttcatca acacgctgcc cgtgatcgtc 3600
gaccacgacg agcaggcggc gctgggcacg gacgcggcca tcgcggacat ccagtccgcc 3660
gtgaccgcca tggccgcccg gagcacggtg gagctgggcc ggctggagac cggcgggatg 3720
aagcgccgcc tcttcgacac cctgctggtc ctggagaact atccgcggct gctctccgag 3780
gacgaggagc gcgcccacga cgagttgctg cgcttcgaga aggcgtacga cgccgacaag 3840
gtcgactatc cgctggccgt ggtcgcccgc gaggagggtg aggagctgac cgtcaccctc 3900
tggtacgcgg gtgagctgtt cgaggacgcc acgatcgaca ccctgctcgg ggtggcggag 3960
accctgttcg accaggtggc cgaggacatc tcgcgccggg tggaccggct ggagctggtc 4020
tcgcccgcga tggtcgagca gtacggggtg tggaaccgga ccacggcccc gttcccggcc 4080
gggcagaccc tgcacggggt cttcgagcgg caggccgccg cctggccgga cgagaccgcg 4140
gtggtcttcc gcgaccggcg gctgacctac cgggagctga acgagcgcgc caaccggctg 4200
gcgcaccatc tgcgctcggt cgcggagctg cgcccgaacg acctggtcgg cctggtcgtc 4260
gacaagaacg agctgatgat caccgcgatc ctggcggtgt ggaagacggg cgcgggctat 4320
gtcccgatcg acccgggcta ccccgacgac cgggtggcgt tcatgctggc ggacaccagc 4380
gccaagctgg tgatcaccaa ccaggggcgg gtggcgcgga tcggggagct gaccggtggc 4440
gcgctgccgg tggtggatct cgaccggctg cccgacggca acggcgcgga cggcgacccg 4500
gtcaccgatg tcaccgccac ggatctcgcg tacgcgatct acacctccgg caccaccggc 4560
aggcccaagg cggtgctggt cgagcaccgg ggtgtggtga acctccagca ctcgctggcg 4620
aagctgttcg gcctggaccg gagcgagggc gtcgaggcgc tgctgtcgtt ctcgaactac 4680
gtcttcgacc acttcgtgga gcagatgacg gacgccctgc tcaacgggca gaagctggtc 4740
gtcctcgacg acaccatgcg cacggacgcc gagcggctgt accggtacat caacgacgag 4800
caggtcacct atctctccgg cacgccgtcg gtgctctcgc tctacgacta ctcgacgacg 4860
acctcgctga cccggatcga cgcgatcggc gaggacttca ccgagccggt cttcaacaag 4920

atccgcgcca tgttcccggg gacgatcatc aacgggtacg ggccgacgga gatctcgatc 4980
accagccaca agcggcccta cacggtcggc gagcggcggt cgacgaagag catcggccac 5040
ccggtggaca actacacggc ctatgtcctg aacaagtcga tgcagcgggt gccggtcggc 5100
ggcatgggcg agctgtacat cggcggtgtc ggcgtcaccc gcggctatct gaaccgcgac 5160
gagctgacgg cggaccgctt cgtcgacaac cccttccaga ccgccgagga gcgggccgag 5220
gggcgcaacg cgcgcgtcta caagaccggt gacctggtgc gctggctgcc caacggcgag 5280
ctggagtgcc tgggccgcac cgacctccag gtcaagatcc ggggacagcg ggtcgagctg 5340
ggcgaggtcg aggccgcgct ctcctcgtac ccgggcgtca cccgttccct ggtcatcgcg 5400
cgggagcacc ggggcgcggg gctcgcgtcg gcgcagaagt acctcgtggg cttctatgtc 5460
ggcacggacg atctggacga gcaggacgtc aggcagtgga tgcggacgaa gctgcccgag 5520
gcggtcgtcc cctcccgggt gctgcggatc accgacatcc cggtgacgcc gagcggcaag 5580
ctggacgtca cacggctccc ggagaccgac ttcggcccgg gtgacagcgg cgagtacatc 5640
gcgccgtcga acgatgtcga gaccaagctg tgcggcatct gggccacggt gctcggtctc 5700
gccccggagc gggtcggcgt caccgacgac ttcttcgccc tcggcggcga cagcctccgg 5760
gcgatggtgc tggcgcaggc catcaccacc ggcttcggcc ggggtctcgg ggtcgcgacc 5820
gtgctccagc acacgaccct cggggcgcag gcgaagcaca tccgcgagag cgccgcgcac 5880
ggcgacgggg agcaggacgg gccggtcgcg ggccgggccg cggcgggcga cggaaacccg 5940
ccggtgtcgc tcgcgcagga acgtctgctg ttcatcgacg agttcgaggg cggcaccgac 6000
gcctacaaca tccccttcgt cctggagatc gcgaccgggg acgccgcctc gacccgggac 6060
gcggtcaccg gggcgctgcg cgccctgctg gggcggcacg cggcgctgcg caccctgctg 6120
cggaccgacg agcacggcgt ccggcggcag tacccgctgc ccgccgccga ggccgcggcc 6180
cggttcgagg tcccgctgca cacggtcgcg gaccgcacgg ccctggacga gcggctggtg 6240
gagcggtccc gcacggtctt ccggctggac gaggagctgc cgctcgtggc cgacctcttc 6300
acggccggtc cggcggacgg ccgcgcgtat ctgagtctgg tcgtccacca cagcgccttc 6360
gacggctggt cgtggagcac cttccgggag gagctggcgg ccctgctgcg cggtgtcccc 6420
gtgacggatc tgcggcaggt ccacggcagc tacgccgact tctcgctgtg gcagcggcag 6480
tatctgtccg ggggcaggct ggcggagctg accgggtact ggacgggggc gctggccggg 6540
tacgagacga tccggctgcc gctggaccgc ccgcgtccgg cgcgcttcga ctaccggggc 6600
cgggagatcg acttcgacct cgacgagggg acggcgcggg cgctgcgcga gctggcgcgg 6660
tccgcgcggg tgagcctgta cagcgtgctg ctcggcgcgt gggcgctcac cctgcgcgtc 6720
tacaccggcc aggacgactt cgtcgtgggc acgcccgccg cgaaccgggg gcgcccggag 6780
ttcgaccgga ccgtcggctt cttcgcgaac ctgctggcgc tgcgggtccg ggtcgacgcg 6840
gacgccggtc tcgcggagta catccgcgcg gtcggcgcgg acgtggtcgc ggcccaggtc 6900
cacggcgagc tgccgttcga gcagctcgtg aaggagctga acccggagaa ggacccgagc 6960

cggcaccccg tgctccaggt caacttcacc ctccagaacg tcaccgaccg cgctcccgtc 7020
ggcggcgggg ccggggcgga gccggagttc accgagtaca agccggacgc cgggggcttc 7080
acggccacca agttcgatct gtcggcgacg gtcaccgaga acgccgcggg gcggctcgcg 7140
ggcaacctca cctacgccgc cgcgctcttc gacgacgaca ccgccgaagg gttcatcgcc 7200
accttccggc aggtcctcgc ggagttcgcg cggctcgcgg acgccgggga gcaggccagg 7260
gtcgcggagg tggacccggt cggcgcggcg gagcgcgcgg ccctcgcggc cggtcccgcc 7320
ggggccctcc aggccgcggc cgccgaggcg ctgcccgccc cccggaccct gcacacggtc 7380
ttcgaggagc aggccgccgc ctggccggac gccgtcgcgg tcgcccacgg cgcgacccgg 7440
ctcacctacc gggagctgaa cgagcgcgcc aaccagctcg cgcactatct gcgctccgtg 7500
gcgcggatcc gtccgagcga tctgatcggc ctggtcgtcg agaagagcga gctgaccgtc 7560
gtcgcgatcc tcgcggtgtg gaaggcgggc gcgggctatc tgccgatcga cccgggccac 7620
cccgacgacc gggtgacctt catgctggcg gacaccgagg cccggctggt ggtcacccag 7680
cgggcccacg gggagcggct gcgccgcctc gcgggcgacg cgctgccggt ggtggagatc 7740
gacgcgctgc cgcagggcta ccgctcgacc gacgacccgg tcacggacac caccccggcg 7800
gacctcgcgt acgcgatcta cacctcgggc accaccggcc gccccaaggc ggtgctggtt 7860
ccgcaccgcg cggtggacag cttccgcgcg acgctggccg gacggtactt cggcccgccc 7920
gacaccggcc gccagggggt gctgttcctc gccaactatg tcttcgactt ctcggtggag 7980
cagctcgcgc tgtccgtcct cggcggtcac accctgatcg tcccgccgcc gtcgcccgcg 8040
caggacgagc ggttctacga ggacgcgaac cgcgccgggc tgacgtacat cagcgggacg 8100
cccacccagg tccagcagtt cgacctggcg cggctgccgg ggctgcgcac agtgctggtc 8160
gccggtgagg cgttccacga gcgccacttc gagaagatcc gcgagggctg ggcggggccg 8220
atcctcaacg cgtacggcac caccgagacg gccgtctaca acaccgtccg gcgcttcgag 8280
ccgggtgacc cctaccgcaa caccctgggt ctgccgctcg ggaacacccg gctgtacgtc 8340
ctcggcgacg ggctgaagcc gctgccgccg ggcgcggtcg gcgagctgta tgtggccggt 8400
gagtgtgtga cggacggcta tctcaaccgg ccggagctga ccggtgagcg cttcctgccg 8460
aacccgttcg acaccgggga ggaggggcgg gagggccgcc acccggtcct ctaccgcagt 8520
ggcgacctgg tccggctgac ccgcggcggc gagctccagt acctgggccg caacgacgcc 8580
cagctcaaga tccgcgggct gcggatcgag ccgggcgagg tggaggcggt cctcgccacc 8640
gctccggggg tccgcgagtg cgcggtcgcc gccgtggccg acgaccgctc cccggggaac 8700
acccggctgg tcggctacta cgtcgccgcc accggcacgg agacggacac ggacaccggc 8760
acgggcgacg ccgggggcgc cgacgaggac gcgatcttcg cggtgctgcg ggcgcggctg 8820
atgccgggca tggtgccctc gctgctgatc cgcatggacg gcccgctgcc gatgaccatc 8880
aacggcaagc tggacaccaa ggcgctgccg aaggcggcgc tgccggagcg gcgcgcggac 8940
cgcacggccc cgcgcaaccg gaccgaggcc cggctgtgcc agctctgggg cgaccggctc 9000
cccggtggca cggtcggcat cgacgacgac ttcttccgct cgggcggcga cagcatcagc 9060

gccctgcacc tggcgagcca ggtgcagcgg gagatcgggc gcagggtcgg cgtcaaggac 9120
atcttcgacc atccgaccgt ccggcacttc gccgagcggg tgctggccgc cccggaccgg 9180
gggcgggacc ccggcgaggg cggcgaggcg cccgagcagg gccggctgga cggtgactgt 9240
ccgctgctgc ccatccagga gtggttcttc gccaagccgc tcgccgaccg gaactggtgg 9300
aaccacaact tcgccgtcac cgccccggag ctggacgtgg cgaggctggg ccaggcgctg 9360
gcgcggctgg tcgagcacca tgacgcgttc agcctgcggt accgctccga gggcggcggc 9420
gacggcgcgg acgagcggtt cgtccagcgc tacgacgccg ccgtgcccga ggtcgtcctg 9480
cacgagctgg atgtgcgcgg gctctccccc gaggagatct ccgaacggct cgcccgctgg 9540
cagagcggct tcgacctgga gcgcggaccg atccactgcg cggcctatct gcacggcttc 9600
gccgatggct cggcccgggt ctggttcgcc ctgcaccacc tggtcgtcga caccgtgagc 9660
tggcacatcc tcgcgcagga cctggagatc ctgtaccacg ggggcgcgct ggaggagaag 9720
acgagcagct accgccagtg ggcgcgggcg ctcgccgcgt acccggcgga cgaggccgag 9780
cggcggctgt gggcggaggc cgtcgcgtcc ctggcggcca ccgcacaggc gctgccggtc 9840
ccggcgccgg gcgcggcgac gctccgggag gagttcgcgc tcgacgcgga gcggaccacg 9900
gcgctgctga ccgagtgcca ctgggcgtac gacaccacca tcaacgatct gctgctgacg 9960
gcggtcgggg gcgcgctgcg ggccgtcacc ggcctggcgg tcaaccatgt caccgtggag 10020
ggccacggcc gggagcggtt cgacggggcg cccgacgtcc gggacacggt gggctggttc 10080
acgaccatgc acccgctggc cgtggaggcg cacccgcagg acgtggggcg cggcatcacc 10140
gccacgaagg aggcttcccg gcgggtgccg caccacggca tcggatacgg ggccctcttc 10200
ggccgctacg gcggggagcg ggcgccgctg ccgccggtga gcttcaacta cctcggccgg 10260
gtctccgacg ccggggagca gacggacggg ccctccgcgg gctggcggct cgactcggcg 10320
atgtcgggga gcaacatctc cggccgcaac cggggcgccg accagttcag tgtggacgtg 10380
accatgcgat gtaccggcgg ccggctcgtc accgcggtgg acagccgact ggacgaggag 10440
accacccggc gcttcacgga cgagctgagg agctggctgg agcggctcgt cgcccacacc 10500
tcggcggtgt cgggcgagaa cggcggccgg gcgcgcgccg ccgagcgggc cggggccggg 10560
acggagttcg acccgtacat cctcgtcgac ggggaccgcg ccgagcgcac cctcttcgtc 10620
ttcccgcccg gtgagggcgg cgccgagagc tatctgagca atctcgcgca gcggctgccc 10680
ggccaccggc tggtgctctt caacaatgtg catctgcacc ggccgatgga gtccttcgag 10740
gcgctggccg acttctatct ccccctcctc cgggagatcc agccgtccgg cccgtaccac 10800
ctgctgggct ggagcttcgg cggggtgctc tcggtggagg tctcgctccg gctggcccgg 10860
gcgggcgagc ggatcgagga gctgttcctc atcgacccgt acttcgacgt ccgggcctcg 10920
tcggcggcga tcgggctgcc ggagaccgag gacatcctcg atccgatcaa ctaccactgg 10980
gcgcccgccc gggaggatct ggagcggctg cgcgcgaaca ccggcgatgt ggtgctgttc 11040
cgggcggacg agccgaacga gatcgtccgg gacgaggagc agcgccggct gttcgacttc 11100

taccagcggt cctccttcaa cgggctggac gcgctgctgc ccgcggagtc catcgaggtc     11160

caccggctgc acggtgagac ccaccattcg tgggtacgaa acgaccggct ggtcgccgac     11220

atctgtgcgc gtgtctcggc gtcgtcgccg gatgcccggt ga                        11262


<210> 4
<211> 30
<212> DNA
<213> Artificial Sequence

<220>
<221> source
<222> 1..30
<223> /mol_type="DNA"
      /note="Oligonucleotide primer codA_XbaI_R   "
      /organism="Artificial Sequence"

<400> 4
gcgctctaga gcgcttgtag tcgatggcct                                      30


<210> 5
<211> 34
<212> DNA
<213> Artificial Sequence

<220>
<221> source
<222> 1..34
<223> /mol_type="DNA"
      /note="Oligonucleotide primer codA_NdeI_F "
      /organism="Artificial Sequence"

<400> 5
gcggcatatg gttgacatct tttgccgatt ctgg                                  34


<210> 6
<211> 37
<212> DNA
<213> Artificial Sequence

<220>
<221> source
<222> 1..37
<223> /mol_type="DNA"
      /note="Oligonucleotide primer Apr (aac3)_XbaI_F  "
      /organism="Artificial Sequence"

<400> 6
cgcgtctaga ggatcttcac ctagatcctt ttggttc                               37


<210> 7
<211> 28
<212> DNA
<213> Artificial Sequence

<220>
<221> source
<222> 1..28
<223> /mol_type="DNA"
      /note="Oligonucleotide primer ApR(aac3)_EcoRI  "
      /organism="Artificial Sequence"

<400> 7
ccgggaattc cccgatccgc tccacgtg                                        28

<210> 8
<211> 28
<212> DNA
<213> Artificial Sequence

<220>
<221> source
<222> 1..28
<223> /mol_type="DNA"
      /note="Oligonucleotide primer pcbAB_L1_F_EcoRI "
      /organism="Artificial Sequence"

<400> 8
gcgcgaattc cgctgacgat cgccgagc                                          28


<210> 9
<211> 28
<212> DNA
<213> Artificial Sequence

<220>
<221> source
<222> 1..28
<223> /mol_type="DNA"
      /note="Oligonucleotide primer pcbAB_L1_R_BamHI  "
      /organism="Artificial Sequence"

<400> 9
gcgcggatcc cgttgaggcg ctggtccg                                          28


<210> 10
<211> 28
<212> DNA
<213> Artificial Sequence

<220>
<221> source
<222> 1..28
<223> /mol_type="DNA"
      /note="Oligonucleotide primer pcbAB_D1_F_BamHI "
      /organism="Artificial Sequence"

<400> 10
gcgcggatcc gcggggtgct ctcggtgg                                          28


<210> 11
<211> 31
<212> DNA
<213> Artificial Sequence

<220>
<221> source
<222> 1..31
<223> /mol_type="DNA"
      /note="Oligonucleotide primer pcbAB_D1_R_HindIII"
      /organism="Artificial Sequence"

<400> 11
gcgcaagctt agcgagacgg acgacaggga g                                      31


<210> 12
<211> 23
<212> DNA

<213> Artificial Sequence

<220>
<221> source
<222> 1..23
<223> /mol_type="DNA"
      /note="Oligonucleotide primer pcbAB_conf_F3 "
      /organism="Artificial Sequence"

<400> 12
agctgttcga ggacacgacg gtc                                              23


<210> 13
<211> 24
<212> DNA
<213> Artificial Sequence

<220>
<221> source
<222> 1..24
<223> /mol_type="DNA"
      /note=" Oligonucleotide primer pcbAB_conf_R3 "
      /organism="Artificial Sequence"

<400> 13
gacggcatca gaactggcat gaaa                                             24


<210> 14
<211> 24
<212> DNA
<213> Artificial Sequence

<220>
<221> source
<222> 1..24
<223> /mol_type="DNA"
      /note="Oligonucleotide primer pcbAB_conf_R3_IN "
      /organism="Artificial Sequence"

<400> 14
agtcgacctt gccgttgaca ttga                                             24


<210> 15
<211> 25
<212> DNA
<213> Artificial Sequence

<220>
<221> source
<222> 1..25
<223> /mol_type="DNA"
      /note="Oligonucleotide primer pcbAB_conf_znotraj_F "
      /organism="Artificial Sequence"

<400> 15
acttccagct caagctgaac ggtgt                                            25


<210> 16
<211> 24
<212> DNA
<213> Artificial Sequence

<220>
<221> source
<222> 1..24

<223> /mol_type="DNA"
      /note="Oligonucleotide primer pcbAB_conf_znotraj_R "
      /organism="Artificial Sequence"

<400> 16
tccttgacgt ggtcgtactc gctc                                              24